(19) Europäisches Patentamt
European Patent Office
Office européen des brevets

(11) Veröffentlichungsnummer : **0 026 157**
**B1**

# (12) EUROPÄISCHE PATENTSCHRIFT

(45) Veröffentlichungstag der Patentschrift : **13.06.84**

(21) Anmeldenummer : **80810290.9**

(22) Anmeldetag : **15.09.80**

(51) Int. Cl.³ : **C 07 C127/15**, C 07 C103/54, C 07 C 93/20, C 07 D295/12, C 07 D233/72, A 61 K 7/09, A 01 N 47/28, A 01 N 43/00, A 01 N 33/12, A 01 N 37/00, D 06 P 1/00

## (54) Quaternäre Ammoniumsalze aus Diepoxyden und Diaminen, ihre Herstellung und Verwendung.

(30) Priorität : **20.09.79 CH 8489/79**

(43) Veröffentlichungstag der Anmeldung : **01.04.81 Patentblatt 81/13**

(45) Bekanntmachung des Hinweises auf die Patenterteilung : **13.06.84 Patentblatt 84/24**

(84) Benannte Vertragsstaaten : **AT BE CH DE FR GB IT LI NL SE**

(56) Entgegenhaltungen :
**DE-B- 1 239 289**
**GB-A- 1 546 809**
**GB-A- 2 000 164**

(73) Patentinhaber : **CIBA-GEIGY AG**
**Postfach**
**CH-4002 Basel (CH)**

(72) Erfinder : **Haase, Jaroslav**
**Helvetierstrasse 15**
**CH-4125 Riehen (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 026 157 B1

**Beschreibung**

Aus der britischen Patentschrift 1 546 809 sind quaternäre Ammoniumsalze aus Diaminen und Dimethylnaphthyl- oder Dimethyldiphenyl -dihalogeniden und aus der britischen Patentanmeldung 2 000 164 A sind quaternäre Ammoniumsalze aus Polyaminen und aliphatischen oder aromatischen Dihalogeniden bekannt. Im Gegensatz hierzu werden in der vorliegenden Erfindung neue quaternäre Ammoniumsalze aus Diaminen und Diepoxyden beschrieben, die leichter zugänglich sind als die bekannten Ammoniumsalze aus Di- oder Polyaminen und Dihalogeniden, da Di- epoxyde ein Grossprodukt der Kunststoffindustrie zur Herstellung von Epoxydharzen darstellen.

Gegenstand der vorliegenden Erfindung sind somit quaternäre Ammoniumsalze, die als kennzeichnendes Merkmal Diepoxyd- und Diaminreste enthaltende Einheiten der Formel

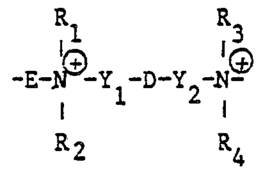

(1)

aufweisen, worin E ein zweiwertiges Brückenglied ist, das einer der Formeln

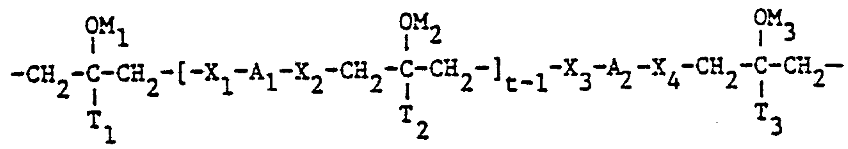

(1.1)

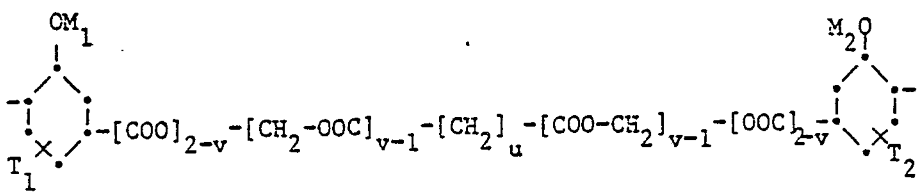

, (1.2)

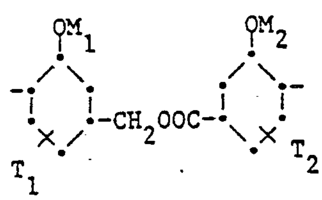

(1.3)

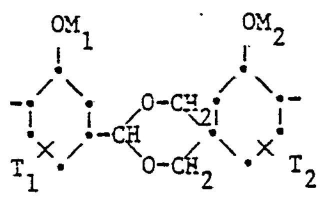

oder (1.4)

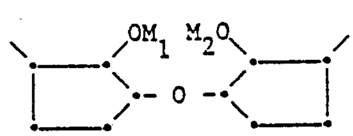

(1.5)

entspricht, worin t eine Zahl von 1 bis 4, u eine ganze Zahl von 1 bis 8, und v 1 oder 2 sind, $R_1$, $R_2$, $R_3$ und $R_4$ gleich oder voneinander verschieden sind und gegebenenfalls durch Hydroxyl, Alkoxy, Alkylthio oder Cyano substituiertes Alkyl, Cycloalkyl oder Alkenyl mit höchstens 8 Kohlenstoffatomen oder gegebenenfalls durch Hydroxyl, Hydroxyalkyl, Alkoxy, Alkoxyalkyl, Alkyl, Alkylthio, Cyano oder Halogen substituiertes Aryl oder Aralkyl bedeuten, oder ($R_1$ und $R_2$) und/oder ($R_3$ und $R_4$) zusammen mit dem Stickstoffatom, mit dem sie verbunden sind, einen heterocyclischen Ring mit 5 oder 6 Ringgliedern bilden, $M_1$, $M_2$ und $M_3$ je Acyl mit 2 bis 5 Kohlenstoffatomen oder Wasserstoff bedeuten, $Y_1$ und $Y_2$ gleich oder voneinander verschieden sind und der Formel

$$—C_mH_{2m}— \quad (1.6)$$

entsprechen, worin m eine ganze Zahl von 1 bis 12 ist, die Summe von m in $Y_1$ und $Y_2$ mindestens 3 beträgt und für m gleich 1 die Bindung zum Brückenglied D nicht über ein Stickstoff- oder Sauerstoffatom erfolgt, oder $Y_1$ und $Y_2$ gegebenenfalls durch Halogen, Hydroxyl, Alkyl, Halogenalkyl, Hydroxyalkyl oder Alkoxy substituiertes Phenylen bedeuten, D einem zweiwertigen Brückenglied einer der Formeln

(1.7) -NHCONH-, (1.8) $-NHCOD_1CONH-$, (1.9) -CONH-,

(1.10) -OCONH-, (1.11) -COO-, (1.12) $-COD_2CO-$,

$$-O\overset{\displaystyle O}{\overset{\|}{C}}-D_3-\overset{\displaystyle O}{\overset{\|}{C}}O-$$ oder $$-O\overset{\displaystyle O}{\overset{\|}{C}}-NH-D_4-HN-\overset{\displaystyle O}{\overset{\|}{C}}O-$$

entspricht, worin $D_1$ die direkte Bindung, Alkylen, Alkenylen, Arylen, Heteroarylen, Diaminoalkylen, Diaminoarylen, gegebenenfalls halogensubstituiertes Dioxyalkylen, Polyoxyalkylenoxy oder Dioxyarylen, $D_2$ Diaminoalkylen, gegebenenfalls halogensubstituiertes Dioxyalkylen, Polyoxyalkylenoxy oder Dithioalkylen, $D_3$ Arylen und $D_4$ Alkylen oder Arylen sind, $T_1$, $T_2$ und $T_3$ je Wasserstoff oder Methyl, $X_1$, $X_2$, $X_3$ und $X_4$ je —COO—, —OOC—, —O— oder die direkte Bindung und $A_1$ und $A_2$ je einen gegebenenfalls durch Alkyl substituierten heterocyclischen Ring mit 5 oder 6 Ringgliedern und 2 Stickstoff- atomen, gegebenenfalls durch Heteroatome unterbrochenes Alkylen, gegebenenfalls durch Alkyl oder Halogen substituiertes 1 oder 2 Ringe aufweisendes Cycloalkylen, Cycloalkenylen, Arylen oder Aralkylen bedeuten, wobei die ringförmigen Brückenglieder über eine direkte Bindung, über ein Heteroatom oder über eine gegebenenfalls durch Heteroatome unterbrochene Alkylenbrücke verbunden sind.

Das Verfahren zur Herstellung dieser Ammoniumsalze und deren Verwendung als kosmetisches Mittel, als Auswaschmittel für kationisch gefärbte oder bedruckte Textilmaterialien, als Papierleimungsmittel, als Fixierungsmittel für anionisch gefärbte, cellulosehaltige Materialien und als algizide, fungizide und bakterizide Mittel bilden weitere Gegenstände der vorliegenden Erfindung. Von den vielfältigen Verwendungszwecken steht die Verwendung der erfindungsgemässen Ammoniumsalze als kosmetisches, insbesondere als haarkosmetisches Mittel im Vordergrund des Interesses. Die vielseitige Verwendungsmöglichkeiten der Ammoniumsalze stellen ein wesentlicher Vorteil der vorliegenden Erfindung dar.

Die Reste $R_1$, $R_2$, $R_3$ und $R_4$ in den kationischen Einheiten der quaternären Ammoniumsalze der Formel (1) sind u. a. geradkettige oder verzweigte Alkylreste mit 1 bis 8 Kohlenstoffatomen, z. B. Methyl, Aethyl, Propyl, Isopropyl, Butyl, Isobutyl, tert. Butyl, Hexyl, Octyl, Isooctyl oder tert. Octyl.

Bevorzugt sind Alkylreste mit 1 bis 4 Kohlenstoffatomen und insbesondere Methyl und Aethyl.

Substituierte Alkylreste sind z. B. Hydroxyalkyl, Cyanoalkyl, Alkoxyalkyl oder Alkylthioalkyl, wobei die Substituenten Alkoxy und Alkylthio vorzugsweise 1 bis 4 und insbesondere 1 oder 2 Kohlenstoffatome aufweisen.

Bei den Cycloalkylresten als Bedeutung für die Reste $R_1$ bis $R_4$ handelt es sich im wesentlichen um Cyclopentyl oder Cyclohexyl, die gegebenenfalls wie die Alkylreste substituiert sein können.

Falls $R_1$ bis $R_4$ Alkenylreste sind, so können 2 bis 8 Kohlenstoffatome enthalten sein. Bevorzugt sind solche mit 2 bis 4 Kohlenstoffatomen. Geeignet sind die Alkenylreste, die den genannten Alkylresten entsprechen. Die für die Alkylreste genannten Substituenten können im allgemeinen auch für die Alkenylreste verwendet werden.

Aryl- und Aralkylreste als Bedeutungen für $R_1$ bis $R_4$ sind insbesondere Phenyl und Benzyl, die gegebenenfalls mit Hydroxyl, Cyano, Halogen, Carboxyl, Alkyl, Hydroxyalkyl, Alkoxy oder Alkylthio substituiert sind, wobei Halogen vorzugsweise Jod, Fluor, insbesondere Brom oder Chlor ist und Alkyl, Hydroxyalkyl, Alkylthio und Alkoxy 1 bis 4, vorzugsweise 1 oder 2 Kohlenstoffatome aufweisen.

Die beiden Substituenten an jedem Stickstoff können ferner zusammen mit dem Stickstoffatom, mit dem sie verbunden sind, einen heterocyclischen Ring mit 5 oder 6 Ringgliedern bilden. Beispiele für solche heterocyclischen Ringe sind der Thiomorpholin-, vorzugsweise der Morpholin-, Pyrrolidin- oder Imidazolin- und insbesondere der Piperidinring.

$Y_1$ und $Y_2$ in Formel (1), die voneinander verschieden oder vorzugsweise gleich sein können, sind beispielsweise die Alkylengruppierung der angegebenen Formel (1.6), worin m eine ganze Zahl von 1 bis 12, vorzugsweise 1 bis 6 ist. Für m gleich 1, d. h. für die $—CH_2—$ Gruppierung, muss die Bindung zum Brückenglied D über andere Atome als Stickstoff und Sauerstoff erfolgen ; insbesondere erfolgt die Bindung über ein Kohlenstoffatom. Ferner muss die Summe von m in den beiden Gruppen der Formel (1.6) in $Y_1$ und $Y_2$ mindestens 3 betragen. Wie durch die Formel (1.6) bereits angezeigt, können die

Alkylenreste verzweigt oder geradkettig sein, wobei letztere bevorzugt sind.

$Y_1$ und $Y_2$ können ferner ein aromatisches Brückenglied, insbesondere ein gegebenenfalls substituiertes Phenylen sein.

Bei den möglichen Substituenten an diesen aromatischen Brückengliedern handelt es sich in der Regel um Niederalkyl, Niederalkoxy, Niederhydroxy- oder -halogenalkyl mit 1 bis 4 Kohlenstoffatomen, Hydroxyl und Halogen, insbesondere Chlor oder Brom.

Das Brückenglied D in Formel (1) entspricht zweiwertigen Resten einer der angegebenen Formeln (1.7) bis (1.14).

$D_1$ in Formel (1.8) ist u. a. die direkte Bindung, oder Alkylen, z. B. mit 1 bis 12 Kohlenstoffatomen. Im einzelnen seien die Gruppierungen $-CH_2-$, $-C_2H_4-$, $-C_3H_6-$, $-C_4H_8-$, $-C_5H_{10}-$ und $-C_6-H_{12}-$, genannt, wobei diese Gruppierungen verzweigt oder vorzugsweise geradkettig sind.

Ist $D_1$ Alkenylen, so kann es z. B. durch die Formeln

(1.15) $-CH=CH-$ oder (1.16) $-CH=CH-CH=CH-$, ferner

(1.17) 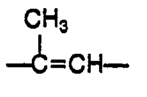 und (1.18) 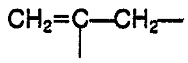

dargestellt werden.

Ist $D_1$ Arylen oder Heteroarylen, so können die entsprechenden aromatischen Brückenglieder z. B. durch eine der Formeln

(1.19) 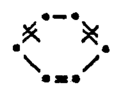, (1.20) 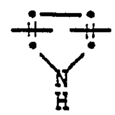, (1.21) 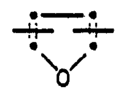,

(1.22) 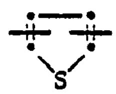, (1.23) 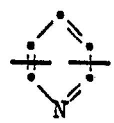, (1.24) 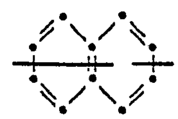

oder (1.25) 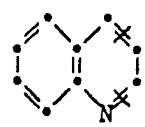

dargestellt werden, wobei die aromatischen Ringe mit Halogen, insbesondere Chlor oder Brom, Alkyl oder Alkoxy substituiert sein können.

Die Alkyl- und Alkoxyreste als Substituenten der aromatischen Brückenglieder einer der Formeln (1.19) bis (1.25) enthalten in der Regel 1 bis 5 Kohlenstoffatome und sind z. B. Methyl, Aethyl, Propyl, Isopropyl, Butyl, Isobutyl oder tert. Butyl ; ferner Methoxy, Aethoxy, Propoxy, Butoxy oder Pentoxy und die entsprechenden verzweigtkettigen Isomeren. Die aromatischen Brückenglieder können einen oder mehrere Substituenten enthalten.

$D_1$ ist ferner Diaminoalkylen, z. B. der Formel

(1.26) $-NH-C_{m1}H_{2m1}NH-$

oder insbesondere

(1.27) $-NH(CH_2)_{m_1}NH-$,

worin $m_1$ eine ganze Zahl von 2 bis 12 ist. Bevorzugt sind die bereits genannten Alkylengruppierungen.

Bei den Diaminoarylenresten als weitere Definition von $D_1$ handelt es sich vorzugsweise um solche der Formel

(1.28) 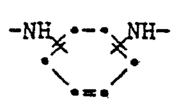

und insbesondere der Formel

(1.29) 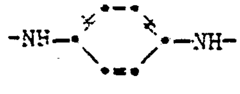

wobei der Phenylkern mit Halogen, insbesondere Chlor oder Brom, Alkyl- oder Alkoxygruppen insbesondere solche mit 1 bis 5 Kohlenstoffatomen der vorstehend angegebenen Art substituiert sein kann und ein oder mehrere Substituenten am Phenylkern vorhanden sein können.

Die Dioxy- und Polyoxyalkylenoxyreste, die als Brückenglieder $D_1$ in Frage kommen, können durch die Formeln

(1.30) $—OG_1O—$ und (1.31) $—(OG_2)_nO—$

dargestellt werden.

$G_1$ in Formel (1.30) ist z. B. geradkettiges oder verzweigtes Alkylen mit 2 bis 12 Kohlenstoffatomen ; insbesondere kommen hier ebenfalls die zuvorgenannten Gruppierungen, die gegebenenfalls mit Halogen, vorzugsweise Chlor oder Brom, substituiert sein können, in Frage.

In Formel (1.31) sind $G_2$ z. B. $—CH_2CH_2—$,

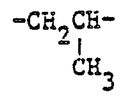

oder $—(CH_2)_4—$ und n eine ganze Zahl von 2 bis 15.

Beispiele für Dioxyalkylenoxyreste sind $—O(CH_2)_2O—$ oder $—O(CH_2)_4O—$ und für Polyoxyalkylenoxyreste $—OCH_2CH_2OCH_2CH_2O—$,

$-(OCH_2CH_2)_{15}O-$, 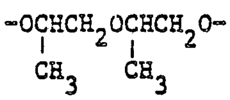 oder 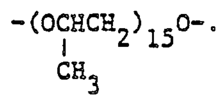.

Als in Betracht kommende Brückenglieder für $D_1$ sind ferner Dioxyarylenreste, die z. B. der Formel

(1.32) 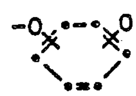 und

insbesondere der Formel

(1.33) 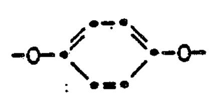

entsprechen, wobei der Phenylkern mit Halogen, insbesondere Chlor oder Brom, Alkyl oder Alkoxy, insbesondere solche mit 1 bis 5 Kohlenstoffatomen der vorstehend angegebenen Art, substituiert sein kann und ein oder mehrere Substituenten am Phenylkern vorhanden sein können. Ferner kommt auch der Rest der Formel

(1.34) 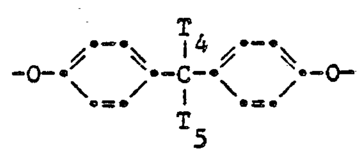

in Betracht, worin $T_4$ und $T_5$ Wasserstoff oder Methyl sind.

Das Brückenglied $D_2$ in Formel (1.12) ist Diaminoalkylen, Dioxyalkylen oder Polyoxyalkylenoxy und kann die gleichen Bedeutungen haben, wie sie zuvor bei der Definition der gleichen Gruppierungen für $D_1$ angegeben wurden. Ist $D_2$ Dithioalkylen so können diese Reste vorzugsweise durch die Formel

(1.35) $—SG_1S—$

dargestellt werden, worin $G_1$ die angegebene Bedeutung hat.

$D_3$ in Formel (1.13) ist Arylen und kann insbesondere Phenylen sein, wobei der Phenylring als Substituenten Halogen oder Niederalkyl, z. B. mit 1 bis 4 Kohlenstoffatomen, enthalten kann.

$D_4$ in formel (1.14) ist Alkylen, insbesondere geradkettiges Alkylen mit 1 bis 12, vorzugsweise 2 bis 6 Kohlenstoffatomen. Ist $D_4$ Arylen, so hat es insbesondere die Bedeutungen, die für $D_3$ angegeben sind.

$M_1$, $M_2$ und $M_3$ in Formeln (1.1) bis (1.5) sind z. B. Methyläthylacetyl, Isobutyryl, Butyryl, Isopropionyl, Propionyl, vorzugsweise Acetyl und insbesondere Wasserstoff. $T_1$, $T_2$ und $T_3$ in Formeln (1.1) bis (1.4) sind Methyl oder vorzugsweise Wasserstoff.

In der Regel stehen $X_1$ und $X_2$ bzw. $X_3$ und $X_4$ in Formel (1.1) für die direkte Bindung, falls $A_1$ bzw. $A_2$ einen heterocyclischen Ring bedeuten und für —OOC— bzw. —COO— oder —O— falls $A_1$ bzw. $A_2$ Alkylen, Cycloalkylen, Cycloalkenylen, Arylen oder Aralkylen bedeuten, wobei im allgemeinen bei den Brückengliedern —OOC— und —COO— die Kohlenstoffatome in benachbarter Stellung zu den Brückengliedern —$A_1$— bzw. —$A_2$— stehen.

Vorzugsweise haben $M_1$, $M_2$ und $M_3$ in Formel (1.1) und $M_1$ und $M_2$ in Formeln (1.2) bis (1.5) einerseits, $T_1$, $T_2$ und $T_3$, sowie $A_1$ bzw. $A_2$ und $X_1$ und $X_2$ bzw. $X_3$ und $X_4$ in Formel (1.1) und $T_1$ und $T_2$ in Formeln (1.2) bis (1.4) andererseits jeweils die gleichen Bedeutungen.

Beispiele heterocyclischer Ringe mit 5 oder 6 Ringgliedern und 2 Stickstoffatomen als Definition der zweiwertigen Radikale $A_1$ und $A_2$ in Formel (1.1) sind Imidazol-, Imidazolidin-, Imidazolin-, Piperazin-, Pyrazin-, Pyrazol-, Pyrazolidin-, Pyrazolin-, Pyridazin- und Pyrimidinringe. Ferner kommen auch in einer bevorzugten Ausführungsart dieser heterocyclischen Ringe Alloxan, Malonylharnstoff, auch Barbitursäure genannt, Oxalyl-, Aethylen- und Propylenharnstoff, Uracil, Dihydrouracil, Diketo-2,5-Pyrazin, Pyrazolon oder Hydantoin in Frage, wobei 5,6-Dihydrouracil, und vor allem 1,3-Propylen- und Aethylenharnstoff, sowie Hydantoin bevorzugt sind.

Als Alkylsubstituenten der heterocyclischen Ringe kommen vor allem solche mit 1 bis 4 Kohlenstoffatomen, vorzugsweise Isopropyl und Aethyl und insbesondere Methyl in Betracht. Somit sind Beispiele alkylsubstituierter heterocyclischer Ringe 1-, 2-, 4- oder 5-Methylimidazol, 1,1-, 1,4- und 1,5-Dimethylimidazol, 3- oder 5-Pyrazol, 3,5-Dimethylpyrazol, 2,4-Dimethylpyrimidin, 1-Methylpyrazolon, 5-Methyluracil auch Thymin genannt, vorzugsweise 5,5-Dimethyl-6-äthyl-5,6-dihydrouracil, 5,5,6-Trimethyl-5,6-dihydrouracil und insbesondere 5,5-Dimethyl-5,6-dihydrouracil, 6-Methyl-5,6-dihydrouracil, 5-Isopropyl-5-methylhydantoin, 5-Aethyl-5-methylhydantoin, 5,5-Dimethylhydantoin und 5-Methylhydantoin.

Falls die Brückenglieder $A_1$ bzw. $A_2$ Alkylen bedeuten, so kommen vor allem Alkylenbrücken mit höchstens 34, vorzugsweise 2 bis 34 und insbesondere 2 bis 8 Kohlenstoffatomen in Frage, die gegebenenfalls durch Heteroatome, vorzugsweise Sauerstoffatome und insbesondere 1 oder 2 Sauerstoffatome unterbrochen sind. Sofern $X_1$ bzw. $X_2$ und $X_3$ bzw. $X_4$ —OOC— bzw. —COO— bedeuten, leiten sich $A_1$ bzw. $A_2$ z. B. von acyclischen aliphatischen Dicarbonsäuren, wie Bernstein-, Glutar- Adipin-, Pimelin-, Kork-, Azelain- und Sebacinsäure, ferner von dimerisierter Linolsäure ab.

Sofern $X_1$ bzw. $X_2$ und $X_3$ bzw. $X_4$ —O— bedeuten, leiten sich hingegen $A_1$ bzw. $A_2$ z. B. von acyclischen aliphatischen Alkoholen wie Aethylen- und Diäthylenglykol, wie Propan-1,2-, Propan-1,3-, Butan-1,4- und Pentan-1,5-diol ab.

$A_1$ bzw. $A_2$ sind ferner zweiwertige ringförmige Brückenglieder wie Cycloalkylen, Cycloalkenylen, Arylen oder Aralkylen, die vorzugsweise 5 bis 26 Kohlenstoffatome aufweisen, wobei diese ringförmigen Brückenglieder 1 oder 2 Ringe aufweisen, die jeweils vorzugsweise 6 Ringkohlenstoffatome aufweisen und gegebenenfalls mit Alkyl, vorzugsweise mit 1 bis 4 Kohlenstoffatomen, insbesondere mit Methyl oder Aethyl und gegebenenfalls mit Halogen, vorzugsweise Brom oder Chlor substituiert sind. Bei den ringförmigen Brückengliedern, die zwei Ringe aufweisen, sind diese Ringe vorzugsweise direkt, über einen Heteroatom, insbesondere die Sulfongruppe, oder über eine Alkylenbrücke der vorstehend angegebenen Art, die gegebenenfalls durch Heteroatome, vorzugsweise 1 oder 2 Sauerstoffatome unterbrochen sind, miteinander verbunden.

Cycloalkenylen als Brückenglied ist vorzugsweise nur mit Alkyl der vorstehend beschriebenen Art gegebenenfalls substituiert und weist vorzugsweise nur einen Ring auf. Demgemäss enthalten Cycloalkylenbrückenglieder insgesamt vorzugsweise 6 bis 10 Kohlenstoffatome. Cycloalkylen ist ebenfalls vorzugsweise nur mit Alkyl substituiert, weist jedoch als Brückenglied sowohl 1 als auch 2 Ringe auf, wobei die beiden Ringe wie vorstehend beschrieben miteinander verbunden sind. Demgemäss enthalten Cycloalkylenbrückenglieder insgesamt vorzugsweise 6 bis 18 Kohlenstoffatome.

Arylen und Aralkylen sind vorzugsweise nur mit Halogen substituiert und weisen einen Ring oder zwei Ringe auf, die wie vorstehend beschrieben verbunden sind. Als Alkylenreste in Aralkylen kommen vor allem Alkylenreste mit 1 bis 4 Kohlenstoffatomen, vorzugsweise Aethylen und insbesondere Methylen in Betracht. Demgemäss enthalten als Brückenglieder Arylen vorzugsweise insgesamt 6 bis 18 und Aralkylen vorzugsweise insgesamt 8 bis 26 Kohlenstoffatome.

Sofern $X_1$ bzw. $X_2$ und $X_3$ bzw. $X_4$ —OOC— bzw. —COO— bedeuten, leiten sich $A_1$ bzw. $A_2$ z. B. von cycloaliphatischen Dicarbonsäuren wie Tetrahydrophthalsäure, 4-Methyltetrahydrophthalsäure, Hexahydrophthalsäure und 4-Methylhexahydrophthalsäure ; und von aromatischen Dicarbonsäuren wie Phthalsäure, Isophthalsäure und Terephthalsäure ab.

Sofern $X_1$ bzw. $X_2$ und $X_3$ bzw. $X_4$ —O— bedeuten, leiten sich hingegen $A_1$ bzw. $A_2$ z. B. von cycloaliphatischen Alkoholen wie Resorcit, Chinit, Bis-(4-hydroxycyclohexyl)-methan, 2,2-Bis-(4-hydroxycyclohexyl)-propan und 1,1-Bis-(hydroxymethyl)-cyclohex-3-en ; von aromatischen Alkoholen wie p,p'-Bis-(2-hydroxyäthylamino)-diphenylmethan ; von einkernigen Phenolen wie Resorcin und Hydrochinon, und insbesondere von mehrkernigen Phenolen wie Bis(4-hydroxyphenyl)-methan, auch Bisphenol F genannt, 4,4'-Dihydroxydiphenyl, Bis(4-hydroxyphenyl)-sulfon, 2,2-Bis(4-hydroxyphenyl)-propan, auch Bisphenol A genannt und 2,2-Bis(3,5-dibrom-4-hydroxyphenyl)-propan ab.

In einer weiteren Ausführungsform können $X_1$ und $X_2$ bzw. $X_3$ und $X_4$ voneinander verschieden sein. So kann z. B. $X_1$ —O— und $X_2$ —COO— bedeuten, wobei in diesem Fall $A_1$ sich von der Salicylsäure ableitet. Vorzugsweise haben jedoch wie bereits angedeutet $X_1$, $X_2$, $X_3$ und $X_4$ jeweils die gleichen

Bedeutungen und stehen in der Regel für die direkte Bindung sofern $A_1$ und $A_2$ einen heterocyclischen Ring der angegebenen Art bedeutet und für —O— oder —OOC— bzw. —COO—, sofern $A_1$ und $A_2$ Alkylen, Cycloalkylen, Cycloalkenylen, Arylen oder Aralkylen bedeuten.

t in Formel (1.1) ist 1 bis 4, vorzugsweise 1 bis 3, wobei nicht nur ganze Zahlen, sondern auch dazwischen beliebige Werte in Betracht kommen.

Das Mittelglied der Brückenglieder der Formel (1.2) in welchem v 2 ist, leitet sich von Dicarbonsäuren ab, die je nach Bedeutung von u 1 bis 8 Kohlenstoffatomen aufweisen wie Malon-, Bernstein-, Glutar-, Adipin-, Pimelin-, Kork-, Azelain- und Sebacinsäure, wobei Malon-, Bernstein- und vor allem Adipinsäure bevorzugt sind. Ist hingegen v in Formel (1.2) 1, so leitet sich das Mittelglied dieser Brückenglieder von den entsprechenden Diolen wie Aethylenglykol, Propan-1,3-, Butan-1,4- und Pentan-1,6-diol ab.

Bevorzugt sind Ammoniumsalze mit kationischen Einheiten der Formel (1) worin E der Formel (1.1) entspricht, d. h. Ammoniumsalze, deren kationische Einheiten der Formel

(2). 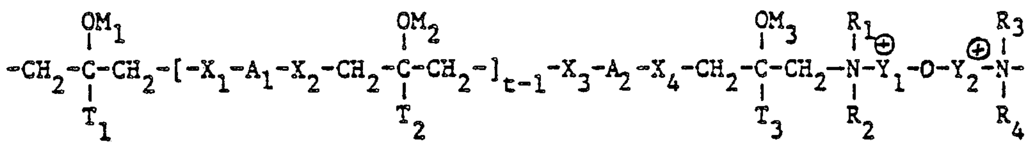

entsprechen, worin $A_1$, $A_2$, D, $M_1$, $M_2$, $M_3$, $R_1$, $R_2$, $R_3$, $R_4$, $T_1$, $T_2$, $T_3$, $X_1$, $X_2$, $X_3$, $X_4$, $Y_1$, $Y_2$ und t die angegebenen Bedeutungen haben.

Weiter bevorzugte erfindungsgemässe Ammoniumsalze enthalten Einheiten der Formel

(3) 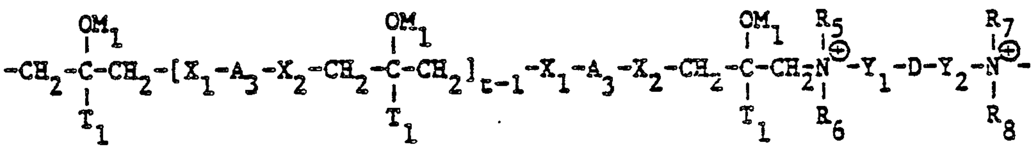

worin $R_5$, $R_6$, $R_7$ und $R_8$ gleich oder voneinander verschieden sind und Alkyl, Hydroxyalkyl, Alkoxyalkyl, Alkylthioalkyl oder Cyanoalkyl mit insgesamt 1 bis 8 Kohlenstoffatomen, Cyclopentyl, Cyclohexyl, Alkenyl mit 2 bis 4 Kohlenstoffatomen, unsubstituiertes Phenyl oder Benzyl oder mit Hydroxyl, Cyano, Chlor, Brom, Alkyl, Hydroxyalkyl, Alkoxy, Alkylthio, Alkoxyalkyl mit je 1 oder 2 Kohlenstoffatomen im Alkyl- und Alkoxyteil substituiertes Phenyl oder Benzyl bedeuten ; oder ($R_5$ und $R_6$) und ($R_7$ und $R_8$) zusammen mit dem Stickstoffatom, mit dem sie verbunden sind, einen heterocyclischen Ring der Formeln

(3.1) 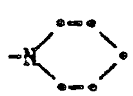 , (3.2) 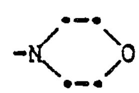 ,

(3.3) 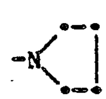 oder (3.4) 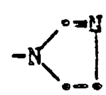

bilden ; $A_3$ einen gegebenenfalls durch Methyl, Aethyl oder Isopropyl substituierten heterocyclischen Ring mit 5 oder 6 Ringgliedern und 2 Stickstoffatomen, gegebenenfalls durch Sauerstoffatome unterbrochenes Alkylen mit 2 bis 34 Kohlenstoffatomen, gegebenenfalls durch Methyl oder Aethyl substituiertes Cycloalkenylen mit 6 bis 10 Kohlenstoffatomen oder ein oder zwei Ringe aufweisendes Cycloalkylen mit 6 bis 18 Kohlenstoffatomen, gegebenenfalls durch Brom oder Chlor substituiertes ein oder zwei Ringe aufweisendes Arylen mit 6 bis 18 Kohlenstoffatomen oder Aralkylen mit 8 bis 26 Kohlenstoffatomen bedeutet und D, $M_1$, $T_1$, $X_1$, $X_2$, $Y_1$, $Y_2$ und t die angegebenen Bedeutungen haben, vor allem Einheiten der Formel

(4) 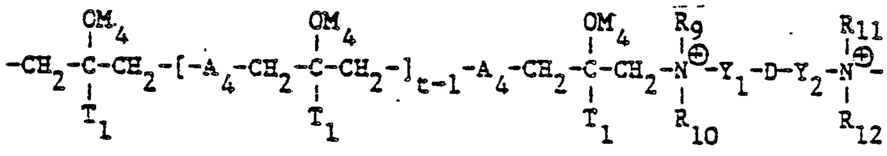

worin $R_9$, $R_{10}$, $R_{11}$ und $R_{12}$ gleich oder voneinander verschieden sind und Phenyl, Benzyl oder Alkyl mit 1 bis 4 Kohlenstoffatomen bedeuten oder ($R_9$ und $R_{10}$) und ($R_{11}$ und $R_{12}$) zusammen mit dem Stickstoffatom, mit dem sie verbunden sind, einen Piperidinring bilden, $M_4$ Propionyl, Acetyl oder Wasserstoff, $A_4$ einen zweiwertigen Rest einer der Formeln

(4.1) 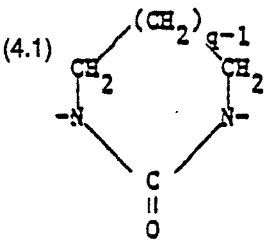

(4.2) 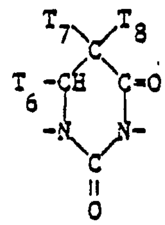

(4.3) 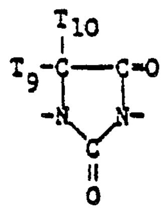

(4.4) $-O-(OC)_{q-1}-Q-(CO)_{q-1}-O-$

(4.5) 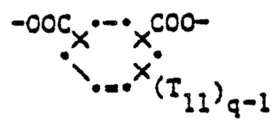

(4.6) 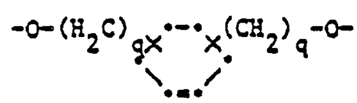

(4.7) 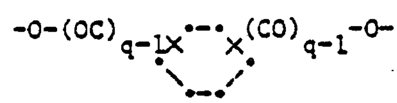

(4.8) 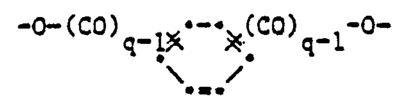

oder (4.9) 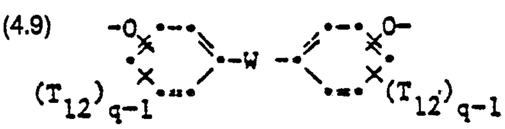

worin Q gegebenenfalls durch 1 oder 2 Sauerstoffatome unterbrochenes Alkylen mit 2 bis 8 Kohlenstoffatomen, W die direkte Bindung, $—SO_2—$ oder Alkylen mit 1 bis 4 Kohlenstoffatomen, $T_6$, $T_7$, $T_8$ und $T_9$ je Wasserstoff oder Methyl, $T_{10}$ Wasserstoff, Methyl, Aethyl oder Isopropyl, $T_{11}$ Methyl oder Aethyl, $T_{12}$ Chlor oder Brom und q 1 oder 2 bedeuten und D, $Y_1$, $Y_2$ und t die in Formel (1) bzw. (1.1) angegebenen Bedeutungen haben, oder insbesondere Einheiten der Formel

(5) 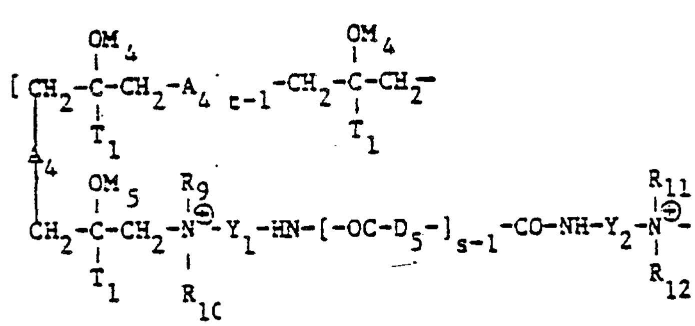

worin s 1 oder 2 ist, $D_5$ die direkte Bindung oder ein zweiwertiges Brückenglied einer der angegebenen Formeln (1.15) bis (1.31) oder Alkylen mit 1 bis 6 Kohlenstoffatomen bedeuten, und $Y_1$, $Y_2$ und t die in Formel (1.3) und $A_4$, $M_4$, $R_9$, $R_{10}$, $R_{11}$ und $R_{12}$ die in Formel (4) angegebenen Bedeutungen haben.

In einer besonders bevorzugten Ausführungsform enthalten die erfindungsgemässen Ammoniumsalze Einheiten der Formel

(6) 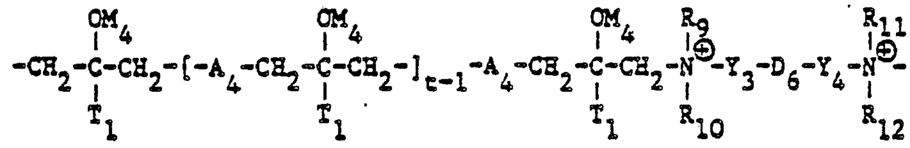

worin $D_6$ einer der Formeln

(6.1) 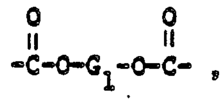 , (6.2) 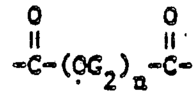 ,

(6.3) 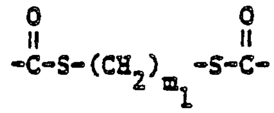 , (6.4) 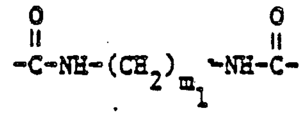

(6.5) 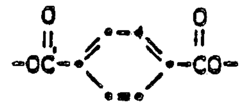 , (6.6) 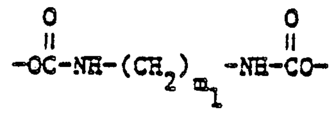 ,

(6.7) 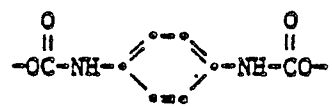 , (6.8) $-HN-OC-CO-NH-$ ,

(6.9) 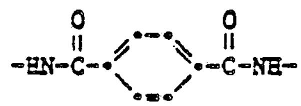 , (6.10) $-HN-CO-(CH_2)_{m_1}-CO-NH-$

oder einer der angegebenen Formeln (1.7), (1.9), (1.10) oder (1.11) entspricht, worin $G_1$ in Formel (6.1), $G_2$ und n in Formel (6.2) die für die Formeln (1.30) und (1.31) angegebenen Bedeutungen haben, $m_1$ in den Formeln (6.3), (6.4), (6.6) und (6.10) eine ganze Zahl von 2 bis 12 ist und $Y_3$ und $Y_4$ je Alkylen mit 1 bis 6 Kohlenstoffatomen oder Phenylen bedeuten und $A_4$, $M_4$, $R_9$, $R_{10}$, $R_{11}$ und $R_{12}$ die in Formel (4) und $T_1$ und t die in Formel (1.1) angegebenen Bedeutungen haben.

Erfindungsgemässe Ammoniumsalze, die im Vordergrund des Interesses stehen, weisen Einheiten auf, die vor allem der Formel

(7) 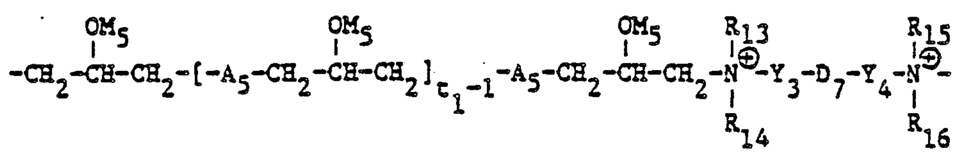

entsprechen, worin $R_{13}$, $R_{14}$, $R_{15}$ und $R_{16}$ je Benzyl, Methyl oder Aethyl sind oder ($R_{13}$ und $R_{14}$) und ($R_{15}$ und $R_{16}$) zusammen mit dem Stickstoffatom, mit dem sie verbunden sind, einen Piperidinring bilden, $M_5$ Acetyl oder Wasserstoff und $t_1$ eine Zahl von 1 bis 3 bedeutet, $A_5$ für die angegebenen Formeln (4.3), (4.4), (4.7) oder (4.9), $D_7$ für die angegebenen Formeln (1.7), (1.9), (6.5), (6.8), (6.9) oder (6.10) stehen, $M_5$ die in Formel (5) und $Y_3$ und $Y_4$ die in Formel (6) angegebenen Bedeutungen haben.

In einer anderen, bevorzugten Ausführungsart weisen erfindungsgemässe Ammoniumsalze kationische Einheiten der Formel (1) auf, worin E einer der Formeln (1.2) bis (1.5) entspricht. Falls E der Formel (1.2) entspricht, weisen solche Ammoniumsalze kationische Einheiten der Formel

(8) 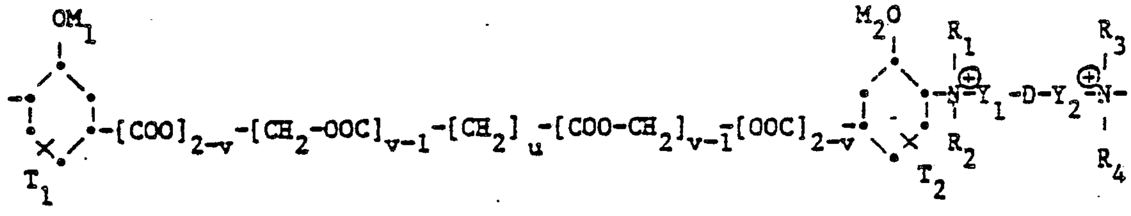

auf, worin D, $M_1$, $M_2$, $R_1$, $R_2$, $R_3$, $R_4$, $T_1$, $T_2$, $Y_1$, $Y_2$ u und v die in Formel (1) bzw. (1.2) angegebenen Bedeutungen haben.

Bevorzugt sind Ammoniumsalze mit kationischen Einheiten der Formel

(9) 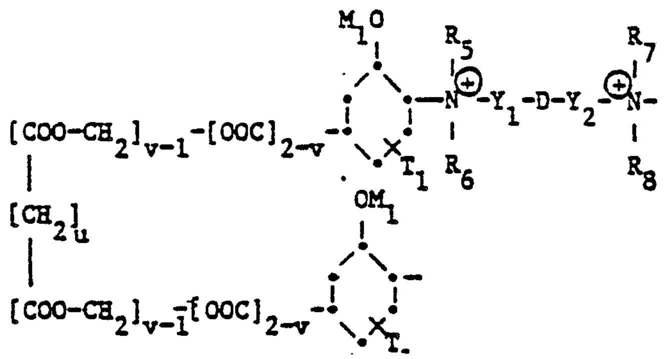 ,

worin D, $M_1$, $T_1$, $Y_1$, $Y_2$, u und v die in Formel (1) bzw. (1.2) und $R_5$, $R_6$, $R_7$ und $R_8$ die in Formel (3) angegebenen Bedeutungen haben, vor allem der Formel

(10) 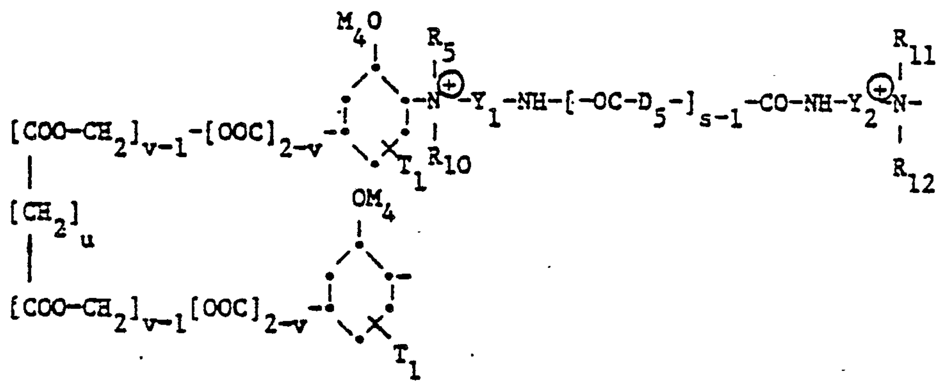

worin $T_1$, $Y_1$, $Y_2$, u und v die in Formel (1) bzw. (1.2) und $M_4$, $R_9$, $R_{10}$, $R_{11}$ und $R_{12}$ die in Formel (4) und $D_5$ und s die in Formel (5) angegebenen Bedeutungen haben, und insbesondere der Formel

(11) 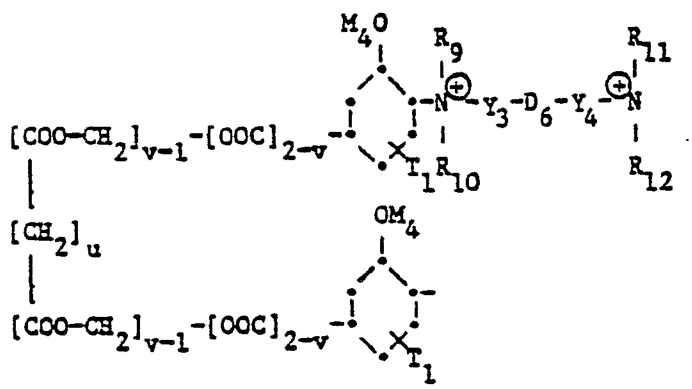

worin $T_1$, u und v die in Formel (1) bzw. (1.2), $M_4$, $R_9$, $R_{10}$, $R_{11}$ und $R_{12}$ die in Formel (4) und $D_6$, $Y_3$ und $Y_4$ die in Formel (6) angegebenen Bedeutungen haben.

Falls v in den Formeln (1.2) und (8) bis (10) 1 ist, entsprechen die kationischen Einheiten der Ammoniumsalze deren Ausführungsart, die im Vordergrund des Interesses steht, der Formel

(12) 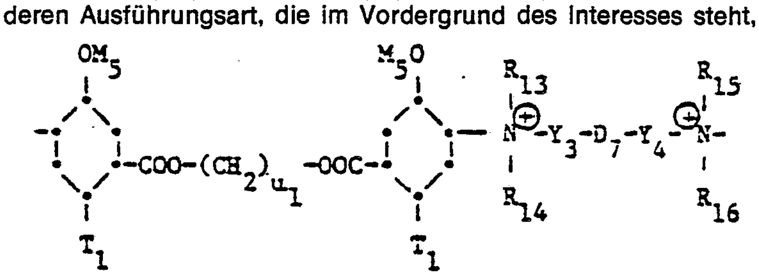 ,

worin $u_1$ eine ganze Zahl von 1 bis 4 und $T_1$ die in Formel (1.2), $Y_3$ und $Y_4$ die in Formel (6) und $D_7$, $M_5$, $R_{13}$, $R_{14}$, $R_{15}$ und $R_{16}$ die in Formel (7) angegebenen Bedeutungen haben.

Die Ammoniumsalze mit kationischen Einheiten der Formeln (8) bis (11), worin v 2 ist, sind den Ammoniumsalzen gegenüber, die kationische Einheiten der Formeln (8) bis (10) aufweisen, worin v 1 ist,

bevorzugt. In dieser besonders bevorzugten Ausführungsart weisen die Ammoniumsalze, die im Vordergrund des Interesses stehen, kationische Einheiten auf, die der Formel

(13) 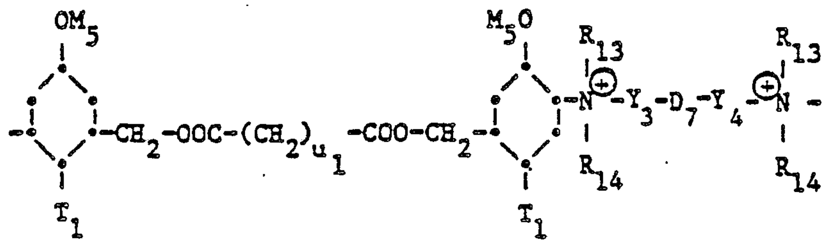

entsprechen, worin $u_1$ eine ganze Zahl von 1 bis 4, $T_1$ die in Formel (1.2), $Y_3$ und $Y_4$ die in Formel (6) und $M_5$, $D_7$, $R_{13}$, $R_{14}$, $R_{15}$ und $R_{16}$ die in Formel (7) angegebenen Bedeutungen haben.

In einer weiteren Ausführungsart der erfindungsgemässen Ammoniumsalze, die kationische Einheiten der Formel (1) aufweisen, worin E einer der Formeln (1.3), (1.4) oder (1.5) entspricht, sind Ammoniumsalze bevorzugt, deren kationische Einheiten einer der Formeln

(14) 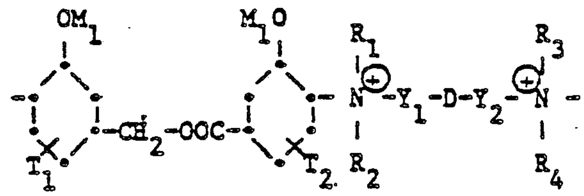 ,

(15) 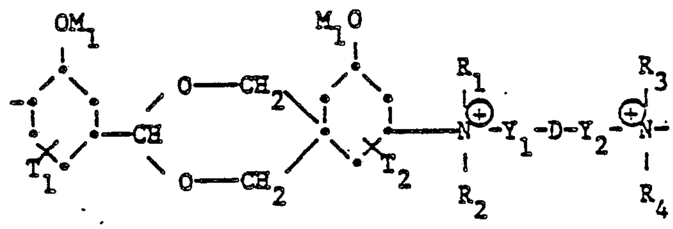 oder

(16) 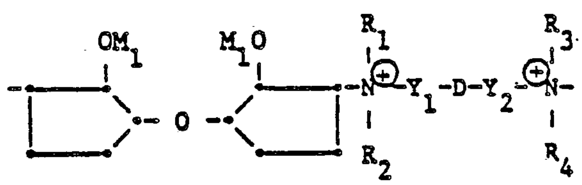

entsprechen, worin D, $M_1$, $R_1$, $R_2$, $R_3$, $R_4$, $T_1$, $Y_1$ und $Y_2$ die in Formel (1) bzw. (1.3) bis (1.5) angegebenen Bedeutungen haben.

Weiter bevorzugt sind Ammoniumsalze mit kationischen Einheiten einer der Formeln

(17) 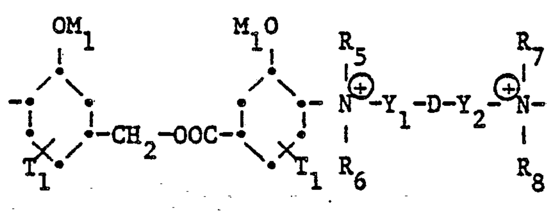 ,

(18) 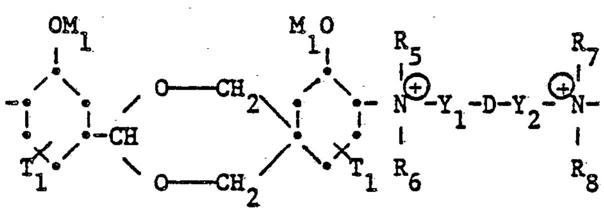 oder

(19) 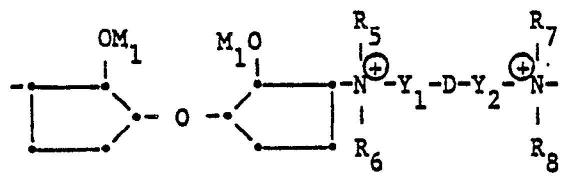 ,

worin D, $M_1$, $T_1$, $Y_1$ und $Y_2$ die in Formel (1) bzw. (1.3) bis (1.5) und $R_5$, $R_6$, $R_7$ und $R_8$ die in Formel (3) angegebenen Bedeutungen haben, vor allem einer der Formeln

(20) 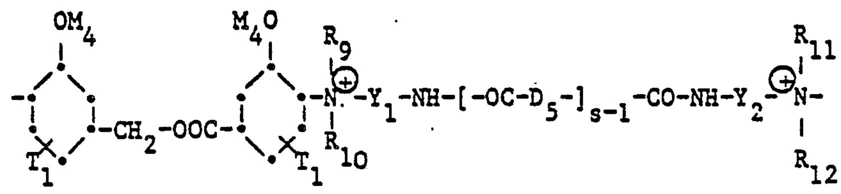 ,

(21) 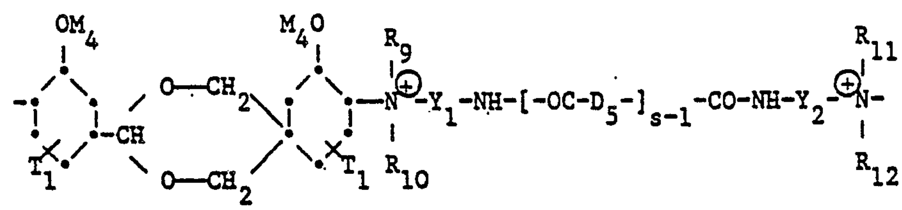 oder

(22) 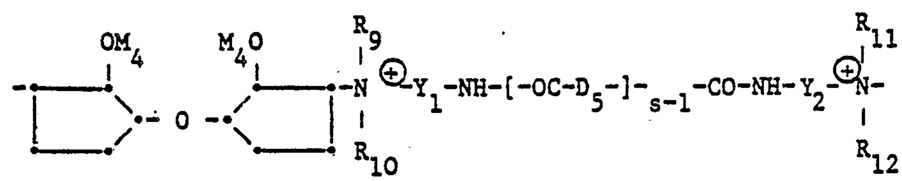 ,

worin $T_1$, $Y_1$ und $Y_2$ die in Formel (1) bzw. (1.3) bis (1.5), $M_4$, $R_9$, $R_{10}$, $R_{11}$ und $R_{12}$ die in Formel (4) und $D_5$ und s die in Formel (5) angegebenen Bedeutungen haben, und insbesondere einer der Formeln

(23) 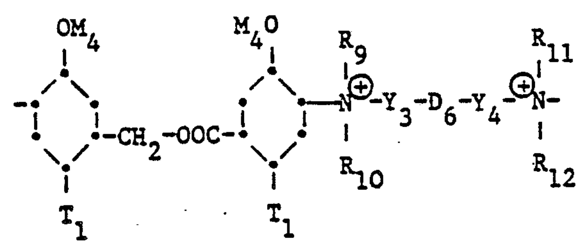 ,

(24) 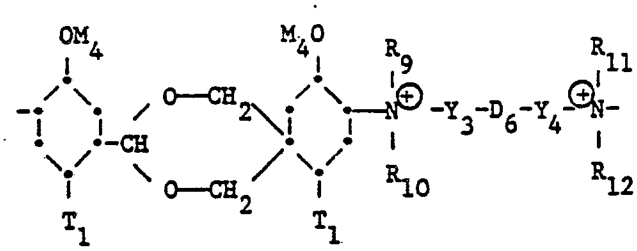 oder

(25) 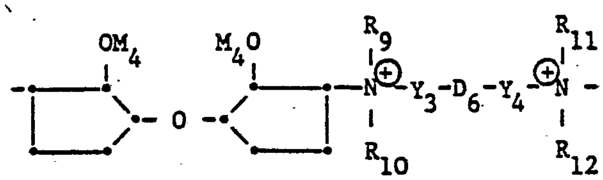 ,

worin $T_1$ die in den Formeln (1.3) bis (1.5), $M_4$, $R_9$, $R_{10}$, $R_{11}$ und $R_{12}$ die in Formel (4), und $D_6$, $Y_3$ und $Y_4$ die in Formel (6) angegebenen Bedeutungen haben.

Die kationischen Einheiten der Ammoniumsalze dieser Ausführungsart, die im Vordergrund des Interesses stehen, entsprechen einer der Formeln

(26)

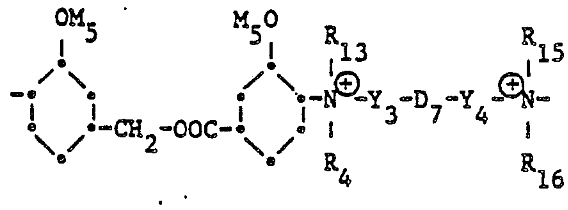

(27)

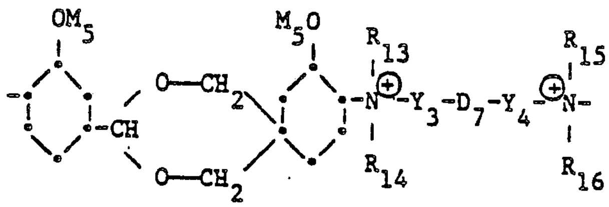

oder

(28)

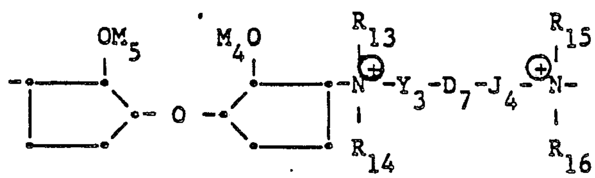

worin $Y_3$ und $Y_4$ die in Formel (6) und $M_5$, $D_7$, $R_{13}$, $R_{14}$, $R_{15}$ und $R_{16}$ die in Formel (7) angegebenen Bedeutungen haben.

Spezifische Vertreter der erfindungsgemässen Ammoniumsalze enthalten z. B. kationische Einheiten der Formeln

(29.1)

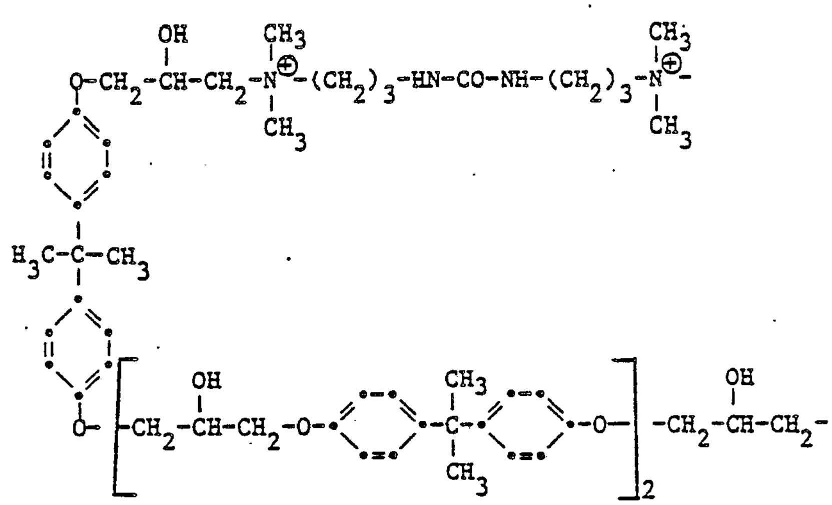

(29.2)

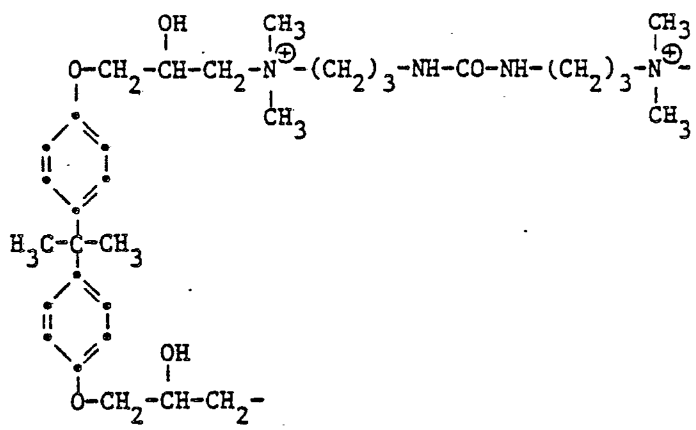

(29.3)

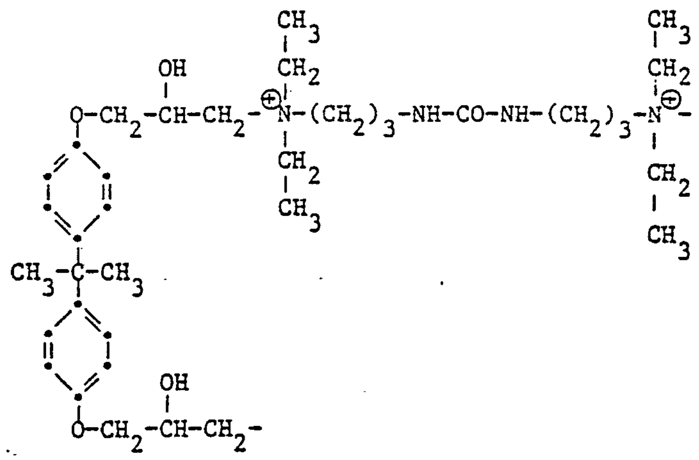

,

(29.4)

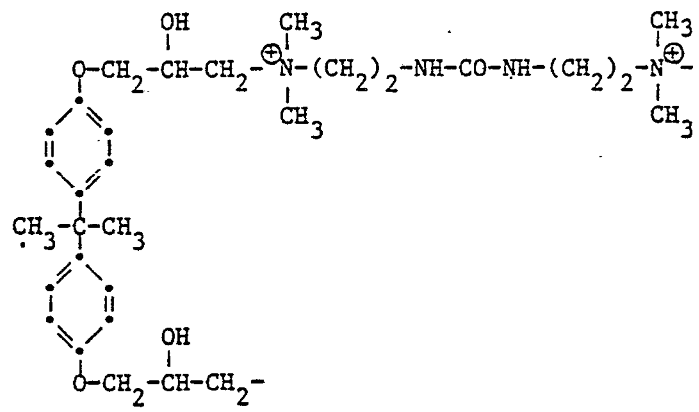

,

(29.5)

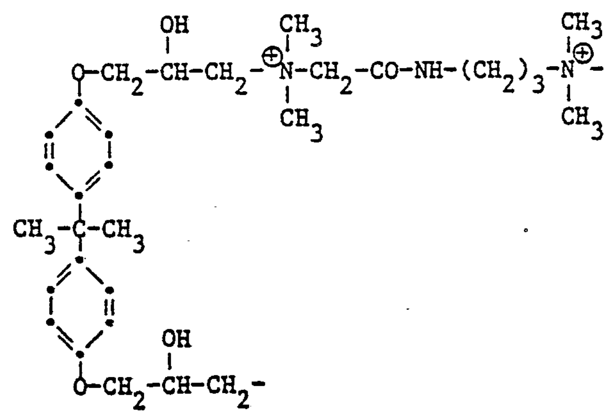

,

(29.6)

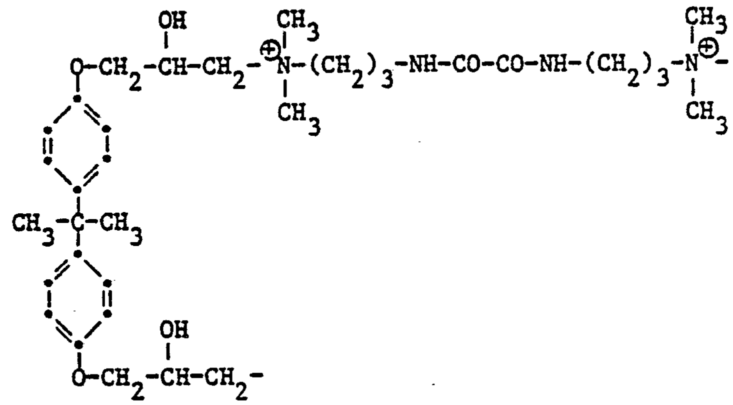

,

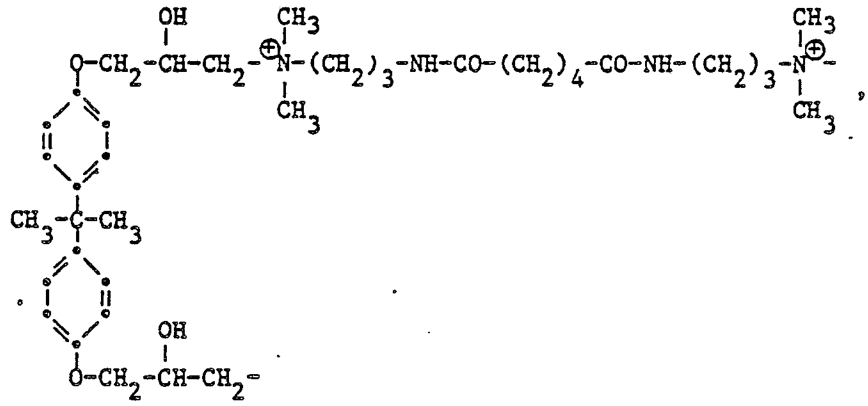

(29.7)

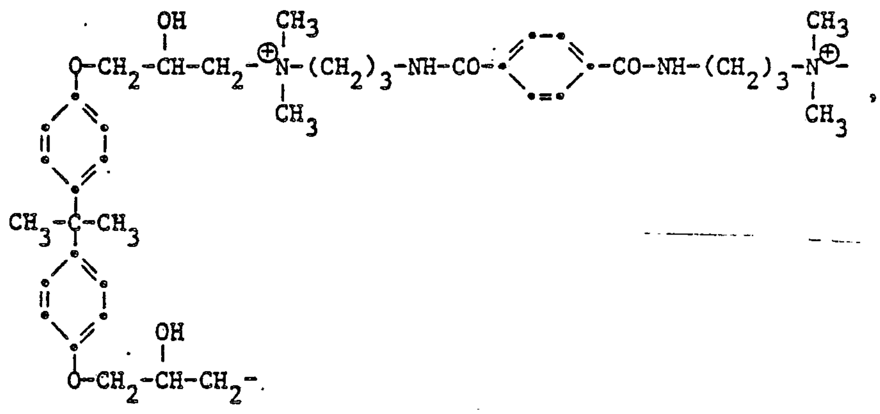

(29.8)

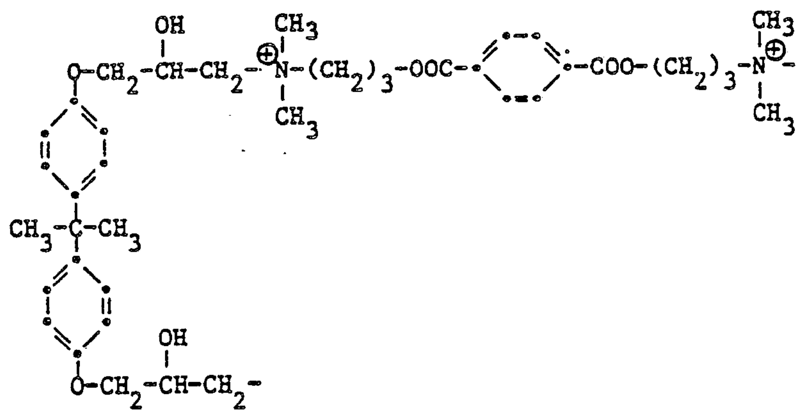

(29.9)

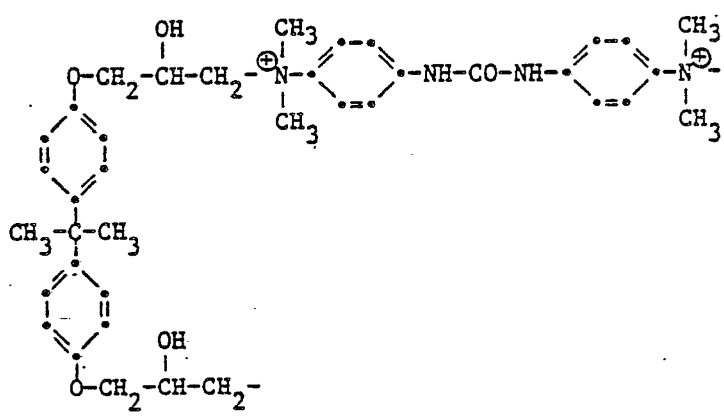

(29.10)

(29.11)

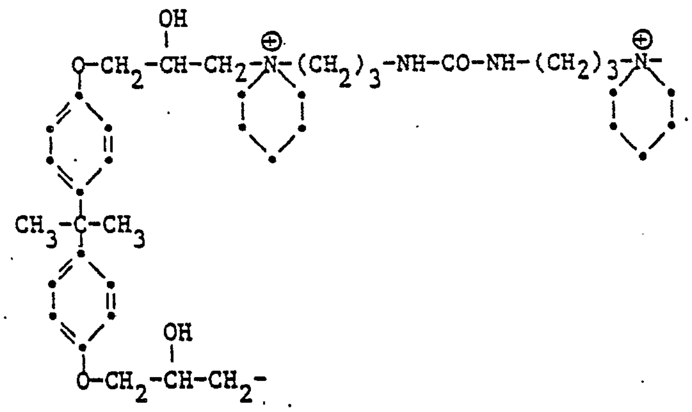

(29.12)

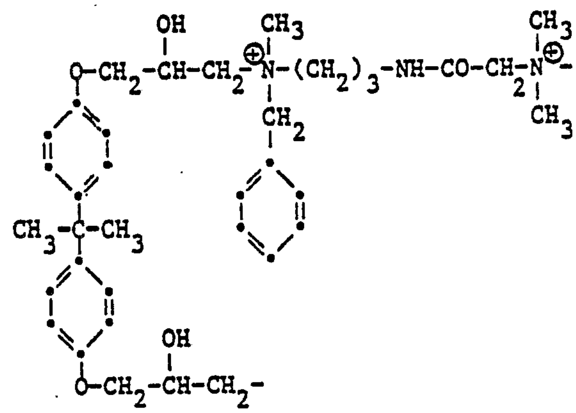

,

(29.13)

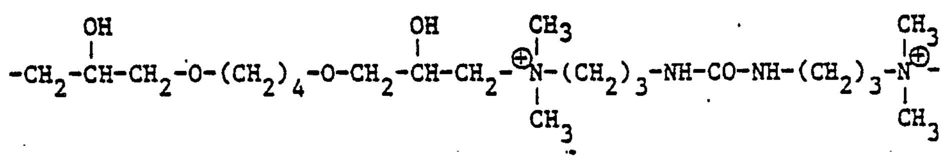

,

(29.14)

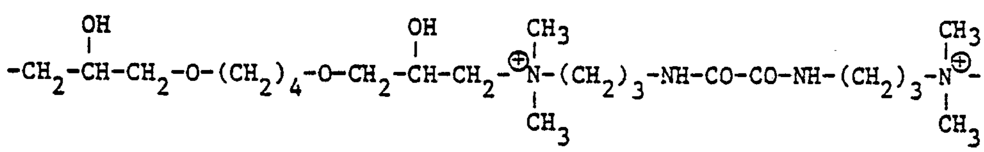

,

(29.15)

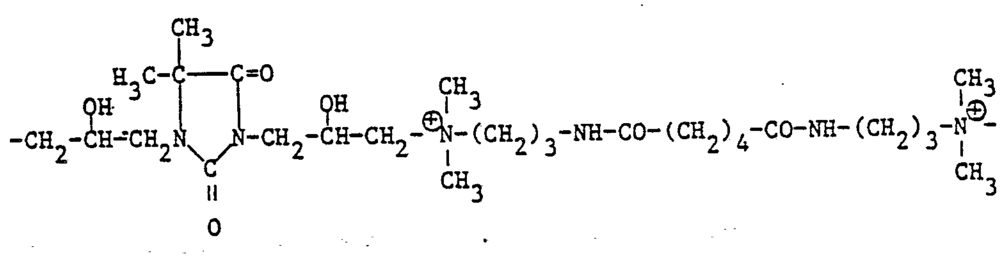

,

(29.16)

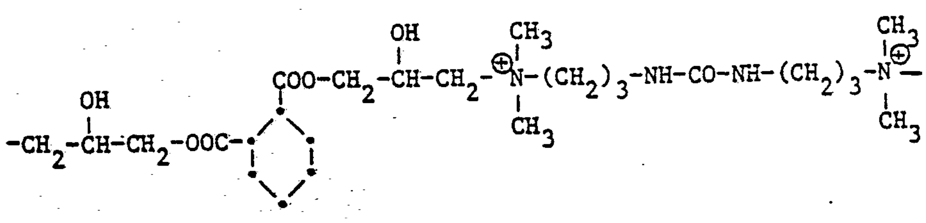

,

(29.17) 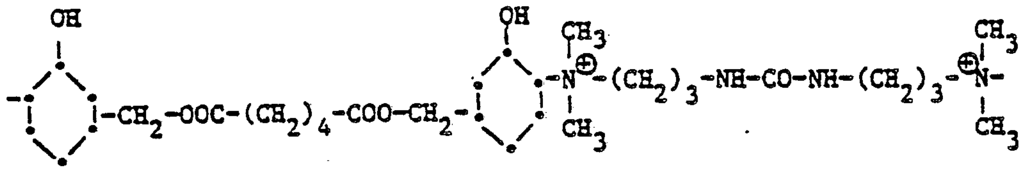 ,

(29.18) 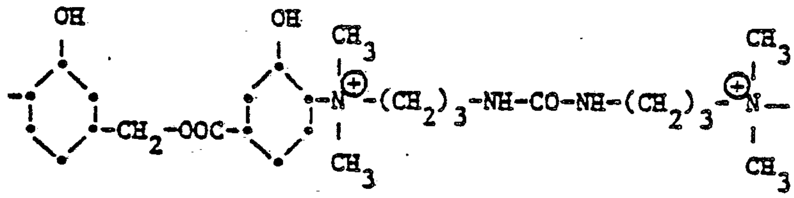 ,

(29.19) 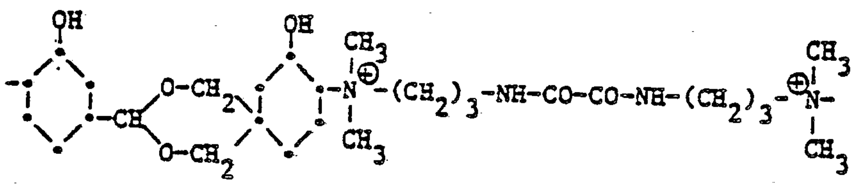 ,

(29.20) 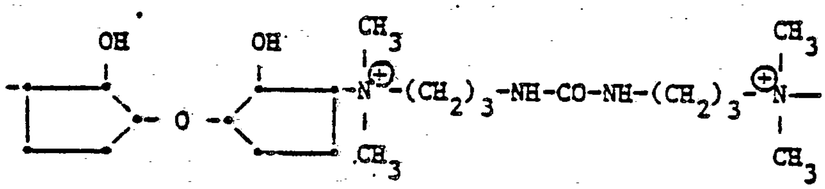

oder

(29.21) 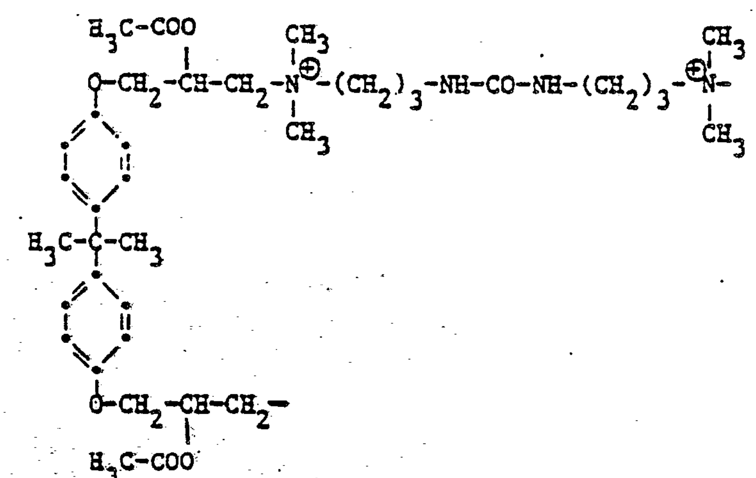

Das mittlere Molekulargewicht der Ammoniumsalze beträgt etwa 1 000 bis etwa 120 000, vorzugsweise etwa 1 000 bis etwa 35 000. Daraus ergibt sich, dass die Ammoniumsalze nicht nur eine, sondern auch vor allem mehrere wiederkehrende Einheiten einer der angegebenen Formeln (1) bis (29) umfassen, wobei in der Regel die wiederkehrenden Einheiten gleich sind. Durchschnittlich weisen die Ammoniumsalze 1 bis etwa 100, vorzugsweise 1 bis etwa 30 solcher Einheiten auf.

Als Anionen für die quaternären Ammoniumsalze kommen alle üblichen anorganischen oder organischen Anionen in Betracht, sofern die kationischen Einheiten der Formel (1) mit diesen Anionen keine Komplexe bilden, die in Wasser, in niedrigen Alkanolen oder in wässrigen Tensidsystemen schwerlöslich sind.

Als höhermolekulare organische Anionen kommen solche aus anionischen, handelsüblichen Tensiden in Betracht.

Als Beispiele solcher Tensidanionen seien genannt : Alkyl- und Alkylen-carboxylate, Alkyläthercarboxylate, Fettalkoholsulfate, Fettalkoholäthersulfate, Alkylolamidsulfate und -sulfonate, Fettsäurealkylolamidpolyglykoläthersulfate, Alkylphenylglykoläthersulfonate, Sulfobernsteinsäurehalbester und -diester oder Fettalkoholätherphosphate, wobei die Fett- bzw. Alkylreste in der Regel 8 bis 24, vorzugsweise 12 bis 18 Kohlenstoffatome enthalten und sich von den entsprechenden Fettsäuren ableiten, z. B. Oelsäure, Palmitinsäure, Stearinsäure, Linol- und Linolensäure, ferner Behensäure und Clupanodonsäure, vor allem in Form ihrer technischen Gemische. Auch Reste der Fettsäuren der genannten Art kommen als Anionen in Frage. Dies trifft vor allem für die Anionen Oleat, Palmitat und insbesondere Laurat und Stearat zu.

Anionen niedermolekularer, organischer Säuren, z. B. von organischen Säuren mit 1 bis 4 Kohlenstoffatomen wie Ameisensäure, Essigsäure, Propionsäure und Oxalsäure, von aromatischen Sulfonsäuren mit 6 bis 9 Kohlenstoffatomen wie Benzol- und Toluolsulfonsäure und vor allem von Mineralsäuren oder deren Alkylestern mit 1 bis 4 Kohlenstoffatomen im Alkylrest wie die Halogenwasserstoffsäure, Salpetersäure, Schwefelsäure und Alkylsulfate sind indessen bevorzugt. Geeignet sind z. B. die Halogenanionen wie $J^-$, $Br^-$ und insbesondere $Cl^-$, Methylsulfat ($CH_3SO_4^-$), Aethylsulfat ($C_2H_5SO_4^-$), Toluolsulfonat, Acetat, Nitrat und Sulfat. Hierbei stehen Stearat, Aethylsulfat und Sulfat, vor allem Laurat, Methylsulfat und Bromid und insbesondere Chlorid im Vordergrund des Interesses. Ammoniumsalze mit Tensidanionen oder Anionen von Fettsäuren der angegebenen Art sin z. B. in Methanol, Aethanol und Isopropanol und deren Gemische in Wasser sowie in wässrigen Tensidsystemen löslich. Ammoniumsalze mit Anionen von Mineralsäuren oder niedermolekularen organischen Säuren der angegebenen Art sind wasserlöslich.

Die Herstellung der Ammoniumsalze kann nach bekannten Methoden erfolgen, indem man z. B. entweder Diaminsalze mit entsprechenden Diepoxyden oder Diepoxydgemischen oder Diamine mit entsprechenden Dihalogenhydrinen oder Dihalogenhydringemischen, die durch Hydrolyse der genannten Diepoxyde oder Diepoxydgemische mit einem Halogenwasserstoff vorgebildet worden sind, in Molverhältnissen von etwa 1 : 2 bis 2 : 1, vorzugsweise von 1 : 0,9 bis 1 : 1, 1 und insbesondere in äquimolaren Mengen umsetzt, und die erhaltenen Umsetzungsprodukte oder Umsetzungsproduktgemische mit einem Säureanhydrid, oder Säureanhydridgemisch in den genannten Molverhältnissen zu den entsprechenden Estern oder Estergemischen gegebenenfalls weiter umsetzt.

So kann man die Verbindungen mit Einheiten der Formel (1) so herstellen, dass man ein Diepoxyd oder ein Diepoxydgemisch einer der Formeln

(30.1) 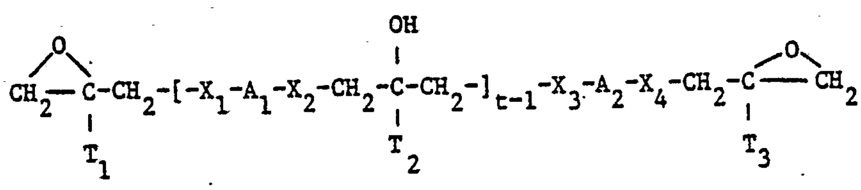 ,

(30.2) 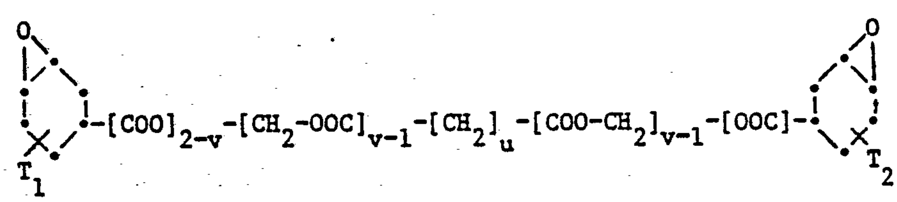 ,

(30.3) 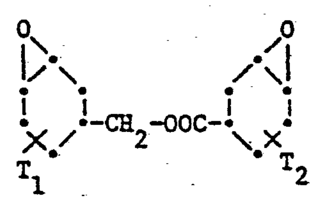 ,

(30.4) 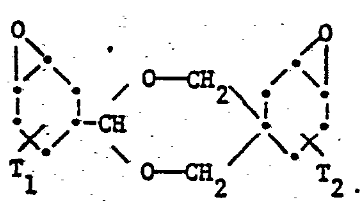 oder

(30.5) 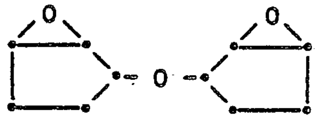

mit einem ditertiären Aminsalz der Formel

(31) 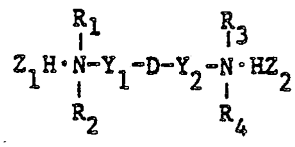

umsetzt, oder ein Diepoxyd oder Diepoxydgemisch einer der Formeln (30.1) bis (30.5) mit einem Halogenwasserstoff der Formel

(32) $Z_3H$

in ein Dihalogenhydrin oder Dihalogenhydringemisch einer der Formeln

(33.1) 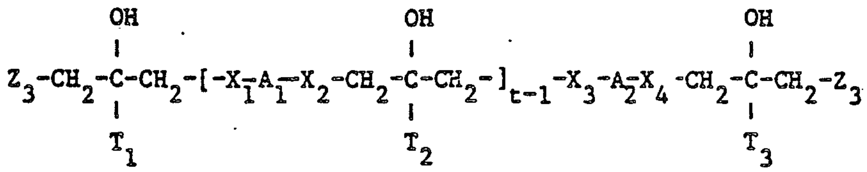 ,

(33.2) 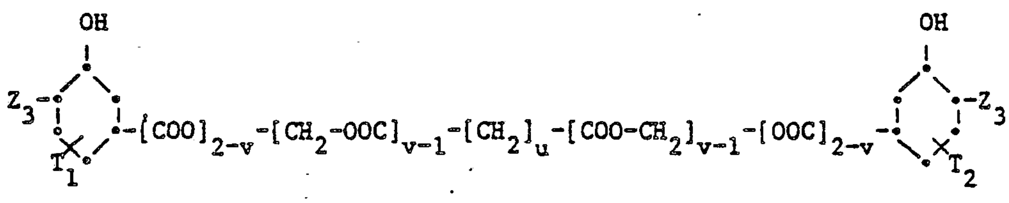 ,

(33.3) 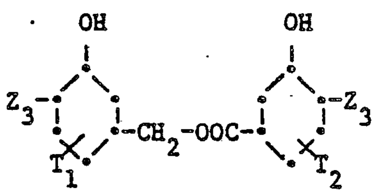 ,

(33.4) 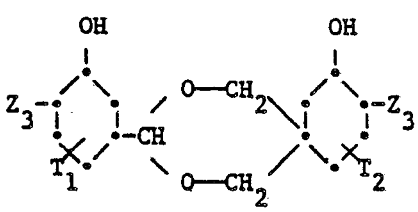 oder

(33.5) 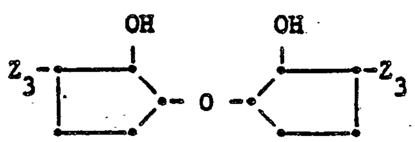

überführt und anschliessend mit einem ditertiären Amin der Formel

(34) 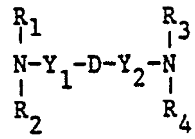

umsetzt, und die erhaltenen Umsetzungsprodukte oder Umsetzungsproduktgemische aus (30.1) bis (30.5) und (31) oder aus (33.1) bis (33.5) und (34), die Einheiten einer der Formeln

(35.1) 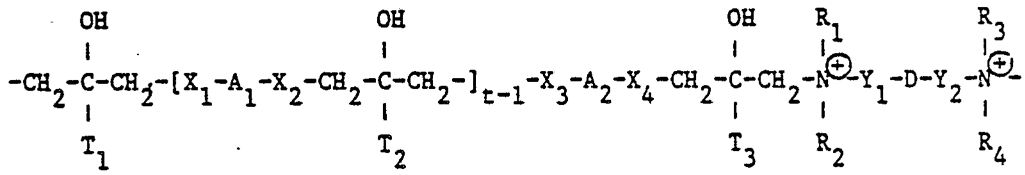 ,

(35.2) 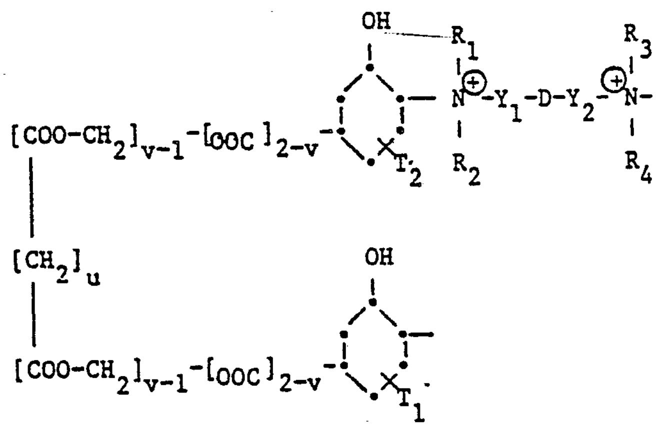 ,

(35.3) 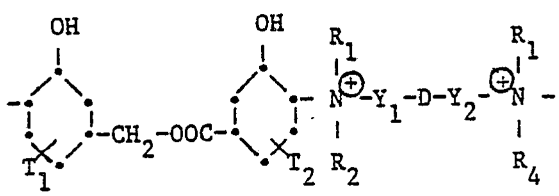 ,

(35.4) 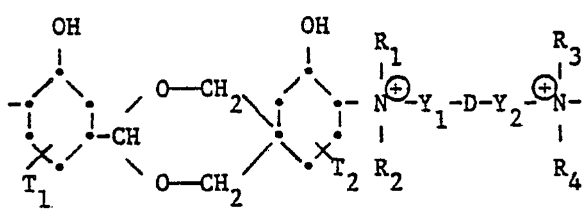 oder

(35.5) 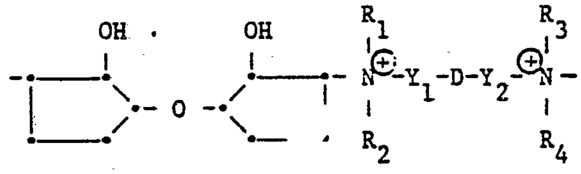

aufweisen, mit einem Säureanhydrid oder Säureanhydridgemisch der Formeln

(36.1) $M_6-OC-O-CO-M_6$,

(36.2) $M_7-OC-O-CO-M_7$ und/oder

(36.3) $M_8-OC-O-CO-M_8$

zu den entsprechenden Estern, die Einheiten einer der Formeln

(37.1) 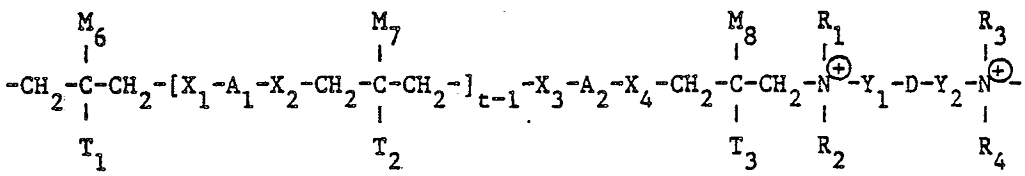 ,

(37.2) 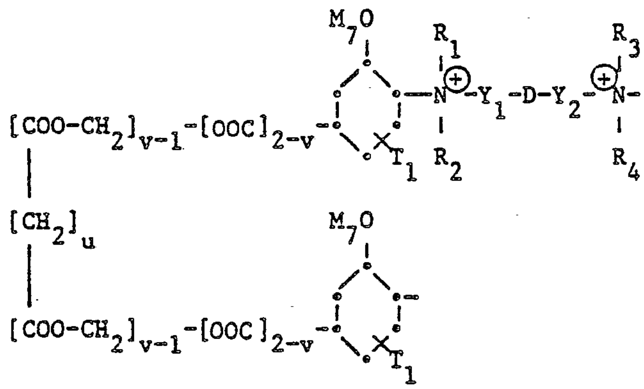

(37.3) 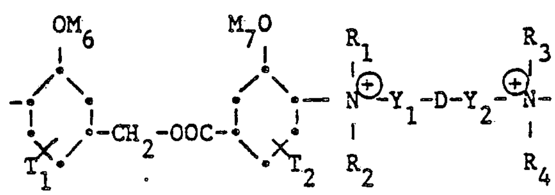 ,

(37.4) 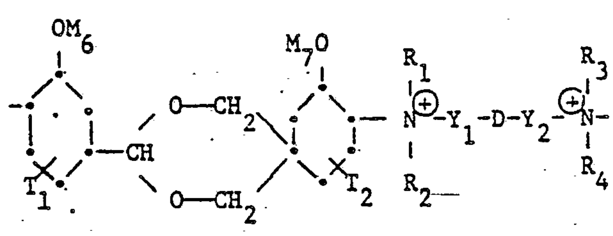 oder

(37.5) 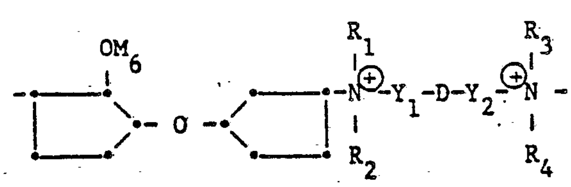

aufweisen,

gegebenenfalls weiter umsetzt, wobei $Z_1$ und $Z_2$ je ein Tensidanion, ein Fettsäureanion, ein Anion von niedermolekularen Säuren oder Mineralsäuren der vorstehend angegebenen Art oder vorzugsweise Halogen, $Z_3$ Halogen und $M_6$, $M_7$ und $M_8$ je Acyl mit 2 bis 5 Kohlenstoffatomen bedeuten und $A_1$, $A_2$, D, $R_1$, $R_2$, $R_3$, $R_4$, $T_1$, $T_2$, $T_3$, $X_1$, $X_2$, $X_3$, $X_4$, $Y_1$, $Y_2$, T, u und v die in Formel (1) angegebenen Bedeutungen haben.

Als Halogen für $Z_1$, $Z_2$ und $Z_3$ in den Formeln (31), (32) und (33.1) bis (33.5) steht Chlor im Vordergrund des Interesses.

Als Ausgangsstoff zur Herstellung der erfindungsgemässen Ammoniumsalze wird ein Epoxid oder Epoxydgemisch eingesetzt, das entweder der Formel (30.1) oder der Formel (30.2), bzw. (30.3), (30.4) und (30.5) entspricht. Als Epoxydgemische kommen somit Gemische aus Verbindungen der Formel (30.1) mit Verbindungen der Formeln (30.2), bzw. (30.3), (30.4) und (30.5) nicht in Frage. Entsprechend kommen als Endprodukte nur Umsetzungsprodukte oder Umsetzungsproduktgemische mit Einheiten einer der Formeln (35.1) bis (35.5) bzw. (37.1) bis (37.5) und nicht Gemische von Einheiten der Formeln (35.1) mit z. B. (35.2) bis (35.5) oder (37.1) mit z. B. (37.2) bis (37.5) in Frage.

Um die Produkte oder Produktgemische, die Einheiten einer der Formeln (35.1) bis (35.5) aufweisen, zu erhalten, ist die einstufige Umsetzung der Verbindungen einer der Formeln (30.1) bis (30.5) mit den Verbindungen der Formel (31) gegenüber der zweistufigen Umsetzung der Verbindungen einer der Formeln (30.1) bis (30.5) mit der Verbindung der Formel (32) zur Verbindungen einer der Formeln (33.1) bis (33.5), die anschliessend mit den Verbindungen der Formel (34) umgesetzt werden, bevorzugt. Die Umsetzung der Produkte, die Einheiten einer der Formeln (35.1) bis (35.5) aufweisen, mit den Verbindungen der Formeln (36.1), (36.2) und/oder (36.3) ist fakultativ und wird nur durchgeführt, sofern $M_1$, $M_2$ und/oder $M_3$ in Formeln (1.1) bis (1.5) Acyl bedeuten.

In einer bevorzugten Ausführungsart des erfindungsgemässen Herstellungsverfahrens wird das Diepoxyd oder Diepoxydgemisch der Formel (30.1) mit dem Diamin der Formel (31), worin $Z_1$ und $Z_2$ je Halogen bedeuten, umgesetzt, oder ein Diepoxyd oder Diepoxydgemisch der Formel (30.1) mit dem Halogenwasserstoff der Formel (32) in ein Dihalogenhydrin oder Dihalogenhydringemisch der Formel (33.1) überführt und anschliessend mit einem ditertiären Amin der Formel (34) umgesetzt, wobei die erhaltenen Umsetzungsprodukte oder Umsetzungsproduktgemische aus (30.1) und (31) oder aus (33.1) und (34), die Einheiten der Formel (35.1) aufweisen, mit einem Säureanhydrid oder Säureanhydridgemisch der Formeln (36.1), (36.2) und/oder (36.3) zu den entsprechenden Estern, die Einheiten der Formel (37.1) aufweisen, gegebenenfalls weiterumsetzt.

Die Ausgangsverbindungen (Diamine, Diepoxyde) zur Herstellung der erfindungsgemässen quaternären Ammoniumsalze sind im allgemeinen bekannte, durch chemische Synthese leicht zugängliche Verbindungen.

Mit der bevorzugten ein- oder zweistufigen Umsetzung der Verbindungen einer der Formeln (30.1) bis (30.5) mit den Verbindungen der Formel (31) und gegebenenfalls (36.1) bis (36.3) werden Ammoniumsalze mit kationischen Einheiten der Formel (1) bzw. einer der Formeln (35.1) bis (35.5) oder (37.1) bis (37.5) erhalten, welche als Gegenion Tensidanionen, Fettsäureanionen, Anionen von niedermolekularen Säuren der vorstehend angegebenen Art oder Halogenanionen aufweisen, während mit den zwei- oder dreistufigen Umsetzung der Verbindungen einer der Formeln (30.1) bis (30.5) mit den Verbindungen der Formeln (32) und (34) und gegebenenfalls der Verbindungen der Formeln (36.1) bis (36.3) Ammoniumsalze mit kationischen Einheiten der Formel (1) bzw. einer der Formeln (35.1) bis (35.5) oder (37.1) bis (37.5) erhalten werden, welche hingegen als Gegenion nur Halogenionen aufweisen.

Diamine mit dem Harnstoffbrückenglied (—HNCONH—) und einem Alkylenbrückenglied zu den tertiären Stickstoffatomen können durch Umsetzung von Harnstoff mit Alkylendiaminen, die ein tertiäres Stickstoffatom enthalten, bei höheren Temperaturen unter Abspaltung von Ammoniak erhalten werden. Eine andere Möglichkeit zur Herstellung der Diamine besteht in der Umsetzung der entsprechenden $\alpha,\omega$-Dihalogenverbindungen mit sekundären Aminen. Die Diamine mit einem Brückenglied, das sich von Dicarbonsäuren ableitet, können z. B. aus den Dichloriden oder Dialkylestern der Säuren und Alkylendiaminen hergestellt werden.

Diepoxyde mit zwei endständigen, an aliphatischen Ketten gebundene 1,2-Epoxydgruppen enthalten je nach Bedeutung von $T_1$, $T_2$ und $T_3$ Gruppen der Formel

(30.6) 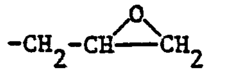 oder (30.7) 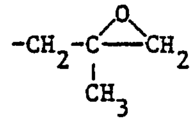 ,

welche mit den Sauerstoffatomen der Brückenglieder —COO—, —OOC— und —O—, die für $X_1$ und $X_2$ bzw. $X_3$ und $X_4$ stehen oder mit dem Stickstoffatom des Brückengliedes $A_1$ bzw. $A_2$, sofern $A_1$ bzw. $A_2$ einen 2 Stickstoffatome aufweisenden heterocyclischen Ring und $X_1$ und $X_2$ bzw. $X_3$ und $X_4$ die direkte Bindung bedeuten, verbunden sind. Die Diepoxyde sind somit je nach Bedeutung von $X_1$ und $X_2$ bzw. $X_3$ und $X_4$ sowie von $A_1$ bzw. $A_2$ O,O'-Diglycidyl- oder Di-(β-methylglycidyl)-ester oder -äther von zwei Carbonsäuregruppen pro Molekül enthaltenden oder zwei alkoholische oder phenolische Hydroxylgruppen pro Molekül enthaltenden aliphatischen, cycloaliphatischen oder aromatischen Verbindungen oder N,N'-Diglycidylverbindungen von zwei Stickstoffatome pro Molekül enthaltenden heterocyclischen Verbindungen. Diese Diglycidylverbindungen können durch Umsetzung der entsprechenden Carbonsäuregruppen, Hydroxylgruppen oder Stickstoffatome enthaltenden Verbindungen mit einem Epihalogenhydrin oder einem β-Methylhalogenhydrin in Gegenwart von Alkali oder auch in Gegenwart eines saueren Katalysators mit nachfolgender Alkalibehandlung erhalten werden und sind, wie eingangs erwähnt, besonders leicht zugänglich.

Cycloaliphatische Diepoxyde enthalten Gruppen der Formeln

(30.8) 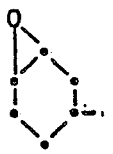 , (30.9) 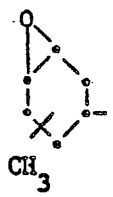 oder (30.10) 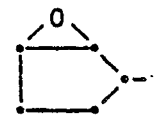

Die Diepoxyde mit Gruppen der Formel (30.8) oder (30.9) können durch Epoxydation der Doppelbindung von entsprechenden Tetrahydrobenzolderivaten mit z. B. Peressigsäure oder vorzugsweise unterchloriger Säure, bzw. Chlor in alkalischem Milieu erhalten werden, wobei die Tetrahydrobenzolderivate als Zwischenprodukte aus Butadien und Aldehyden wie Crotonaldehyd oder Acrolein leicht erhältlich sind und miteinander kondensiert, verestert oder mit Dicarbonsäuren oder Diolen weiter umgesetzt werden. Das Diepoxyd mit Gruppen der Formel (30.10) kann durch Epoxydation der Doppelbindungen wie vorstehend beschrieben eines Dicyclopentenyläthers erhalten werden, wobei dieser Aether aus Cyclopentadien leicht erhältich ist. Somit sind die cycloaliphatischen Diepoxyde der angegebenen Art auch besonders leicht zugänglich als Grossprodukte der Kunststoff-Industrie zur Herstellung von Epoxydharzen.

Die Herstellung der erfindungsgemässen Ammoniumsalze wird durch Umsetzung der Verbindungen der Formeln (30.1) bis (30.5) und (31) oder (33.1) bis (33.5) und (34) und gegebenenfalls der Verbindungen der Formeln (36.1) bis (36.3) bei etwa 20 bis 150 °C durchgeführt. In der Regel erfolgen die Umsetzungen z. B. in der Schmelze oder vorzugsweise in gegenüber den Reaktionspartnern inerten Lösungsmitteln, z. B. Alkoholen, Glykolen, Ketonen, wie Aceton oder cyclischen Aethern wie Dioxan oder Tetrahydrofuran. Von den Alkoholen sind die niederen Alkohole, vor allem Methanol, insbesondere Isopropanol und Aethanol bevorzugt. Weiter bevorzugt sind polare Lösungsmittel wie Aetheralkohole, insbesondere 1-Methoxy-2-äthanol. Die angegebenen Reaktionstemperaturen richten sich normalerweise nach den Siedepunkten der eingesetzten Lösungsmittel.

Gegebenenfalls kann man auch in Wasser oder Wasser-Alkoholmischungen als Lösungsmittel arbeiten.

Bei der fakultativen, weiteren Umsetzung der Umsetzungsprodukte bzw. Umsetzungsproduktgemische, die Einheiten einer der Formeln (35.1) bis (35.5) aufweisen, mit dem Säureanhydrid, bzw. Säureanhydridgemisch der Formeln (36.1) bis (36.3) ist es zweckmässig, einen Veresterungskatalysator, z. B. ein gegebenenfalls substituiertes Pyridin, vorzugsweise 4-Dialkylaminopyridine, insbesondere 4-Dimethylaminopyridin, einzusetzen.

Durch die bevorzugte Verwendung von billigen und leicht zugänglichen Dichlorverbindungen, d. h. von Verbindungen der Formeln (31) bzw. (33.1) bis (33.4), in welchen $Z_1$, $Z_2$ und $Z_3$ Chlor sind, bei der Herstellung der erfindungsgemässen quaternären Ammoniumsalze enthalten die Salze als Anionen bevorzugt Chlorionen. Die Einführung anderer Anionen kann vorzugsweise so vorgenommen werden, dass man in die Chlorionen enthaltenden Ammoniumsalze (Reaktionsprodukte) durch Ionenaustausch andere Anionen z. B. Tensidionen einführt. Es können aber auch, in einer weiteren Ausführungsart des Herstellungsverfahrens ditertiäre Aminsalze der Formel (31) eingesetzt werden, die andere Anionen als Halogenanionen, d. h. Tensidanionen, Fettsäureanionen oder Anionen niedermolekularer Säuren der vorstehend angegebenen Art bereits als Ausgangsstoffe aufweisen.

Die quaternären Ammoniumsalze, die Einheiten der Formel (1) enthalten, haben u. a. interessante kosmetische Eigenschaften, wenn sie in kosmetische Mittel aufgenommen werden, welche als solche verwendet und z. B. auf die Haut oder vorzugsweise auf die Haare aufgetragen werden, und die vorliegende Erfindung betrifft auch kosmetische Mittel, welche die Einheiten der Formel (1) aufweisende Ammoniumsalze enthalten, sowie Verfahren zur Herstellung der kosmetischen Mitteln und zur Behandlung von Haaren, insbesondere menschlichen Haaren, und die verfahrensgemäss behandelten Haaren, insbesondere Perücken. Diese kosmetischen Mittel enthalten im allgemeinen zusätzlich mindestens ein übliches in kosmetischen Mitteln verwendetes Hilfsmittel.

Die erfindungsgemässen kosmetischen Mittel können die Einheiten der Formel (1) aufweisende Ammoniumsalze entweder als aktiven Hauptbestandteil oder als Zusatz enthalten.

Diese kosmetischen Mittel können in Form von wässrigen, alkoholischen oder wässrig-alkoholischen Lösungen, wobei niedere Alkohole, wie Aethanol oder Isopropanol verwendet werden, oder als

Emulsionen, ferner cremeförmig, gelartig, aber auch pulverförmig oder in Tablettenform sowie als Aerosole, die zusätzlich ein Treibmittel enthalten, vorliegen.

Die erfindungsgemässen kosmetischen Mittel können ausserdem weitere in der Kosmetik gebräuchliche Zusätze enthalten, z. B. Konservierungsmittel, Riechstoffe, Farbstoffe, Lösungsmittel, Trübungsmittel und Perlglanzmittel, beispielsweise Ester von Fettsäuren mit Polyolen oder Dispersionen auf der Basis von Mischpolymeren ; Verdickungsmittel, ferner Pflanzenextrakte, Eiweissderivate, wie Gelatine, Collagenhydrolysate, Polypeptide auf natürlicher und synthetischer Basis, Lecithin, Lanolin und Lanolinderivate, Fette, Oele, Fettalkohole, Silicone, desodorierende Mittel, antimikrobiell wirksame Substanzen und Sequestriermittel.

Die erfindungsgemässen kosmetischen Mittel können sowohl als anwendungsfertige Mittel als auch Konzentrate die vor der Anwendung verdünnt werden missen, vorliegen.

Die kosmetischen Mittel für die Haut liegen beispielsweise in Form von Cremen, Gelen, Emulsionen oder wässrigen, alkoholischen oder wässrig-alkoholischen Lösungen vor.

Die in diesen kosmetischen Mitteln enthaltenen veiteren Zusätze sind beispielsweise Parfums, Farbstoffe, Konservierungsmittel, Sequestriermittel, Emulgiermittel und Verdickungsmittel.

Diese Mittel für die Haut stellen z.B. pflegende Cremes oder Lotionen für die Hände oder das Gesicht, Sonnenschutzcremes, getönte Cremes, schäumende Oele oder Flüssigkeiten für Bäder, oder auch deodorierende Präparate dar.

Diese Mittel werden nach den üblichen Methoden hergestellt, die durch Vermischen der Mittelkomponenten erfolgt.

Um beispielsweise eine Creme zu erhalten, kann man eine flüssige Phase, die Polymere und gegebenenfalls andere Zusätze enthält und eine ölige Phase emulgieren.

Die ölige Phase kann aus verschiedenen Produkten bestehen, wie beispielsweise Paraffinöl, Vaselineöl, Olivenöl, Fettsäureestern, wie Glycerinmonostearat, Aethyl- oder Isopropylpalmitaten, Alkylmyristaten, wie Propyl-, Butyl- oder Cetylmyristat. Man kann darüber hinaus Fettalkohole wie Cetylalkohol, oder Wachse, beispielsweise Bienenwachs, zugeben.

Die erfindungsgemässen quaternären Ammoniumsalze können in den kosmetischen Mitteln für die Haut entweder als Zusatz, oder als aktiver Haupbestandteil in pflegenden Cremes oder Lotionen für die Hände oder das Gesicht, oder auch als Zusatz in Sonnenschutzcremes, getönten Cremes, Abschminkmilch oder schäumenden Flüssigkeiten für Bäder vorliegen.

Im Vordergrund des Interesses stehen die kosmetischen Mittel für die Haarbehandlung.

Im allgemeinen beträgt die Konzentration der quaternären Ammoniumsalze, die Einheiten der Formel (1) aufweisen, in den erfindungsgemässen haarkosmetischen Mitteln 0,01 bis 10, vorzugsweise 0,5 bis 5 und insbesondere 0,8 bis 2,5 Gewichtsprozent. Beispiele haarkosmetischer Mittel sind Haarfestiger, Haarspülmittel, Haarregeneriermittel, Haarfärbemittel, Vorbehandlungsmittel, Haarwässer, Frisiercremes, Frisiergels, Haaröle, Haarpomaden oder Haarbrillantinen.

Bevorzugt ist die Verwendung der quaternären Ammoniumsalze, die Einheiten der Formel (1) aufweisen, als Haarlacke und Haarsprays, vor allem als Aerosol- und insbesondere als Pumpsprays, sowie als Shampoo und insbesondere als Einlegemittel oder Wasserwellotionen.

Haarfärbemittel enthalten neben den quaternären Ammoniumsalzen, die Einheiten der Formel (1) aufweisen, in der Regel noch einen Träger.

Der Träger wird bevorzugt so gewählt, dass man eine Creme oder ein Gel erhält.

Für eine Oxydationsfärbung kann das Färbemittel in zwei Teilen vorliegen, wovon der zweite Teil Wasserstoffperoxyd ist. Die beiden Teile werden vor der Anwendung vermischt.

Haarkosmetische Mittel zur Vorbehandlung, die in Form von wässrigen oder wässrig-alkoholischen Lösungen, gegebenenfalls in Aerosolflacons oder als Creme oder Gel vorliegen, können dazu verwendet werden, vor dem Schampoonieren, insbesondere vor dem Schampoonieren mit einem anionischen und/oder nicht-ionischen Schampoo, vor einer Oxydationsfärbung, der ein anionisches und/oder nichtionisches Schampoonieren folgt, oder auch vor einer Dauerwellbehandlung aufgetragen zu werden.

Bei diesen Vorbehandlungsmitteln ist das quaternäre Ammoniumsalz mit Einheiten der Formel (1) der aktive Bestandteil, und seine Konzentration schwankt im allgemeinen zwischen 0,1 und 10 insbesondere zwischen 0,2 und 5 Gewichtsprozent. Der pH-Wert dieser Mittel variiert im allgemeinen zwischen etwa 3 und 9.

Liegen die haarkosmetischen Mittel als Shampoos vor, so enthalten sie mindestens einen Waschrohstoff (Tensid), wobei anionische, kationische, nichtionische und amphotere Verbindungen infrage kommen können. Bevorzugt sind kationische oder nichtionische Verbindungen.

Beispiele für anionische Tenside sind bereits vorstehend bei der Definition der Tensidanionen erwähnt. Bevorzugt werden diese Tenside im Ueberschuss, bezogen auf die kationischen Einheiten der Formel (1) eingesetzt, so dass solche Tenside gleichzeitig als Gegenion zu den kationischen Einheiten der Formel (1) und als Waschrohstoff in Shampoos verwendet werden. Diese anionischen Tenside und deren Mischungen werden in Form ihrer wasserlöslichen oder in Wasser dispergierbaren Salze benutzt, beispielsweise in Form der vorstehend beschriebenen Aethersulfate, -sulfonate oder -phosphate.

Geeignete kationische Tenside sind beispielsweise quaternäre Ammonium-Salze wie Dialkyldimethyl-ammonium-chlorid oder -bromid, Alkyl-dimethyl-äthylammonium-chlorid oder -bromid ; Alkyltrimethyl-ammonium-chlorid oder -bromid, Alkyl-dimethyl-benzyl-ammonium-chlorid oder -bromid, N-Alkyl-pyridinium-chlorid oder -bromid ; Salze des N,N-Diäthylaminoäthyl-stearylamids und -oleylamids mit Salzsäure, Essigsäure, Phosphorsäure und N-Acylamidoäthyl-N,N-diäthyl-N-benzylammonium-chlorid, -bromid oder -monoalkysulfat, wobei Acyl vorzugsweise für die Reste der Stearin- oder Oelsäure steht.

Als nicht ionogene Tenside, die als waschaktive Substanzen eingesetzt werden können, kommen beispielsweise in Betracht : Fettalkoholäthoxylate, Alkylphenylpolyäthylenglykole ; Alkylaminopolyäthylenglykole ; Fettsäureäthoxylate ; Fettaminäthoxylate ; Polypropylenglykoläthoxylate, Fettsäureamidopolyäthylenglykole ; Saccharoseester oder Sorbitester. Aethoxylierte Fettamine, z. B. ein äthoxyliertes Talgfettamin sind als nicht ionogenes Tensid von besonderem Interesse.

Beispiele für amphotere Tenside sind z. B. Betaine, wie N-Acylamidoalkyl-N,N-dimethyl-acetobetain, vorzugsweise N-Acylamido-propyl-N,N-dimethyl-acetobetain oder Alkyl-dimethyl-sulfopropylbetain (Alkyl mit 12 bis 18 Kohlenstoffatomen) ; Amphotenside auf Basis Imidazolin, vorzugsweise das Natrium-Salz des 1-(β-Carboxymethyloxyäthyl)-1-(carboxymethyl)-2-lauryl-imidazoliniums ; Aminoxyde, z. B. Alkyldimethylamin-oxyde (Alkyl mit 12 bis 18 Kohlenstoffatomen).

Die haarkosmetischen Mittel in Form von Shampoos können beispielsweise zusätzlich Parfums, Farbstoffe, Konservierungsmittel, Verdickungsmittel, Schaumstabilisierungsmittel, Weichmacher oder ein kosmetisches Harz enthalten.

In der Regel enthalten diese Shampoos etwa 0,8 bis 2,5 Gewichtsteile mindestens eines quaternären Ammoniumsalzes, die Einheiten der Formel (1) aufweist, etwa 10 bis 20 Gewichtsteile mindestens eines anionischen, kationischen, nicht ionischen oder amphoteren Waschrohstoffes der vorstehend angegebenen Art und sind mit Wasser, vorzugsweise deionisiertem Wasser auf insgesamt 100 Gewichtsteile verdünnt.

Haarlacke als haarkosmetische Mittel liegen in der Regel als alkoholische oder wässrige-alkoholische Lösung eines für Lacke gebräuchlichen kosmetischen Harzes und mindestens eines quaternären Ammoniumsalzes, die Einheiten der Formel (1) enthalten, vor. Sie werden als Aerosolsprays mit einem Treibmittel aus einer Aerosolbombe oder vorzugsweise als Pumpsprays appliziert.

Falls die haarkosmetischen Mittel als Aerosolspray vorliegen, sind sie in der Regel wasserfrei und enthalten Aethanol oder Isopropanol als Lösungsmittel und zusätzlich ein Treibmittel. Als Treibmittel kommen vor allem fluorierte und gegebenenfalls chlorierte Kohlenwasserstoffe mit 1 bis 4, vorzugsweise 1 oder 2 Kohlenstoffatomen wie Octafluorbutan, Tetra- und Trifluormethan, z. B. Dichlortetrafluoräthan, Dichlordifluoräthan und insbesondere Trichlorfluormethan und Dichlordifluormethan, ferner auch niedere Alkane wie Propan, Isobutan und insbesondere Butan in Betracht. Vorzugsweise werden Gemische der aufgezählten Verbindungen eingesetzt. Auch Kohlendioxyd kann als Treibgas erfolgreich eingesetzt werden. Letzteres und insbesondere die niederen Alkane werden den fluorierten Kohlenwasserstoffen gegenüber als Treibmittel bevorzugt. Bei der Applikation als Aerosolsprays sind Ammoniumsalze, die als Gegenion zu den kationischen Einheiten der Formel (1) Tensidanion der vorstehend angegebenen Art aufweisen, wegen ihrer hohen Löslichkeit in Aethanol oder Isopropanol besonders bevorzugt.

In der Regel enthalten die Aerosolsprays etwa 0,1 bis 2,5 Gewichtsteile mindestens eines quaternären Ammoniumsalzes, die Einheiten der Formel (1) aufweist, etwa 15 bis 65 Gewichtsteile mindestens eines Treibgases der angegebenen Art und etwa 30 bis 70 Gewichtsteile Aethanol und/oder Isopropanol, sodass insgesamt 100 Gewichtsteile vorhanden sind.

Wasserwell-Lotionen als haarkosmetische Mittel sind insbesondere für empfindlich gewordene Haare geeignet und enthalten mindestens ein quaternäres Ammoniumsalz, das Einheiten der Formel (1) aufweist, in der Regel in wässriger oder wässrig-alkoholischer Lösung.

Sie können darüber hinaus eines oder mehrere kosmetische Harze, insbesondere Homopolymere oder Vinylcopolymere, wie beispielsweise Polyvinylpyrrolidon, die Mischpolymerisate von Vinylpyrrolidon und Vinylacetat, Mischpolymerisate von Crotonsäure und Vinylacetat oder quaternäre Stickstoffatome aufweisende Celluloseäther enthalten, wie sie z. B. aus der US-Patentschrift 3 472 840 bekannt sind.

Falls diese Lotionen oder Einlegemittel als wässrige Lösungen oder als wässrig-alkoholische Lösungen vorliegen, die weniger als 5 bis 10 Gewichtsprozent eines Alkohols aufweisen, enthalten sie zweckmässigerweise ein Konservierungsmittel, z. B. ein p-Oxybenzoesäure-alkylester, vorzugsweise p-Oxybenzoesäure-methylester.

Der pH dieser Wasserwell-Lotionen liegt im allgemeinen zwischen 4,5 und 7,5. Gegebenenfalls kann man den pH-Wert, beispielsweise durch Zusatz eines Alkanolamins, wie Monoäthanolamin oder Triäthanolamin oder einer Säure wie Salz- oder Phosphorsäure, oder vorzugsweise Essig-, Milch- oder Zitronensäure verändern.

Falls die haarkosmetischen Mittel als Einlegemittel bzw. Wasserwell-Lotionen vorliegen, enthalten sie Wasser und gegebenenfalls Aethanol oder Isopropanol als Lösungsmittel. Vorzugsweise enthalten die Einlegemittel etwa 0,8 bis 25 Gewichtsteile mindestens eines quaternären Ammoniumsalzes, das Einheiten der Formel (1) aufweist, 0 bis etwa 70 Gewichtsteile Aethanol und/oder Isopropanol und sind mit etwa 30 bis 99 Gewichtsteilen Wasser auf insgesamt 100 Gewichtsteile verdünnt.

Falls die haarkosmetischen Mittel als Pumpspray vorliegen, enthalten sie Aethanol oder Isopropanol und gegebenenfalls Wasser als Lösungsmittel. Vorzugsweise enthalten die Pumpsprays etwa 0,8 bis 2,5 Gewichtsteile mindestens eines quaternären Ammoniumsalzes, das Einheiten der Formel (1) aufweist,

0 bis etwa 10 Gewichtsteile Wasser und sind auf insgesamt 100 Gewichtsteile mit etwa 87 bis 99 Gewichtsteilen Aethanol und/oder Isopropanol verdünnt.

Im erfindungsgemässen Herstellungsverfahren der haarkosmetischen Mittel wird mindestens ein quaternäres Ammoniumsalz, das Einheiten der Formel (1) enthält, mit den übrigen Mittelkomponenten, z. B. ein Lösungsmittel der angegebenen Art wie Aethanol, Isopropanol und/oder Wasser, gegebenenfalls ein Waschrohstoff oder ein Treibmittel und/oder weitere Zusätze der angegebenen Art vorzugsweise bei Raumtemperatur miteinander vermischt.

Das erfindungsgemässe, kosmetische Behandlungsverfahren besteht darin, dass man eine Lösung auf Haare aufbringt, die Aethanol, Isopropanol und/oder Wasser als Lösungsmittel und mindestens 0,01 Gewichtsprozent mindestens eines quaternären Ammoniumsalzes, das Einheiten der Formel (1) aufweist, enthält. Hierbei wird die Lösung z. B., in Form eines Aerosolsprays oder vorzugsweise eines Pumpsprays, auf die Haare aufgesprüht oder die Haare werden in die Lösung eingelegt.

Beim Eintauchen, Einlegen oder Waschen der Haare mit den haarkosmetischen Mitteln in Form von Einlegemittel oder Shampoos, werden diese Mittel bei physiologisch verträglichen Temperaturen von vorzugsweise 20 bis 45 °C auf das Haar aufgebracht. Die Einlegemittel werden auf die Haare vorzugsweise so aufgebracht, dass man mit Wasser angenetzte Haare bei 20 bis 45 °C imprägniert, auf Lockenwickler aufrollt und bei 20 bis 60 °C trocknet. Die Shampoos werden bei 20 bis 45°, vorzugsweise 20 bis 40 °C auf die Haare aufgebracht, dann lässt man vorzugsweise das Shampoo während etwa 0,5 bis 5 Minuten auf das Haar einwirken. Anschliessend werden die Haare bei 20 bis 45 °C ausgewaschen, zur Bildung von Locken aufgerollt und bei etwa 20 bis 60 °C getrocknet. Die Menge Shampoo beträgt in der Regel 2 mal 5 bis 10 ml pro Kopf.

Menschliches Haar, das mit einem Shampoo, das die quaternären Ammoniumsalze, die Einheiten der Formel (1) aufweisen, enthält, gewaschen und anschliessend gespült wird, ist einerseits im nassen Zustand sehr gut kämmbar und kann andererseits zur Bildung von Locken direkt — d. h. ohne Verwendung eines zusätzlichen Einlegemittels — auf Lockenwickler aufgerollt und dann an der Luft oder mit einem Föhn getrocknet werden. Nach dem Entfernen der Lockenwickler ist das Haar sehr gut frisierbar, es zeigt Fülle und natürlichen Glanz sowie sehr gute Festigkeit, insbesondere auch in feuchtem Klima. Die Shampoos zeigen also einen doppelten Effekt, indem sie einmal zur Reinigung des Haars dienen und ausserdem dem gewaschenen Haar Eigenschaften verleihen, die ein leichtes Einlegen ermöglichen ohne dass ein spezielles Einlegemittel angewendet werden muss (Einlege-Shampoo).

Beim Aufbringen der haarkosmetischen Mittel in Form von Aerosolsprays oder Pumpsprays werden die Haare bei Raumtemperatur besprüht und anschliessend vorzugsweise bei Raumtemperatur getrocknet. Haare aus natürlichen oder synthetischen Fasern, vorzugsweise menschliche Haare in Form von z. B. Perücken oder menschliches Haar in vivo können nach dem erfindungsgemässen Verfahren behandelt werden.

Die erfindungsgemässen haarkosmetische Mittel ergeben, nachdem sie auf die Haare appliziert worden sind, nach dem Trocknen einen festen und kontinuierlichen Film, der flexibel, nicht spröde, nicht ölig, nicht schmierig, farblos und durchsichtig und daher auch unsichtbar ist, welcher dem behandelten Haar eine ausgezeichnete Festigkeit verleiht. Die erhaltenen Filme auf dem Haar können mit Wasser, durch kräftiges Brüsten, oder vorzugsweise mit Haarshampoos mühelos wieder entfernt werden. Ueberraschenderweise hat aber die gute Auswaschbarkeit der Filme keinen negativen Einfluss auf die gute Formbeständigkeit der behandelten Haare, selbst bei hoher Luftfeuchtigkeit.

Die Ammoniumsalze, die kationische Einheiten der Formel (1) aufweisen, sind als Auswaschmittel für kationische Färbungen, d. h. für Textilmaterialien, die mit kationischen Farbstoffen gefärbt oder bedruckt sind, besonders gut geeignet.

Somit bildet ein weiterer Gegenstand der vorliegenden Erfindung ein Verfahren zum Auswaschen in wässrigem Medium von Textil-material, das mit mindestens einem kationischen Farbstoff bedruckt oder gefärbt ist, wobei bedrucktes oder gefärbtes Material zusammen mit unbedrucktem oder ungefärbtem Material oder Materialien, die jeweils mit verschiedenartigen, kationischen Farbstoffen bedruckt oder gefärbt sind, oder ein einzelnes, stellenweise bedrucktes Material ausgewaschen werden, dadurch gekennzeichnet, dass das Waschwasser mindestens ein erfindungsgemässes Ammoniumsalz enthält, das kationische Einheiten der Formel (1) aufweist.

Das erfindungsgemäss ausgewaschene Textilmaterial, die wässrige Flotte zur Durchführung des erfindungsgemässen Verfahrens, die mindestens ein Ammoniumsalz der angegebenen Art enthält, stellen weitere Gegenstände der vorliegenden Erfindung dar.

Zweckmässig setzt man in der wässrigen Waschflotte 0,05 bis 1,0 g, vorzugsweise 0,5 bis 0,8 g mindestens eines der erfindungsgemässen Ammoniumsalze als reine Wirksubstanz pro Liter Waschwasser ein. Das Auswaschen wird vorzugsweise bei erhöhten Temperaturen, z. B. bis zu 100 °C, insbesondere 60 bis 75 °C, durchgeführt. Bei hohen Temperaturen darf die Waschzeit kürzer gehalten werden. Sie beträgt im allgemeinen 5 bis 60 Minuten, insbesondere 10 bis 20 Minuten, falls das Auswaschen bei 60 bis 75 °C durchgeführt wird.

Als bedruckte oder gefärbte Textilmaterialien, die erfindungsgemäss ausgewaschen werden, kommen vor allem textile Fasern, d. h. insbesondere synthetische Fasern für sich allein oder Mischungen aus synthetischen und natürlichen Fasern in Betracht, sofern diese Fasern mit kationischen Farbstoffen anfärbbar sind. Auch Mischungen von verschiedenen synthetischen Fasern kommen in Frage.

Diese Fasermaterialien liegen in den verschiedensten Verarbeitungszuständen, z. B. als Kabel, Kammzüge, Fäden, Garne, Gewebe, Gewirke, Non-Woven-Artikel, oder fertige Kleidungsstücke vor.

Als mit kationischen Farbstoffen anfärbbaren Textilmaterialien aus natürlichen Fasern kommen u. a. solche aus cellulosischen Materialien, insbesondere aus Baumwolle in Betracht, während die Textilmaterialien aus synthetischen Fasern, z. B. solche aus hochmolekularen, modifizierten Polyestern, wie Polyäthylenterephthalat oder Polycyclohexandimethylenterephthalat, aus modifizierten Polyamiden, wie Polyhexamethylendiaminadipat, Poly-ε-caprolactam oder Poly-ω-aminoundecansäure, aus Polyacrylnitrilen oder Acrylnitrilmischpolymerisaten, ausserdem aus Viskose, Kunstseide, Cellulose-2 1/2-acetat und Cellulosetriacetat sind.

Vor allem bedruckte oder gefärbte Fasern aus Cellulose, vorzugsweise Baumwolle, ferner aber auch regenerierter Cellulose wie Viskose, aus modifizierten, kationisch färbbaren Polyester oder Polyamid und insbesondere aus Modacryl oder Polyacrylnitril sind besonders geeignet, erfindungsgemäss ausgewaschen zu werden, wobei die schnellziehenden Fasern auch in Betracht kommen. Modifizierte Polyester und Polyamidfasern sind z. B. in Teintures et Apprêts *144*, Seiten 163 bis 167 (1974) beschrieben. Schnellziehende Polyacrylnitrilfasern sind z. B. in Melliand Textilberichte 12 (1968), Seiten 1436 bis 1443, in J. Soc. Dyers and Colourists, May 1971, Seiten 149 bis 155 und Febr. 1978, Seiten 49 bis 52, in Teintex 5 (1973), Seiten 281 bis 296, in Chemiefaserntextilind. Mai 1974, Seiten 391 bis 396 und Jan. 1974, Seiten 52 bis 60 beschrieben.

Bei den Modacrylfasern handelt es sich um Fasern aus Acrylnitrilmischpolymerisaten, für deren Herstellung ausser Acrylnitril auch noch andere Vinylverbindungen, z. B. Vinylchlorid, Vinylacetat, Vinylidenchlorid, Vinylidencyanid und Acrylsäurealkylester, verwendet worden sind, sofern der Anteil dieser anderen Vinylverbindungen nicht höher als 20 Prozent, bezogen auf das Gewicht der Materialien, beträgt.

Vor allem bedruckte oder gefärbte Polyacrylnitrilgewebe oder -gewirke sind besonders geeignet, nach dem erfindungsgemässen Verfahren ausgewaschen zu werden.

Vor dem Auswaschen gemäss erfindungsgemässem Verfahren werden die Textilmaterialien nach konventionellen Methoden bedruckt oder gefärbt, wobei färberische Zubereitungen eingesetzt werden, die als wässrige und/oder organische Lösungen oder Dispersionen oder als Druckpasten oder -tinten vorliegen, die neben einem Farbstoff noch weitere Zusätze, z. B. Säuren, Salze, Harnstoff und weitere Hilfsmittel wie Oxalkylierungsprodukte von Fettaminen, Fettalkoholen, Alkylphenolen, Fettsäuren und Fettsäureamiden enthalten. Die kationischen Farbstoffe, die hierbei zur Anwendung kommen, gehören den verschiedenartigsten Gruppen an. Geeignete Farbstoffe sind z. B. Di- und Triphenylmethanfarbstoffe, Rhodaminfarbstoffe und Oniumgruppen enthaltende Azo- oder Anthrachinonfarbstoffe, ferner Thiazin-, Oxazin-, Methin- und Azomethinfarbstoffe. Diese kationischen Farbstoffe sind z. B. in Colour Index, 3. Auflage (1971), Band 1, unter der Rubrik « Basic dyes » beschrieben.

Erfindungsgemäss können wie bereits angedeutet bedrucktes oder gefärbtes Textilmaterial zusammen mit unbedrucktem oder ungefärbtem Textilmaterial ausgewaschen werden. Hierbei wird das Anschmutzen des unbedruckten oder ungefärbten Textilmaterials mit den aus dem bedrucktem oder gefärbtem Material ausgewaschenen Farbstoffen dadurch verhindert, dass das Waschwasser gemäss erfindungsgemässen Verfahren ein Ammoniumsalz der angegebenen Art enthält. Auch beim erfindungsgemässen Auswaschen von mehreren Materialien, die jeweils mit verschiedenartigen kationischen Farbstoffen bedruckt oder gefärbt sind, z. B. von blau bedrucktem Material zusammen mit gelb bedrucktem Material, wird das gegenseitige Anschmutzen des Materials mit einer unerwünschten Komplementärfärbung, z. B. Grünfärbung, verhindert. Vorzugsweise wird erfindungsgemäss bedrucktes Textilmaterial, und insbesondere ein einzelnes stellenweise bedrucktes Textilmaterial ausgewaschen, das sogenannte Bunt- oder vor allem Weissreserven aufweist. Durch die Gegenwart von Ammoniumsalzen im Waschwasser beim Auswaschen des stellenweise bedruckten Textilmaterials gemäss erfindungsgemässem Verfahren wird eine unerwünschte Wiederanfärbung der Bunt- oder Weissreserven vollständig ausgeschaltet. Zudem werden die Wasch- und Lichtechtheiten des gefärbten oder bedruckten Textilmaterials erhöht.

Die polymeren Ammoniumsalze mit kationischen Einheiten der Formel (1) haben ebenfalls interessante Eigenschaften als Papierleimungsmittel. Somit bilden weitere Gegenstände der Erfindung ein Verfahren zur Massen- und insbesondere zur Oberflächenleimung von Papier unter Einsatz der erfindungsgemässen Ammoniumsalze und das nach diesem Verfahren geleimte Papier.

Vor ihrer Verwendung als Papierleimungsmittel werden die erfindungsgemässen Ammoniumsalze mit Wasser so verdünnt, dass wässrige Leimflotten entstehen, deren Gehalt, berechnet auf reine Wirksubstanz, 0,02 bis 1, vorzugsweise 0,02 bis 0,2 Gewichtsprozent beträgt. Bei der Massenleimung werden 0,3 bis 4 Gewichtsprozent des Ammoniumsalzes, berechnet auf reine Wirksubstanz und bezogen auf den Feststoffgehalt des herzustellenden Papiers unter Einschluss von Bindemitteln, wie etwa Stärke, in Form der verdünnten Leimflotte vor dem Stoffauflauf der Papiermaschine in die Faserstoffsuspension eingearbeitet. Bei der bevorzugten Oberflächenleimung wird das Papier mit der verdünnten Leimflotte im Allgemeinen bei Raumtemperatur z. B. durch Aufsprühen oder insbesondere durch Foulardieren imprägniert, und anschliessend bei 60 bis 140°, vorzugsweise 90 bis 110 °C während 0,1 bis 10, vorzugsweise 1 bis 6 Minuten getrocknet. Nach dem Trocknen werden Flächenaufträge der Ammoniumsalze, berechnet als reine Wirksubstanz, von 50 bis 150, vorzugsweise 60 bis 120 mg/m$^2$ erhalten.

Mit bereits geringen Mengen an erfindungsgemässen Ammoniumsalzen, können auf Papier sehr gute Leimungseffekte erzielt werden, die aufgrund von positiven Testergebnissen wie Wasseraufnahme nach Cobb und alkalische Tintenschwimmdauer bestätigt werden. Insbesondere ermöglichen bei der bevorzugten Oberflächenleimung die geringen Flächenaufträge eine rasche Arbeitsweise, so dass bei einer Trocknungstemperatur von z. B. 90 bis 110 °C bereits innerhalb etwa 20 bis 40 Sekunden gute Leimungen erzielt werden. Zudem neigen in der Regel Ammoniumsalze nicht zu einer unerwünschten Schaumbildung.

Dank ihrer kationischen Einheiten der Formel (1) sind die erfindungsgemässen Ammoniumsalze ebenfalls geeignet für ihren Einsatz als Fixierungsmittel beim Färben von cellulosehaltigen Materialien mit zweckmässigen anionischen Farbstoffen. Ein Verfahren zum Nachbehandeln von cellulosehaltigen Materialien, die mit anionischen Farbstoffen gefärbt sind, mittels der erfindungsgemässen Ammoniumsalze, vorzugsweise in Form ihrer wässrigen, verdünnten Lösung bildet somit einen weiteren Erfindungsgegenstand. Als Materialien kommen vor allem Fasermaterialien in Betracht, z. B. Textilfasern in den verschiedensten, vorstehend angegebenen Verarbeitungszuständen, die gegebenenfalls im Gemisch mit synthetischen Fasern aus z. B. Polyester, Polyamid, Polyacryl, wie etwa Baumwoll-Polyester-Mischgewebe, jedoch vorzugsweise aus textilen Fasern aus gegebenenfalls regenerierter Cellulose für sich allein, wie Viskose, Kunstseide und vorzugsweise Baumwolle vorliegen. Bevorzugte cellulosehaltige Materialien sind indessen Holz und insbesondere Karton oder Papier. Bei den eingesetzten anionischen Farbstoffen handelt es sich vor allem um wasserlösliche, substantive, direktziehende Farbstoffe. Solche Farbstoffe sind u. a. im Kapitel « Direktfarbstoffe » im Lehrbuch « Künstliche organische Farbstoffe und ihre Zwischenprodukte » von H. R. Schweizer, Springer Verlag Berlin (1964), Seite 481 bis 495, beschrieben. Unter diesen Farbstoffen kommen vor allem Azofarbstoffe, insbesondere Disazo- oder Trisazofarbstoffe, welche Wasserlöslichkeit verleihende, anionische Sulfonsäuregruppen enthalten, in Betracht. Den Triazinylfarbstoffen kommt hierbei eine besondere Bedeutung zu.

In der im Vordergrund des Interesses stehenden Ausführungsart des erfindungsgemässen Färbeverfahrens wird das Papier vorzugsweise in der Masse, d. h. in die Faserstoffsuspension vor ihrer Aufarbeitung zu fertigen Papiererzeugnissen gefärbt. Zu diesem Zweck werden in der Regel der Faserstoffsuspension 0,01 bis 1, vorzugsweise 0,02 bis 1, insbesondere 0,08 bis 0,7 Gewichtsprozent der vorstehend erwähnten Farbstoffe, berechnet als wasserfreien und coupagefreien Farbstoff, bezogen auf den Trockenfasergehalt der Faserstoffsuspension, und 0,05 bis 3, vorzugsweise 0,2 bis 0,8, insbesondere 0,4 bis 0,8 Gewichtsprozent der erfindungsgemässen Ammoniumsalze, berechnet als reine Wirksubstanz, bezogen auf den Trockenfasergehalt der Faserstoffsuspension, zugegeben.

Im Verfahren zum Färben von Papier in der Masse wird zuerst der Farbstoff und dann das Ammoniumsalz in die Faserstoffsuspension gegeben, wobei die Zugabe des Farbstoffes vorzugsweise 20 bis 120, insbesondere 30 bis 40 Minuten und die Zugabe des Ammoniumsalzes 3 bis 300, vorzugsweise 10 bis 120, insbesondere 5 bis 45 Sekunden vor dem Stoffauflauf der Papiermaschine erfolgt.

Die Faserstoffsuspension, in welche die Farbstoffe und Ammoniumsalze zugegeben werden, ist im allgemeinen neutral bis schwach alkalisch oder schwach sauer, weist in der Regel einen pH-Wert von 6 bis 8 oder von 7 bis 8, einen Trockenfasergehalt von 0,1 bis 4,5, vorzugsweise 0,6 bis 1,5, insbesondere 0,2 bis 1,2 Gewichtsprozent und einen Schopper-Riegler-Mahlgrad von 20 bis 60°, vorzugsweise 30 bis 50°, insbesondere 25 bis 35° auf und enthält in der Regel Sulfitzellstoff, vorzugsweise Nadelholzsulfitzellstoff, Sulfatzellstoff, vorzugsweise Buchensulfatzellstoff und gegebenenfalls Holzschliff.

Die Faserstoffsuspension kann zudem organische oder mineralische Füllstoffe enthalten. Als organische Füllstoffe kommen u. a. synthetische Pigmente, z. B. Polykondensationsprodukte aus Harnstoff oder Melamin und Formaldehyd mit grossen spezifischen Oberflächen, die in hochdisperser Form vorliegen und u. a. in den britischen Patentschriften 1 043 937 und 1 318 244 beschrieben sind, und als mineralische Füllstoffe kommen u. a. Talkum, Titandioxyd und vor allem Kaolin und/oder Calciumcarbonat in Betracht. In der Regel enthält die Faserstoffsuspension 0 bis 40, vorzugsweise 5 bis 15 Gewichtsprozent, bezogen auf den Trockenfasergehalt, an Füllstoffen der angegebenen Art.

Bei Zusatz von z. B. Calciumcarbonat werden im allgemeinen schwach alkalische Faserstoffsuspensionen, hingegen werden bei Zusatz von z. B. Kaolin im allgemeinen schwach saure Faserstoffsuspensionen erhalten.

Die Faserstoffsuspension wird im erfindungsgemässen Verfahren auf an sich bekannte Weise auf Blattbildnern oder vorzugsweise kontinuierlich auf Papiermaschinen üblicher Bauart zu Papier oder Karton weiterverarbeitet. Somit bildet nach dem erfindungsgemässen Verfahren gefärbtes Papier oder gefärbtes Karton einen weiteren Gegenstand der vorliegenden Erfindung. Diese Erzeugnisse zeichnen sich durch ihre besonders echten, vor allem guten wasserechten und insbesondere guten lichtechten Färbungen aus.

Die guten algizide, fungizide und bakterizide Eigenschaften der erfindungsgemässen Ammoniumsalze mit kationischen Einheiten der Formel (1) können z. B. in Verfahren zur Wasseraufbereitung oder zur Behandlung von Substraten ausgenützt werden, die weitere Erfindungsgegenstände darstellen. Diese Verfahren bestehen darin, dass man die Ammoniumsalze dem aufzubereitenden Wasser zusetzt oder gegebenenfalls als organisch-wässrige feinverteilte Emulsion auf die Substrate aufbringt.

In den nachstehenden Beispielen angegebenen Teile und Prozente beziehen sich auf das Gewicht.

Beispiel 1

40,5 Teile eines Diepoxydes mit einem Epoxydäquivalentgewicht von 467, das der Formel

(30.11)
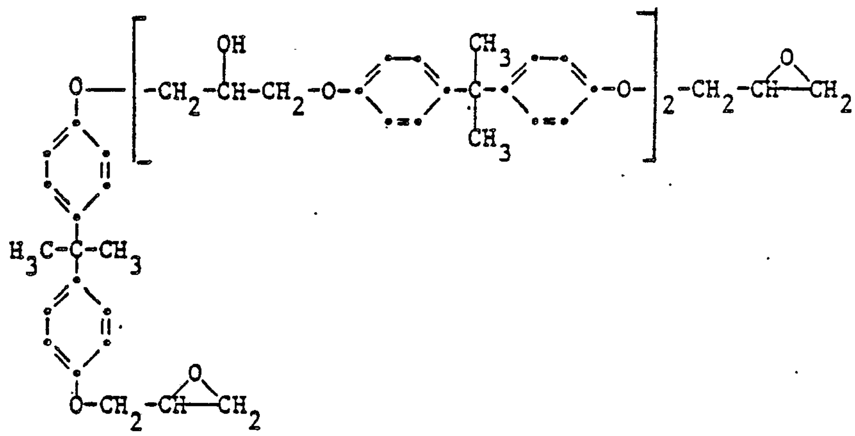

entspricht (0,043 Mol) und 13,05 Teile 1,3-Bis(3'-Dimethylaminopropyl)-harnstoffdihydrochlorid (0,043 Mol) werden in 250 Teile 1-Methoxy-2-äthanol gelöst und während 5 Stunden bei 120 bis 125 °C umgesetzt.

Das Lösungsmittel wird nach beendeter Umsetzung bei etwa 80 °C und einem verminderten Druck von etwa 0,2 bar abdestilliert. Der viskose Destillationsrückstand wird zur besseren Handhabung mit einem Gemisch 1 : 1 aus Methanol, Aethanol oder Isopropanol und entionisiertem Wasser zu einer Lösung verdünnt, die 10 % eines Ammoniumsalzes enthält, die kationische Einheiten der Formel (29.1) aufweist, worin 2 $Cl^{\ominus}$ als Anionen vorhanden sind. Man kann aber auch den Destillationsrückstand in 1 000 Teile Aceton eintragen und den so erhaltenen, weissen Niederschlag durch Filtration abtrennen und unter vermindertem Druck bei 60 °C trocknen.

Beispiel 2

Man verfährt wie in Beispiel 1 angegeben, setzt jedoch 16,3 Teile eines Diepoxydes mit einem Epoxydäquivalentgewicht von 189 ein, das der Formel

(30.12)
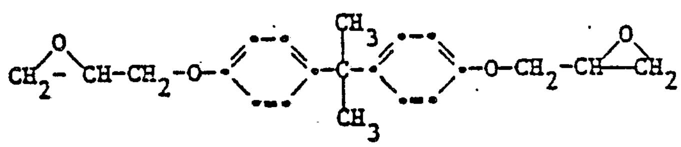

entspricht (0,043 Mol).

Die verdünnte 10 %ige Lösung im Gemisch 1 : 1 von Wasser : Methanol, Aethanol oder Isopropanol enthält ein Ammoniumsalz, das kationische Einheiten der Formel (29.2) aufweist, worin 2 $Cl^{\ominus}$ als Anionen vorhanden sind.

Beispiel 3

40,5 Teile des Diepoxydes der in Beispiel 1 angegebenen Formel (30.11) (0,043 Mol) und 15,05 Teile des Salzes aus 1,3-Bis(3'-dimethylaminopropyl)-harnstoff und Essigsäure (0,043 Mol) werden in 400 Teilen Wasser eingetragen und während 20 Stunden bei 90-95 °C umgesetzt, wobei im Verlaufe der Umsetzung 100 Teile Wasser aus dem Reaktionsgemisch abdestilliert werden. Zur Aufarbeitung wird das Reaktionsgemisch in 4 500 Teile Aceton eingetragen, das so ausgefällte Ammoniumsalz durch Filtration abgetrennt und unter vermindertem Druck bei 70 °C getrocknet. Man erhält ein Ammoniumsalz, das kationische Einheiten der Formel (29.1) aufweist, worin $2^{\ominus}OOC—CH_3$ als Anionen vorhanden sind.

Beispiel 4

40,5 Teile des Diepoxydes der in Beispiel 1 angegebenen Formel (30.11) (0,043 Mol) und 27,9 Teile des Salzes aus 1,3-Bis(3'-dimethyl-aminopropyl)-harnstoff und Laurinsäure (0,043 Mol) werden in 350 Teilen 1-Methoxy-2-äthanol gelöst und während 5 Stunden bei 125 °C umgesetzt. Zur Aufarbeitung wird das Reaktionsgemisch in 4 000 Teile Aceton eingetragen und das ausgefällte Ammoniumsalz durch Filtration abgetrennt und unter vermindertem Druck bei 70 °C getrocknet. Man erhält ein Ammoniumsalz, das kationische Einheiten der Formel (29.1) aufweist, worin $2^{\ominus}OOC—(CH_2)_{10}—CH_3$ als Anionen vorhanden sind.

Beispiel 5

123,6 Teile des mit Aceton gefällten Ammoniumsalzes gemäss Beispiel 1, das kationische Einheiten der Formel (29.1) aufweist, worin 2 $Cl^{\ominus}$ als Anionen vorhanden sind, werden in 650 Teilen Wasser und 100 Teilen Aethanol gelöst. In diese Lösung wird eine Lösung aus 30,6 Teilen Stearinsäurenatriumsalz in 1 200 Teilen Aethanol und 300 Teilen Wasser zugegeben. Das Reaktionsgemisch wird unter vermindertem Druck bei 60 °C auf 1 000 Teile eingeengt, wobei das Ammoniumsalz ausfällt. Nach Zugabe von 2 000 Teilen Wasser wird das Ammoniumsalz durch Filtration abgetrennt und unter vermindertem Druck bei 50 °C getrocknet. Man erhält ein in Aethanol lösliches Ammoniumsalz, das kationische Einheiten der Formel (29.1) aufweist, worin ca. 50 % der ursprünglich vorhandenen 2 $Cl^{\ominus}$ Anionen durch $^{\ominus}OOC—(CH_2)_{16}—CH_3$ ersetzt sind.

Beispiel 6

123,6 Teile des mit Aceton gefällten Ammoniumsalzes gemäss Beispiel 1 werden in 1 500 Teilen Wasser gelöst. In diese Lösung wird eine Lösung aus 58,8 Teilen Natriumlaurylsulfat in 500 Teilen Wasser unter intensivem Rühren innerhalb von 30 Minuten zugegeben, wobei das Ammoniumsalz gleichzeitig ausfällt. Das Ammoniumsalz wird durch Filtration abgetrennt und unter vermindertem Druck bei 60 °C getrocknet. Infolge des quantitativen Austausches der ursprünglich vorhandenen Chloridionen durch Laurylsulfationen weist das erhaltene Ammoniumsalz kationische Einheiten der Formel (29.1) auf, worin $2^{\ominus}SO_3—O—(CH_2)_{11}—CH_3$ als Anionen vorhanden sind.

Beispiel 7

Man verfährt wie im Beispiel 1 angegeben, setzt jedoch 17,1 Teile des in Beispiel 2 angegebenen Epoxyds der Formel (30.12) ein (0,045 Mol oder Molverhältnis Epoxyd : Diamin von 1 : 0,95). Man erhält das Ammoniumsalz mit kationischen Einheiten der Formel (29.2), worin 2 $Cl^{\ominus}$ als Anionen vorhanden sind.

Beispiel 8

Man verfährt wie in Beispiel 1 angegeben, setzt jedoch 15,5 Teile des in Beispiel 2 angegebenen Epoxyds der Formel (30.12) ein (0,041 Mol oder Molverhältnis Epoxyd : Diamin von 1 : 1,05). Man erhält das Ammoniumsalz mit kationischen Einheiten der Formel (29.2), worin 2 $Cl^{\ominus}$ als Anionen vorhanden sind.

Beispiel 9

Man verfährt wie in Beispiel 1 angegeben, setzt jedoch 16,3 Teile des in Beispiel 2 angegebenen Diepoxydes der Formel (30.12) (0,043 Mol) und 15,5 Teile des ditertiären Aminsalzes der Formel

(31.1) $$(CH_3-CH_2)_2-\overset{H}{\underset{\oplus}{N}}-(CH_2)_3-NH-CO-NH-(CH_2)_3-\overset{H}{\underset{\oplus}{N}}-(CH_2-CH_3)_2 \quad 2\ Cl^{\ominus}$$

(0,043 Mol) ein. Man erhält ein Ammoniumsalz mit kationischen Einheiten der Formel (29.3), worin 2 $Cl^{\ominus}$ als Anionen vorhanden sind.

Beispiel 10

Man verfährt wie in Beispiel 1 angegeben, setzt jedoch 16,3 Teile des in Beispiel 2 angegebenen Diepoxydes der Formel (30.12) (0,043 Mol) und 11,9 Teile des ditertiären Aminsalzes der Formel

(31.2) $$(CH_3)_2-\overset{H}{\underset{\oplus}{N}}-(CH_2)_2-NH-CO-NH-(CH_2)_2-\overset{H}{\underset{\oplus}{N}}-(CH_3)_2 \quad 2\ Cl^{\ominus}$$

(0,043 Mol) ein. Man erhält ein Ammoniumsalz mit kationischen Einheiten der Formel (29.4), worin 2 $Cl^{\ominus}$ als Anionen vorhanden sind.

Beispiel 11

Man verfährt wie in Beispiel 1 angegeben, setzt jedoch 16,3 Teile des in Beispiel 2 angegebenen Diepoxydes der Formel (30.12) (0,043 Mol) und 11.2 Teile des ditertiären Aminsalzes der Formel

(31.3) $(CH_3)_2-\overset{H}{\underset{\oplus}{N}}-CH_2-CO-NH-(CH_2)_3-\overset{H}{\underset{\oplus}{N}}-(CH_3)_2 \quad 2\ Cl^{\ominus}$

(0,043 Mol) ein. Man erhält ein Ammoniumsalz mit kationischen Einheiten der Formel (29.5), worin 2 $Cl^{\ominus}$ als Anionen vorhanden sind.

Beispiel 12

Man verfährt wie in Beispiel 1 angegeben, setzt jedoch 16,3 Teile des in Beispiel 2 angegebenen Diepoxyds der Formel (30.12) (0,043 Mol) und 14,3 Teile des ditertiären Aminsalzes der Formel

(31.4) $(CH_3)_2-\overset{H}{\underset{\oplus}{N}}-(CH_2)_3-NH-CO-CO-NH-(CH_2)_3-\overset{H}{\underset{\oplus}{N}}-(CH_3)_2 \quad 2\ Cl^{\ominus}$

(0,043 Mol) ein. Man erhält ein quaternäres Ammoniumsalz mit kationischen Einheiten der Formel (29.6), worin 2 $Cl^{\ominus}$ als Anionen vorhanden sind.

Beispiel 13

Man verfährt wie in Beispiel 1 angegeben, setzt jedoch 16,3 Teile des in Beispiel 2 angegebenen Diepoxyds der Formel (30.12) (0,043 Mol) und 16,7 Teile des ditertiären Aminsalzes der Formel

(31.5) $(CH_3)_2-\overset{H}{\underset{\oplus}{N}}-(CH_2)_3-NH-CO-(CH_2)_4-CO-NH-(CH_2)_3-\overset{H}{\underset{\oplus}{N}}-(CH_3)_2 \quad 2\ Cl^{\ominus}$

(0,043 Mol) ein. Man erhält ein Ammoniumsalz mit kationischen Einheiten der Formel (29.7), worin 2 $Cl^{\ominus}$ als Anionen vorhanden sind.

Beispiel 14

Man verfährt wie in Beispiel 1 angegeben, setzt jedoch 16,3 Teile des in Beispiel 2 angegebenen Diepoxyds der Formel (30.12) (0,043 Mol) und 17,6 Teile des ditertiären Aminsalzes der Formel

(31.6) 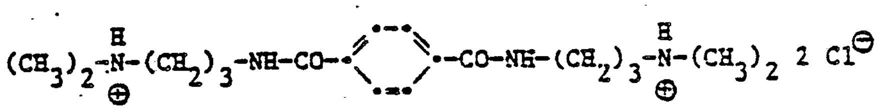

(0,043 Mol) ein. Man erhält ein Ammoniumsalz mit kationischen Einheiten der Formel (29.8), worin 2 $Cl^{\ominus}$ als Anionen vorhanden sind.

Beispiel 15

Man verfährt wie in Beispiel 1 angegeben, setzt jedoch 16,3 Teile des in Beispiel 2 angegebenen Diepoxyds der Formel (30.12) (0,043 Mol) und 17,6 Teile des ditertiären Aminsalzes der Formel

(31.7) 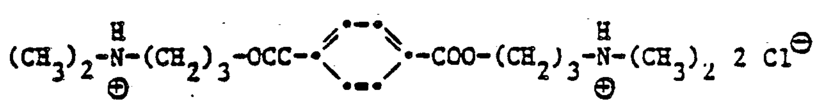

(0,043 Mol) ein. Man erhält ein quaternäres Ammoniumsalz mit kationischen Einheiten der Formel (29.9), worin 2 $Cl^{\ominus}$ als Anionen vorhanden sind.

Beispiel 16

Man verfährt wie in Beispiel 1 angegeben, setzt jedoch 16,3 Teile des in Beispiel 2 angegebenen Diepoxyds der Formel (30.12) (0,043 Mol) und 16,0 Teile des ditertiären Aminsalzes der Formel

(31.8) 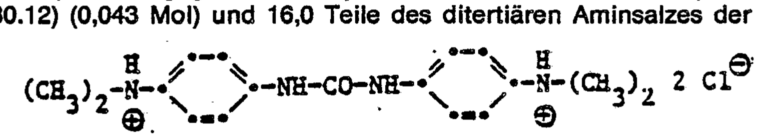

(0,043 Mol) ein. Man erhält ein Ammoniumsalz mit kationischen Einheiten der Formel (29.10), worin 2 $Cl^{\ominus}$ als Anionen vorhanden sind.

Beispiel 17

Man verfährt wie in Beispiel 1 angegeben, setzt jedoch 16,3 Teile des in Beispiel 2 angegebenen Diepoxyds der Formel (30.12) (0,043 Mol) und 16,5 Teile des ditertiären Aminsalzes der Formel

(31.9) 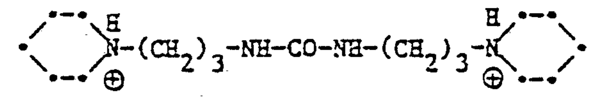 2 $Cl^{\ominus}$

(0,043 Mol) ein. Man erhält ein Ammoniumsalz mit kationischen Einheiten der Formel (29.11), worin 2 $Cl^{\ominus}$ als Anionen vorhanden sind.

Beispiel 18

Man verfährt wie in Beispiel 1 angegeben, setzt jedoch 16,3 Teile des in Beispiel 2 angegebenen Diepoxyds der Formel (30.12) (0,043 Mol) und 14,5 Teile des ditertiären Aminsalzes der Formel

(31.10) 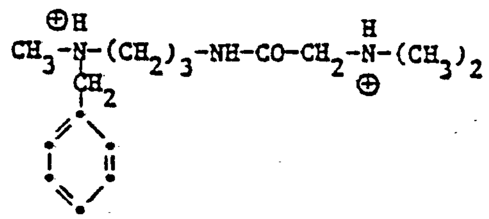 2 $Cl^{\ominus}$

(0,043 Mol) ein. Man erhält ein Ammoniumsalz mit kationischen Einheiten der Formel (29.12), worin 2 $Cl^{\ominus}$ als Anionen vorhanden sind.

Beispiel 19

Man verfährt wie in Beispiel 1 angegeben, setzt jedoch 8,8 Teile eines Diepoxyds mit einem Epoxydäquivalentgewicht von 102, das der Formel

(30.13) 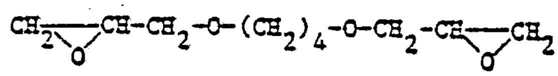

entspricht (0,043 Mol) und 13,05 Teile 1,3-Bis(3'-Dimethylaminopropyl)-harnstoffdihydrochlorid (0,043 Mol) ein. Man erhält ein quaternäres Ammoniumsalz mit kationischen Einheiten der Formel (29.13), worin 2 $Cl^{\ominus}$ als Anionen vorhanden sind.

Beispiel 20

Man verfährt wie in Beispiel 1 angegeben, setzt jedoch 8,8 Teile des in Beispiel 19 angegebenen Epoxyds der Formel (30.13) (0,043 Mol) und 14,3 Teile des in Beispiel 12 angegebenen ditertiären Aminsalzes der Formel (31.4) (0,043 Mol) ein. Man erhält ein Ammoniumsalz mit kationischen Einheiten der Formel (29.14), worin 2 $Cl^{\ominus}$ als Anionen vorhanden sind.

Beispiel 21

Man verfährt wie in Beispiel 1 angegeben, setzt jedoch 12,0 Teile eines Diepoxyds mit einem Epoxidäquivalentgewicht von 139, das der Formel

(30.14) 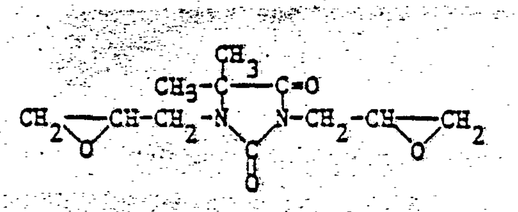

entspricht (0,043 Mol) und 16,7 Teile des in Beispiel 13 angegebenen ditertiären Aminsalzes der Formel (31.5) (0,043 Mol) ein. Man erhält ein Ammoniumsalz mit kationischen Einheiten der Formel (29.15), worin 2 $Cl^{\ominus}$ als Anionen vorhanden sind.

## Beispiel 22

Man verfährt wie in Beispiel 1 angegeben, setzt jedoch 15,7 Teile eines Diepoxyds mit einem Epoxydäquivalentgewicht von 170, das der Formel

(30.15) 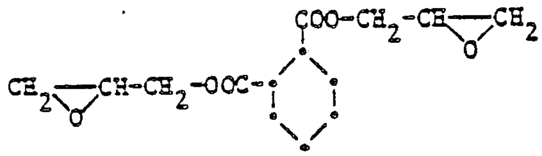

entspricht (0,043 Mol) und 13,05 Teile 1,3-Bis(3'-dimethylaminopropyl)-harnstoffdihydrochlorid (0,043 Mol) ein. Man erhält ein Ammoniumsalz mit kationischen Einheiten der Formel (29.16), worin 2 $Cl^{\ominus}$ als Anionen vorhanden sind.

## Beispiel 23

Man verfährt wie in Beispiel 1 angegeben, setzt jedoch 17,2 Teile eines Diepoxyds mit einem Epoxydäquivalentgewicht von 200, das der Formel

(30.16) 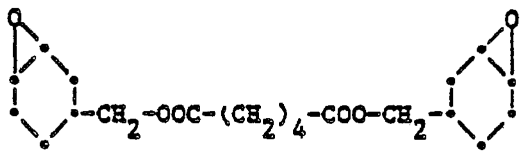

entspricht (0,043 Mol) und 13,05 Teile 1,3-Bis(3'-dimethylaminopropyl)-harnstoffdihydrochlorid (0,043 Mol) ein. Man erhält ein Ammoniumsalz mit kationischen Einheiten der Formel (29.17), worin 2 $Cl^{\ominus}$ als Anionen vorhanden sind.

## Beispiel 24

Man verfährt wie im Beispiel 1 angegeben, setzt jedoch 11,6 Teile eines Diepoxydes mit einem Epoxydäquivalentgewicht von 135, das der Formel

(30.17) 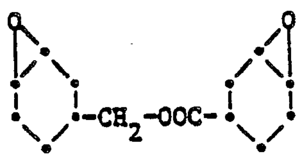

entspricht (0,043 Mol) und 13,05 Teile 1,3-Bis-(3'-dimethylaminopropyl)-harnstoffdihydrochlorid (0,043 Mol) ein. Man erhält ein Ammoniumsalz mit kationischen Einheiten der Formel (29.18), worin 2 $Cl^{\ominus}$ als Anionen vorhanden sind.

## Beispiel 25

Man verfährt wie in Beispiel 1 angegeben, setzt jedoch 13,8 Teile eines Diepoxydes mit einem Epoxydäquivalentgewicht von 160, das der Formel

(30.17) 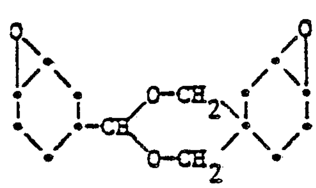

entspricht, (0,043 Mol) und 14,3 Teile des in Beispiel 12 angegebenen ditertiären Aminsalzes der Formel (31.4) (0,043 Mol) ein. Man erhält ein Ammoniumsalz mit kationischen Einheiten der Formel (29.19), worin 2 $Cl^{\ominus}$ als Anionen vorhanden sind.

## Beispiel 26

Man verfährt wie in Beispiel 1 angegeben, setzt jedoch 7,85 Teile eines Diepoxyds mit einem Epoxydäquivalentgewicht von 91, das der angegebenen Formel (30.5) entspricht (0,043 Mol), und 13,05

Teile 1,3-Bis-(3'-dimethylaminopropyl)-harnstoffdihydrochlorid (0,043 Mol) ein. Man erhält ein Ammoniumsalz mit kationischen Einheiten der Formel (29.20), worin 2 $Cl^{\ominus}$ als Anionen vorhanden sind.

Beispiel 27

Eine 10 %ige Lösung eines Ammoniumsalzes mit kationischen Einheiten der Formel (29.2), worin 2 $Cl^{\ominus}$ als Anionen vorhanden sind, wird unter vermindertem Druck zur Trockene eingedampft und bei 70 °C und 0,05 Torr getrocknet. Das erhaltene hydroskopische, spröde Pulver (27,7 Teile) wird in 120 Teilen Tetrahydrofuran suspendiert. In diese Suspension werden 0,2 Teile 4-Dimethylaminopyridin und 13,3 Teile Essigsäureanhydrid (0,13 Mol oder 3 Mol pro Mol angesetztes Ammoniumsalz) zugegeben. Das Reaktionsgemisch wird auf die Rückflusstemperatur von ca. 70 °C aufgeheizt und während 5 Stunden bei dieser Temperatur gehalten. Nach dieser Zeit werden dem Reaktionsgemisch 300 Teile Aethanol zugegeben. Das Reaktionsgemisch wird anschliessend auf die Rückflusstemperatur von ca. 80 °C aufgeheizt, und noch während 2 Stunden bei dieser Temperatur gehalten, wobei aus der ursprünglichen Suspension eine klare Lösung entsteht, die unter vermindertem Druck zur Trockene eingedampft wird. Nach dem Trocknen des Rückstandes bei 70 °C und 0,05 Torr erhält man 36,3 Teile eines Ammoniumsalzes mit kationischen Einheiten der Formel (29.21) worin 2 $Cl^{\ominus}$ als Anionen vorhanden sind, das als wasserlösliches, gelbliches Pulver vorliegt.

Beispiel 28

10 Teile der äthanolischen Lösung des gemäss Beispiel 1 erhaltenen quaternären Ammoniumsalzes werden mit 25 Teilen Aethanol (96 %) und 65 Teilen entionisiertem Wasser zu insgesamt 100 Teilen eines haarkosmetischen Einlegemittels vermischt, dessen pH-Wert mit Milchsäure auf den pH-Wert von 6,0 gestellt wird.

Mit Wasser angenetzte menschliche Haare werden mit diesem Einlegemittel bei Raumtemperatur imprägniert, anschliessend auf Lockenwickler aufgerollt und bei Raumtemperatur getrocknet.

Das Haar weist einen unsichtbaren Film auf, der dem Haar nach dem Entfernen der Lockenwickler eine ausgezeichnete Formbeständigkeit verleiht, die sogar in feuchtem Klima erhalten bleibt.

Der Film kann bei einer Haarwäsche mit handelsüblichen Shampoos leicht entfernt werden.

Aehnliche Ergebnisse werden mit dem gemäss Beispiel 2 erhaltenen quaternären Ammoniumsalz erzielt.

Beispiel 29

20 Teile der äthanolischen Lösung des gemäss Beispiel 1 erhaltenen quaternären Ammoniumsalzes werden mit 50 Teilen Aethanol (96 %) und 10 Teilen entionisiertem Wasser zu insgesamt 100 Teilen eines haarkosmetischen Pumpsprays vermischt.

Mit einem Handzerstäuber werden frisierte menschliche Haare bei Raumtemperatur besprüht. Nach etwa 1 Minute Trockenzeit bei Raumtemperatur weist das frisierte Haar einen unsichtbaren Film auf, der dem Haar eine ausgezeichnete Formbeständigkeit verleiht, die sogar in feuchtem Zustand erhalten bleibt.

Aehnliche Ergebnisse werden mit dem gemäss Beispiel 2 erhaltenen quaternären Ammoniumsalz erzielt.

Beispiel 30

1 Teil des gemäss Beispiel 4 erhaltenen Ammoniumsalzes wird in 39 Teilen absolutem Alkohol gelöst. Diese Lösung wird in einer Aerosolbombe mit 60 Teilen eines Treibmittels versetzt, das aus einem 1 : 1 Gemisch aus Dichlortetrafluoräthan und Dichlordifluoräthan besteht. Mit diesem Aerosolspray behandeltes Menschenhaar weist eine ausgezeichnete Formbeständigkeit, die sogar in feuchtem Klima erhalten bleibt. Das auf das Haar besprühte Polymerisat kann beim Waschen der Haare mit handelsüblichen Shampoos leicht entfernt werden.

Beispiel 31

Ein Polyacrylnitrilgewebe wird mit einer wässrigen Druckpaste stellenweise bedruckt, die 2,5 % des roten Farbstoffes der Formel

(38.1) 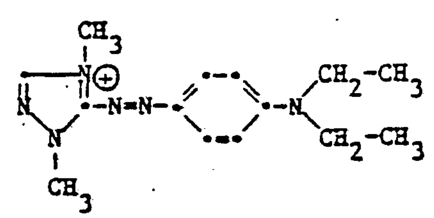 $ZnCl_3^{\ominus}$ ,

3 % eines 10 %igen Kondensationsproduktes aus einer Naphtalinsulfonsäure und Formaldehyd und 94,5 % einer wässrigen Stammlösung eines Verdickungsmittels enthält.

Die wässrige Stammlösung des Verdickungsmittels enthält 50 % eines 16 %igen, neutralen, nicht ionogenen Kernmehläthers, 3 % einer 50 %igen Weinsäure und 15 % N,N-Bis(Cyanäthyl)-formamid.

Das Gewebe wird nach dem Bedrucken getrocknet und zur Fixierung des Druckes mit Sattdampf bei 110 °C während 30 Minuten behandelt.

Nun wird das so bedruckte Polyacrylnitrilgewebe während 20 Minuten bei 75 °C in einer wässrigen Flotte ausgewaschen, die 0,05 g/l bezogen auf Wirksubstanz, des Ammoniumsalzes gemäss Beispiel 2 enthält.

Das ausgewaschene Gewebe weist scharfe rote Musterungen auf, wobei die nicht bedruckten Stellen (Weissreserven) vollständig ungefärbt bleiben. Wird hingegen das Gewebe mit Wasser in Abwesenheit des Ammoniumsalzes bei identischen Bedingungen ausgewaschen, so weisen die nicht bedruckten Stellen des Gewebes eine durch Ausbluten des Farbstoffes bedingte, unerwünschte Rosafärbung auf.

Zudem ist das in Gegenwart des Ammoniumsalzes erfindungsgemäss ausgewaschene bedruckte Gewebe gemäss DIN 54 010, 54 006 und 54 004 wasch- und lichtecht, was bei nicht ausgewaschenen oder in Abwesenheit des Ammoniumsalzes ausgewaschenen, bedruckten Geweben nicht der Fall ist.

Aehnliche Ergebnisse werden mit den Ammoniumsalzen gemäss einem der Beispiele 1 oder 3 bis 27 erzielt.

## Beispiel 32

Ein Filterpapier aus reiner Cellulose mit einem Flächengewicht von 110 g/m$^2$ wird mit einer Geschwindigkeit von 4 m/Min. und einem Anpressdruck von 10 bar mit einer wässrigen Flotte foulardiert, welche 0,1 %, bezogen auf Wirksubstanz, des Ammoniumsalzes gemäss Beispiel 1 enthält. Das foulardierte Papier wird während 10 Minuten bei 90 °C getrocknet. Die erzielte Oberflächenleimung des behandelten Papiers wird aufgrund des Wasseraufnahmetests nach Cobb bei 30 Sekunden Einwirkungsdauer (WA Cobb$_{30}$) gemäss DIN 53 132 geprüft. Je tiefer die Wasseraufnahme im WA Cobb$_{30}$ Test ist, desto besser ist die Oberflächenleimung des behandelten Papiers. Bei dieser Prüfung beträgt die WA Cobb$_{30}$ der erfindungsgemäss behandelten Papiers 20 g/m$^2$, während unbehandeltes Papier eine WA Cobb$_{30}$ von 188 g/m$^2$ aufweist.

## Beispiel 33

Eine Faserstoffsuspension aus 50 % gebleichtem Nadelholzsulfitzellstoff und 50 % gebleichtem Buchensulfatzellstoff, welche einen Schopper-Riegler-Mahlgrad von 22° aufweist, wird in einer Mischbütte mit 10 % gefälltem Calciumcarbonat als 30 %ige wässerige Aufschlämmung versetzt. Nach der Calciumcarbonatzugabe stellt sich in der Faserstoffsuspension ein pH-Wert von 7,4 bis 7,5 ein.

Die Faserstoffsuspension wird mit Wasser kontinuierlich auf einen Trockenfasergehalt von 1,2 % verdünnt.

Der Faserstoffsuspension werden 30 Minuten vor dem Stoffauflauf der Papiermaschine 0,13 % des Farbstoffes der Formel

(38.2) 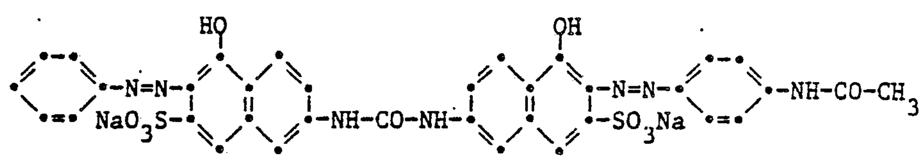

zugegeben. Hierauf werden ca. 25 Sek. vor dem Stoffauflauf der Papiermaschine 1,5 %, bezogen auf Wirksubstanz, des Ammoniumsalzes gemäss Beispiel 1 der Faserstoffsuspension zugesetzt.

Die Faserstoffsuspension wird in einer Laborpapiermaschine bei konstanter Maschineneinstellung zu einem Papier mit einem Flächengewicht von 75 ± 2 g/m$^2$ verarbeitet. Hierbei wird das Papier in der Maschine so getrocknet, dass eine Restfeuchte des Papiers von 5 % erhalten bleibt. Die erhaltenen Papierbogen werden während 24 Stunden bei 65 % relativer Luftfeuchtigkeit konditioniert.

Das auf diese Weise hergestellte, gefärbte Papier weist eine gute Licht- und Wasserechtheit auf.

## Beispiel 34

Ein Phosphatpuffermedium (pH 5, 7 und 8), das 1 000 ppm des Ammoniumsalzes gemäss Beispiel 7 enthält, wird mit jeweils einem Teststamm (Bakterien : O/n-Kulturen ; Pilze : Sporensuspension, 14-tägige Kulturen) inoculiert (Endkonzentration 10$^6$ Keime/ml). Nach einer Inkubationsdauer von 18 Stunden bei

20 °C auf einem Magnetrührwerk wird geprüft, bei welcher Konzentration eine Abtötung der Keime eingetreten ist. Das geprüfte Ammoniumsalz zeigt in diesem Test eine gute keimtötende Wirkung.

Als Testkeime werden verwendet :

| | |
|---|---|
| Staphylococcus aureus | ATCC 6538 |
| Escherichia coli | ATCC 11229 |
| Pseudomona aeruginosa | ATCC 15442 |
| Aspergillus niger | ATCC 6275 |

Medium : Phosphatpuffer nach Sörensen (1/15 molar) mit 2 % Brain-Heart-Infusion-Broth.

In diesem Test weisen die Ammoniumsalze gemäss einem der Beispiele 1 bis 6 und 8 bis 27 ebenfalls gute bakterizide und fungizide Eigenschaften auf.

**Ansprüche**

1. Quaternäre Ammoniumsalze, dadurch gekennzeichnet, dass sie Diepoxyd- und Diaminrest enthaltende Einheiten der Formel

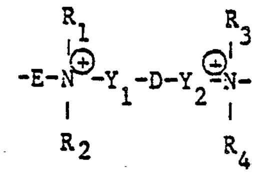

aufweisen, worin E ein zweiwertiges Brückenglied ist, das einer der Formeln

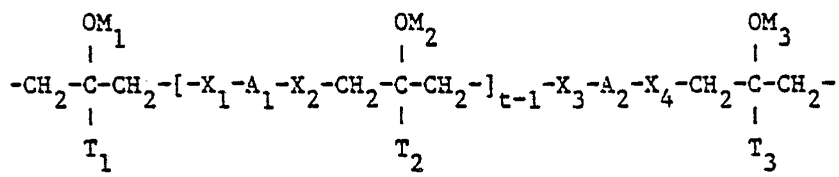

,

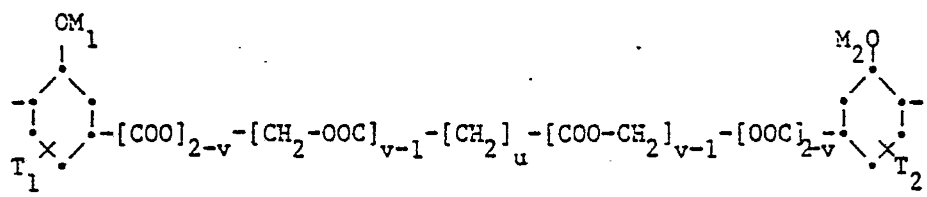

,

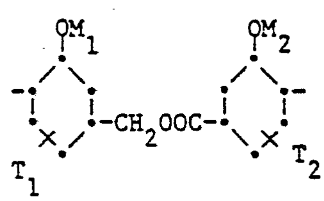

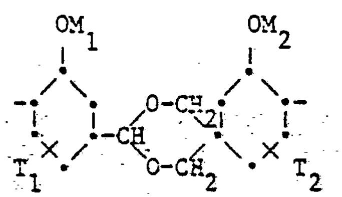

oder

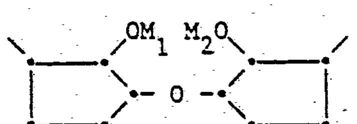

entspricht,

worin t eine Zahl von 1 bis 4, u eine ganze Zahl von 1 bis 8 und v 1 oder 2 sind, $R_1$, $R_2$, $R_3$ und $R_4$ gleich

oder voneinander verschieden sind und gegebenenfalls durch Hydroxyl, Alkoxy, Alkylthio oder Cyano substituiertes Alkyl, Cycloalkyl oder Alkenyl mit höchstens 8 Kohlenstoffatomen oder gegebenenfalls durch Hydroxyl, Hydroxyalkyl, Alkoxy, Alkoxyalkyl, Alkyl, Alkylthio, Cyano oder Halogen substituiertes Aryl oder Aralkyl bedeuten, oder ($R_1$ und $R_2$) und/oder ($R_3$ und $R_4$) zusammen mit dem Stickstoffatom, mit dem sie verbunden sind, einen heterocyclischen Ring mit 5 oder 6 Ringgliedern bilden, $M_1$, $M_2$ und $M_3$ je Acyl mit 2 bis 5 Kohlenstoffatomen oder Wasserstoff bedeuten, $Y_1$ und $Y_2$ gleich oder voneinander verschieden sind und der Formel

$$-C_mH_{2m}-$$

entsprechen, worin m eine ganze Zahl von 1 bis 12 ist, die Summe von m in $Y_1$ und $Y_2$ mindestens 3 beträgt und für m gleich 1 die Bindung zum Brückenglied D nicht über ein Stickstoff- oder Sauerstoff erfolgt, oder $Y_1$ und $Y_2$ gegebenenfalls durch Halogen, Hydroxyl, Alkyl, Halogenalkyl, Hydroxyalkyl oder Alkoxy substituiertes Phenylen bedeuten, D einem zweiwertigen Brückenglied einer der Formeln

$-NHCONH-$, $-NHCOD_1CONH-$, $-CONH-$,

$-OCONH-$, $-COO-$, $-COD_2CO-$,

$$-O\overset{O}{\overset{\|}{C}}-D_3-\overset{O}{\overset{\|}{C}}O- \quad \text{oder} \quad -O\overset{O}{\overset{\|}{C}}-NH-D_4-HN-\overset{O}{\overset{\|}{C}}O-$$

entspricht, worin $D_1$ die direkte Bindung, Alkylen, Alkenylen, Arylen, Heteroarylen, Diaminoalkylen, Diaminoarylen, gegebenenfalls halogensubstituiertes Dioxyalkylen, Polyoxyalkylenoxy oder Dioxyarylen, $D_2$ Diaminoalkylen, gegebenenfalls halogensubstituiertes Dioxyalkylen, Polyoxyalkylenoxy oder Dithioalkylen, $D_3$ Arylen und $D_4$ Alkylen oder Arylen sind, $T_1$, $T_2$ und $T_3$ je Wasserstoff oder Methyl, $X_1$, $X_2$, $X_3$ und $X_4$ je —COO—, —OOC—, —O— oder die direkte Bindung und $A_1$ und $A_2$ je einen gegebenenfalls durch Alkyl substituierten heterocyclischen Ring mit 5 oder 6 Ringgliedern und 2 Stickstoffatomen, gegebenenfalls durch Heteroatome unterbrochenes Alkylen, gegebenenfalls durch Alkyl oder Halogen substituiertes 1 oder 2 Ringe aufweisendes Cycloalkylen, Cycloalkenylen, Arylen oder Aralkylen bedeuten, wobei die ringförmigen Brückenglieder über eine direkte Bindung, über ein Heteroatom oder über eine gegebenenfalls durch Heteroatome unterbrochene Alkylenbrücke verbunden sind.

2. Ammoniumsalze nach Anspruch 1, dadurch gekennzeichnet, dass sie Einheiten der Formel

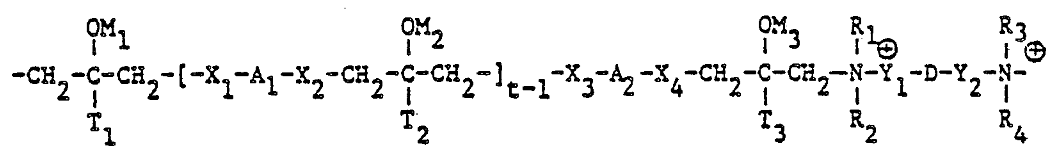

aufweisen, worin $A_1$ und $A_2$, D, $M_1$, $M_2$, $M_3$, $R_1$, $R_2$, $R_3$, $R_4$, $T_1$, $T_2$, $T_3$, $X_1$, $X_2$, $X_3$, $X_4$, $Y_1$, $Y_2$ und t die in Anspruch 1 angegebenen Bedeutungen haben.

3. Ammoniumsalze nach Anspruch 1, dadurch gekennzeichnet, dass sie Einheiten einer der Formeln

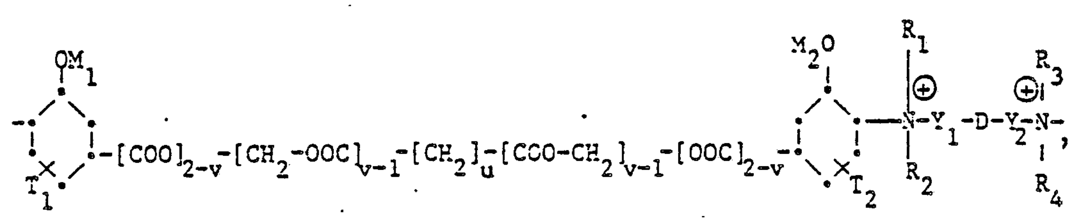

,

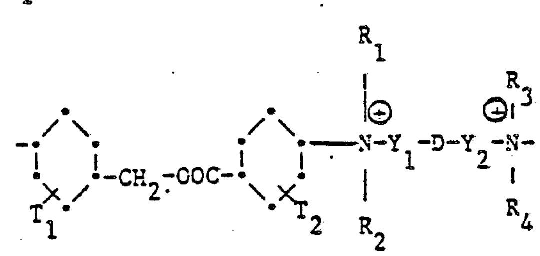

,

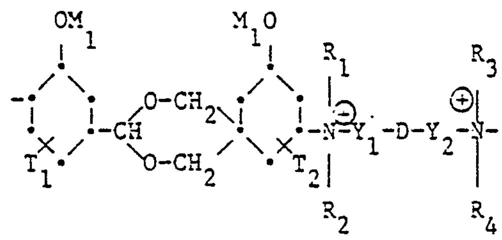

oder

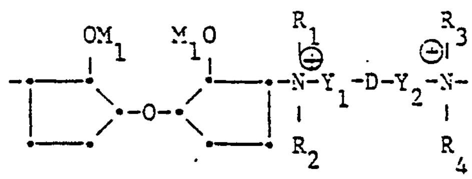

aufweisen, worin D, $M_1$, $M_2$, $R_1$, $R_2$, $R_3$, $R_4$, $T_1$, $T_2$, $Y_1$, $Y_2$, u und v die in Anspruch 1 angegebenen Bedeutungen haben.

4. Ammoniumsalze nach einem der Ansprüche 1 bis 3, dadurch gekennzeichnet, dass sie Einheiten einer der Formeln

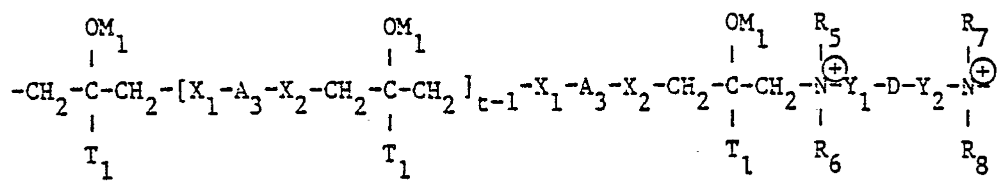

,

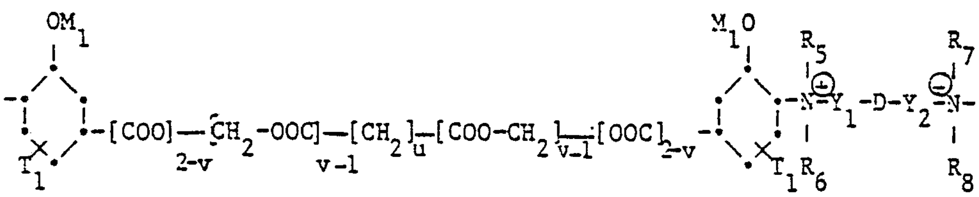

,

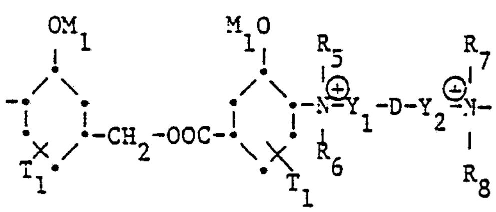

,

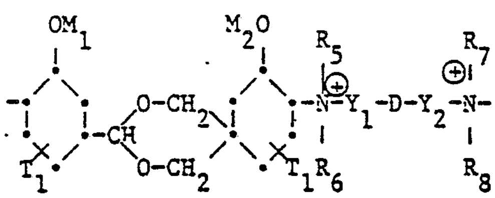

oder

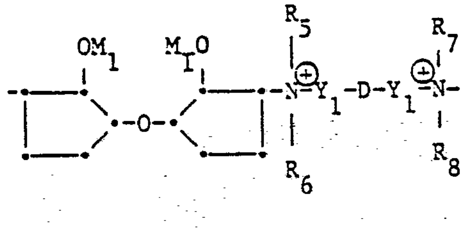

aufweisen, worin $R_5$, $R_6$, $R_7$ und $R_8$ gleich oder voneinander verschieden sind und Alkyl, Hydroxyalkyl, Alkoxyalkyl, Alkylthioalkyl oder Cyanoalkyl mit insgesamt 1 bis 8 Kohlenstoffatomen, Cyclopentyl, Cyclohexyl, Alkenyl mit 2 bis 4 Kohlenstoffatomen, unsubstituiertes Phenyl oder Benzyl oder mit Hydroxyl, Cyano, Chlor, Brom, Alkyl, Hydroxyalkyl, Alkoxy, Alkylthio, Alkoxyalkyl mit je 1 oder 2 Kohlenstoffatomen im Alkyl- und Alkoxyteil substituiertes Phenyl oder Benzyl bedeuten; oder ($R_5$ und $R_6$) und ($R_7$ und $R_8$) zusammen mit dem Stickstoffatom, mit dem sie verbunden sind, einen heterocyclischen Ring einer der Formeln

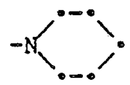 , 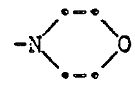 , 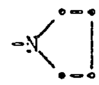 oder 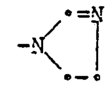

bilden ; $A_3$ einen gegebenenfalls durch Methyl, Aethyl oder Isopropyl substituierten heterocyclischen Ring mit 5 oder 6 Ringgliedern und 2 Stickstoffatomen, gegebenenfalls durch Sauerstoffatome unterbrochenes Alkylen mit 2 bis 34 Kohlenstoffatomen, gegebenenfalls durch Methyl oder Aethyl substituiertes Cycloalkenylen mit 6 bis 10 Kohlenstoffatomen oder ein oder zwei Ringe aufweisendes Cycloalkylen mit 6 bis 18 Kohlenstoffatomen, gegebenenfalls durch Brom oder Chlor substituiertes ein oder zwei Ringe aufweisendes Arylen mit 6 bis 18 Kohlenstoffatomen oder Aralkylen mit 8 bis 26 Kohlenstoffatomen bedeutet und D, $M_1$, $T_1$, $X_1$, $X_2$, $Y_1$, $Y_2$, t, u und v die in Anspruch 1 angegebenen Bedeutungen haben.

5. Ammoniumsalze nach Anspruch 4, dadurch gekennzeichnet, dass sie Einheiten einer der Formeln

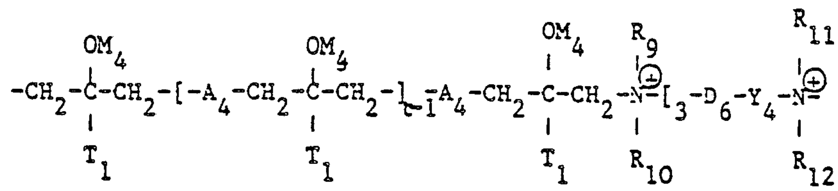 ,

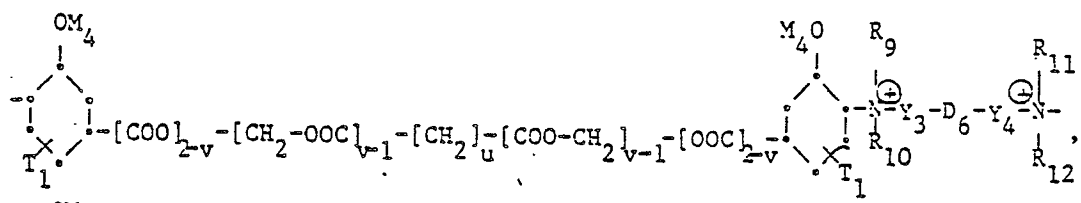 ,

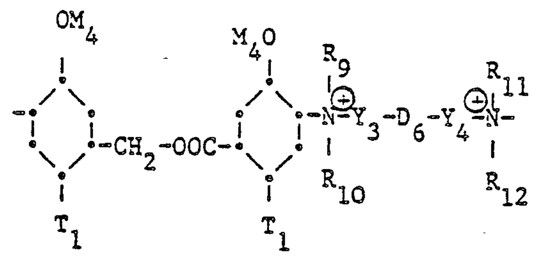 ,

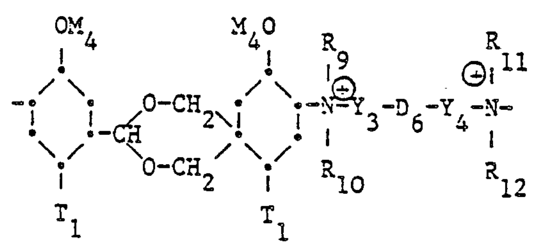 oder

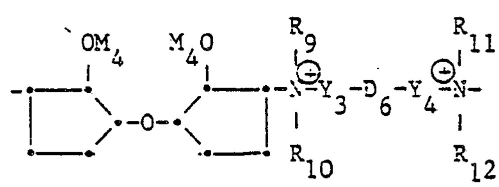

aufweisen, worin $D_6$ einer der Formeln

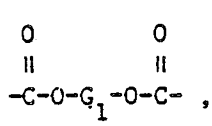 , 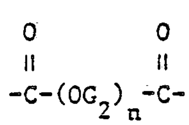 , 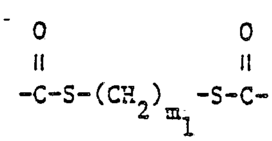 ,

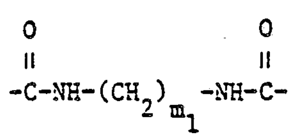 , 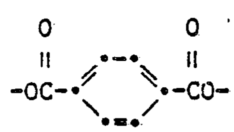 , 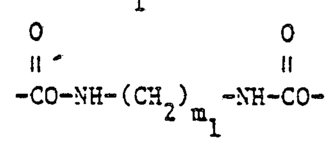 ,

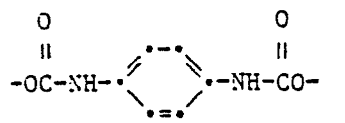 , $-HN-OC-CO-NH-$, 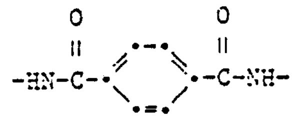 ,

$-HN-CO-(CH_2)_{m_1}-CO-NH-$ , $-NH-CO-NH-$ , $-CONH-$ ,

$-OCONH$ oder $-COO-$

entspricht, worin $G_1$ Alkylen mit 2 bis 12 Kohlenstoffatomen, n eine ganze Zahl von 2 bis 15 und $m_1$ eine ganze Zahl von 2 bis 12, sind, $R_9$, $R_{10}$, $R_{11}$ und $R_{12}$ gleich oder voneinander verschieden sind und Phenyl, Benzyl oder Alkyl mit 1 bis 4 Kohlenstoffatomen bedeuteten oder ($R_9$ und $R_{10}$) und ($R_{11}$ und $R_{12}$) zusammen mit dem Stickstoffatom, mit dem sie verbunden sind, einen Piperidinring bilden, $M_4$ Propionyl, Acetyl oder Wasserstoff bedeutet, $A_4$ einem zweiwertigen Rest einer der Formeln

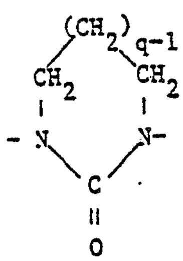 , 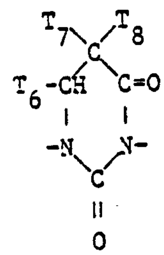 ,

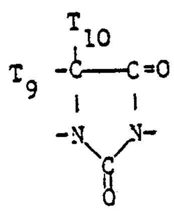 , $-O-(OC)_{q-1}-Q-(CO)_{q-1}-O-$

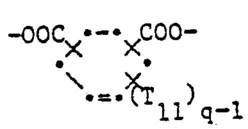 , 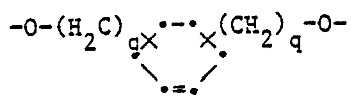 ,

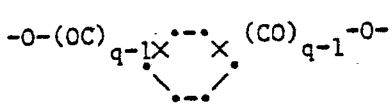 , 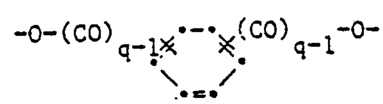

oder

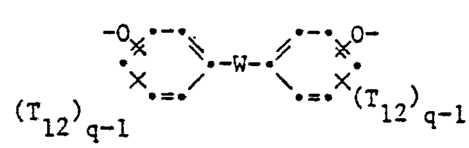

entspricht, worin Q gegebenenfalls durch 1 oder 2 Sauerstoffatome unterbrochenes Alkylen mit 2 bis 8 Kohlenstoffatomen, W die direkte Bindung, $-SO_2-$ oder Alkylen mit 1 bis 4 Kohlenstoffatomen, $T_6$, $T_7$, $T_8$ und $T_9$ je Wasserstoff oder Methyl, $T_{10}$ Wasserstoff, Methyl, Aethyl oder Isopropyl, $T_{11}$ Methyl oder Aethyl, $T_{12}$ Chlor oder Brom und q 1 oder 2 bedeuten und $Y_1$, $Y_2$, $T_1$, t, u und v die in Anspruch 1 angegebenen Bedeutungen haben.

6. Ammoniumsalze nach Anspruch 5, dadurch gekennzeichnet, dass sie Einheiten einer der Formeln

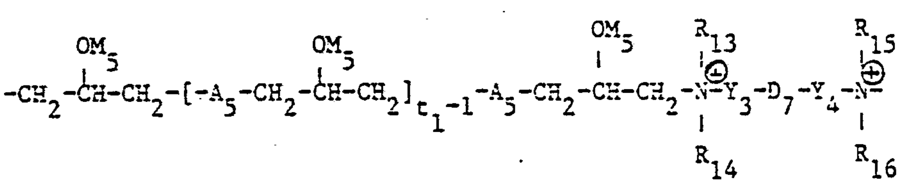

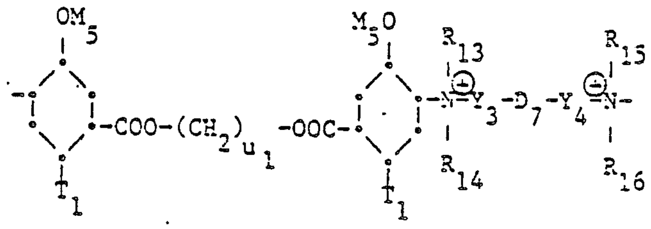

,

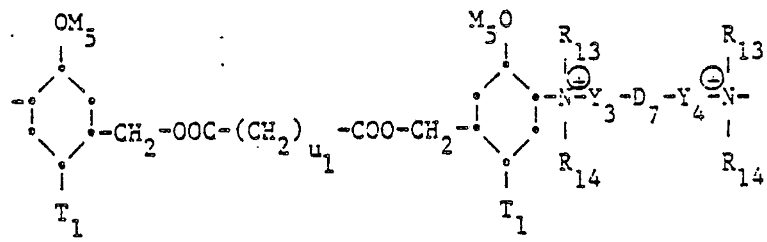

,

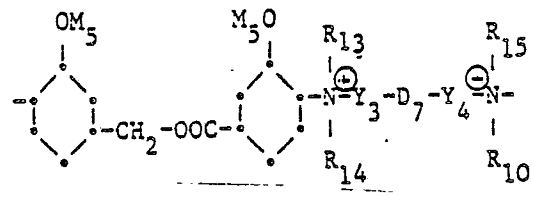

,

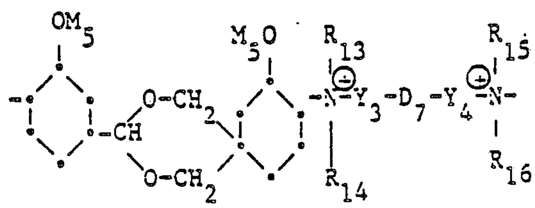

oder

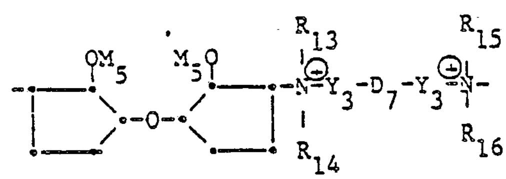

aufweisen worin $R_{13}$, $R_{14}$, $R_{15}$ und $R_{16}$ je Benzyl, Methyl oder Aethyl sind oder ($R_{13}$ und $R_{14}$) und ($R_{15}$ und $R_{16}$) zusammen mit dem Stickstoffatom, mit dem sie verbunden sind, einen Piperidinring bilden, $M_5$ Acetyl oder Wasserstoff, $T_1$ Methyl oder Wasserstoff, $Y_3$ und $Y_4$ je Alkylen mit 1 bis 6 Kohlenstoffatomen oder Phenylen, $t_1$ eine beliebige Zahl von 1 bis 3 und u eine ganze Zahl von 1 bis 4 bedeuten, $A_5$ einer der Formeln

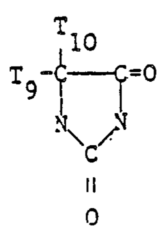

,

$$-O-(OC)_{q-1}-Q-(CO)_{q-1}-O-$$

,

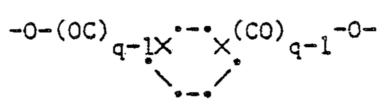

oder

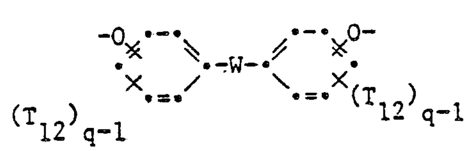

entspricht, worin Q gegebenenfalls durch 1 oder 2 Sauerstoffatome unterbrochenes Alkylen mit 2 bis 8 Kohlenstoffatomen, W die direkte Bindung, $-SO_2-$ oder Alkylen mit 1 bis 4 Kohlenstoffatomen, $T_9$ Wasserstoff oder Methyl, $T_{10}$ Wasserstoff, Methyl, Aethyl oder Isopropyl, $T_{12}$ Chlor oder Brom und q 1 oder 2 bedeuten und $D_7$ einer der Formeln

-NH-CO-NH-, -CO-NH- , 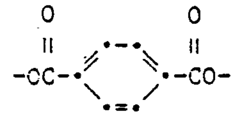

-HN-OC-CO-NH- , 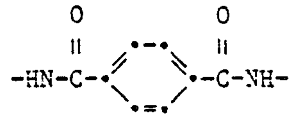 oder

$-HN-CO-(CH_2)_{m_1}-CO-NH-$

entspricht, worin $m_1$ eine ganze Zahl von 2 bis 12 ist.

7. Verfahren zur Herstellung der Ammoniumsalze gemäss einem der Ansprüche 1 bis 6, dadurch gekennzeichnet, dass man ein Diepoxyd oder ein Diepoxydgemisch einer der Formeln

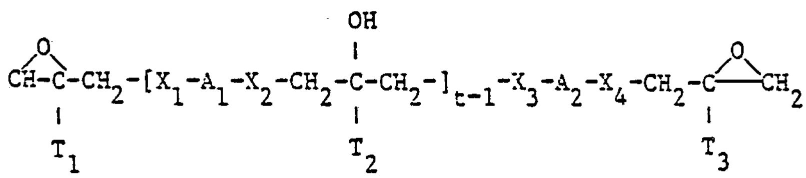

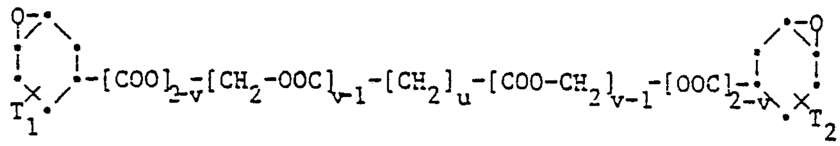

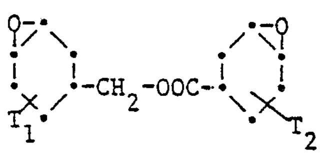 , 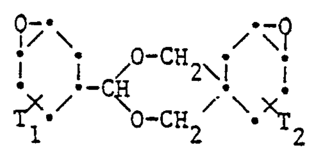 oder

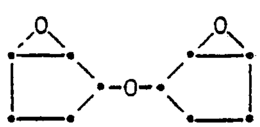

mit einem dietertiären Aminsalz der Formel

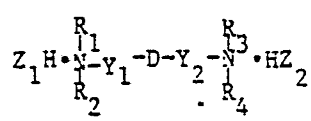

umsetzt, oder ein Diepoxyd oder Diepoxydgemisch einer der vorstehend angegebenen Formeln mit einem Halogenwasserstoff der Formel

$Z_3H$

in ein Dihalogenhydrin oder Dihalogenhydringemisch einer der Formeln

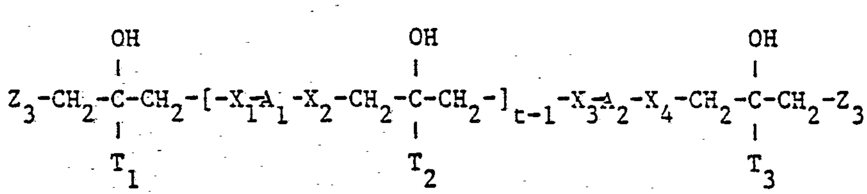 ,

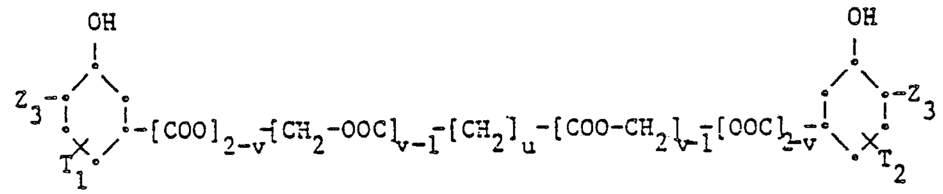
,

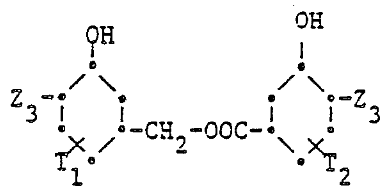

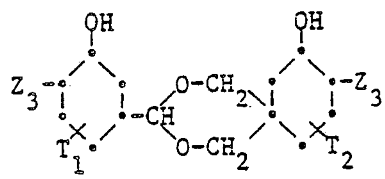
oder

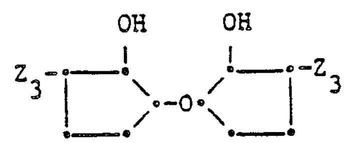

überführt und anschliessend mit einem ditertiären Amin der Formel

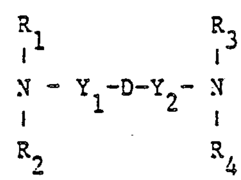

umsetzt, und die erhaltenen Umsetzungsprodukte oder Umsetzungsproduktgemische, die Einheiten einer der Formeln

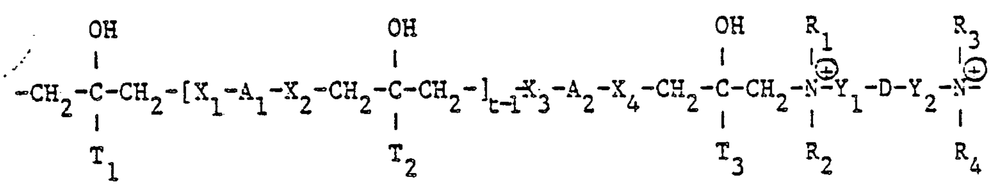
,

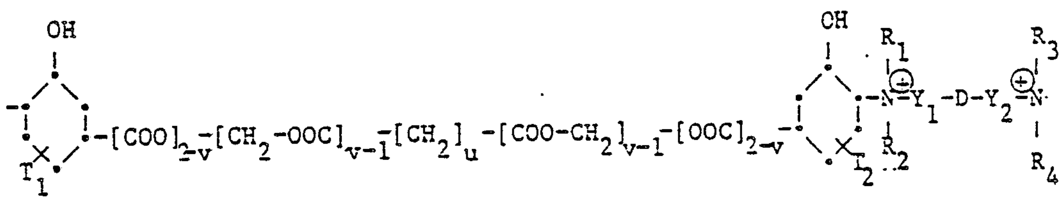

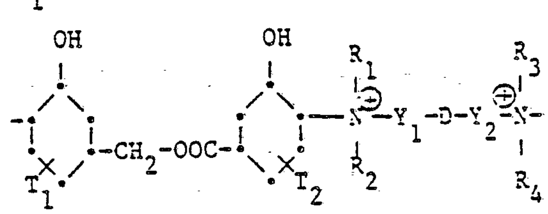
,

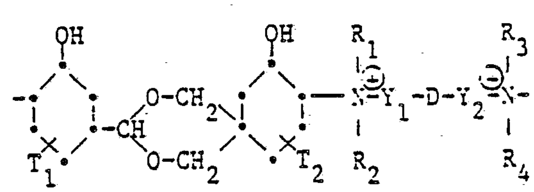
oder

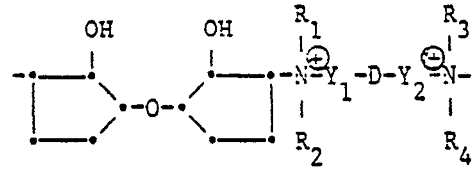

aufweisen, mit einem Säureanhydrid oder Säureanhydridgemisch der Formeln

$M_6$—OC—O—CO—$M_6$
$M_7$—OC—O—CO—$M_7$ und/oder
$M_8$—OC—O—CO—$M_8$

zu den entsprechenden Estern, die Einheiten einer der Formeln

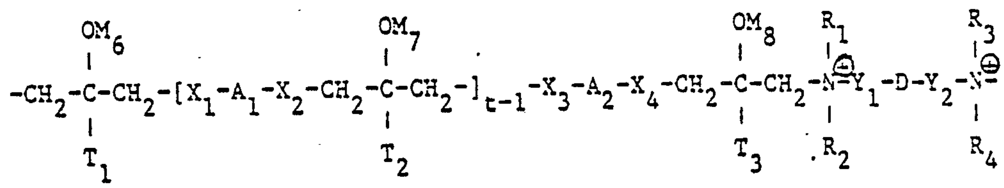

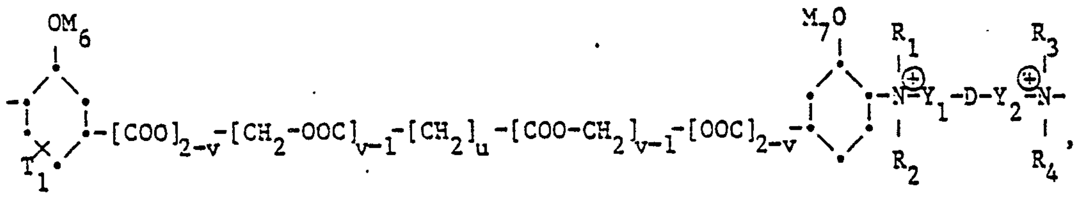,

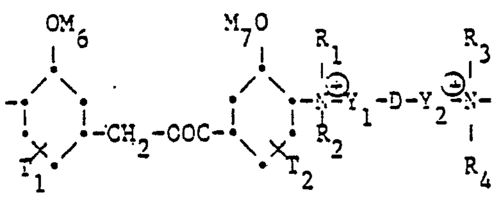,

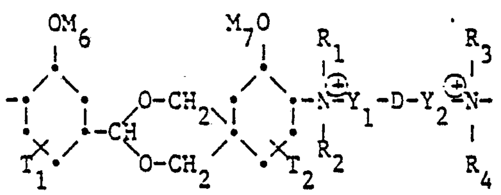

oder

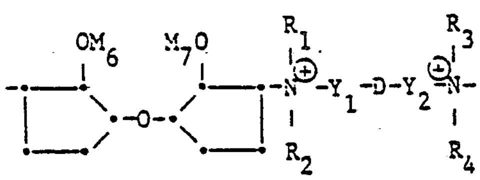

aufweisen, gegebenenfalls weiter umsetzt, wobei in den angegebenen Formeln $Z_1$ und $Z_2$ ein Tensidanion, ein Anion einer Fettsäure, ein Anion von organischen Säuren mit 1 bis 4 Kohlenstoffatomen, ein Anion von aromatischen Sulfonsäuren mit 6 bis 9 Kohlenstoffatomen oder ein Anion von Mineralsäuren oder deren Alkylester mit 1 bis 4 Kohlenstoffatomen im Alkylrest, $Z_3$ Halogen und $M_6$, $M_7$ und $M_8$ je Acyl mit 2 bis 5 Kohlenstoffatomen bedeuten und $A_1$, $A_2$, D, $R_1$, $R_2$, $R_3$, $R_4$, $T_1$, $T_2$, $T_3$, $X_1$, $X_2$, $X_3$, $X_4$, $Y_1$, $Y_2$, t, u und v die Anspruch 1 angegebenen Bedeutungen haben.

8. Verfahren nach Anspruch 7, dadurch gekennzeichnet, dass man ein Diepoxyd oder Diepoxydgemisch der Formel

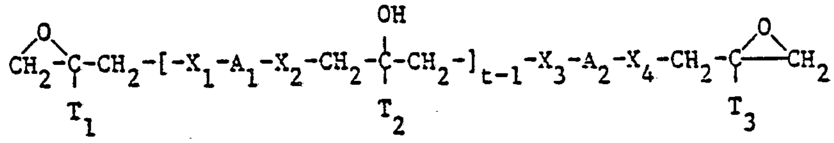,

mit einem ditertiären Aminsalz der Formel

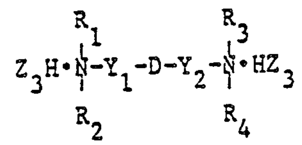

umsetzt, oder ein Diepoxyd oder Diepoxydgemisch der vorstehend angegebenen Formel mit einem Halogenwasserstoff der Formel

$Z_3H$

in ein Dihalogenhydrin oder Dihalogenhydringemisch der Formel

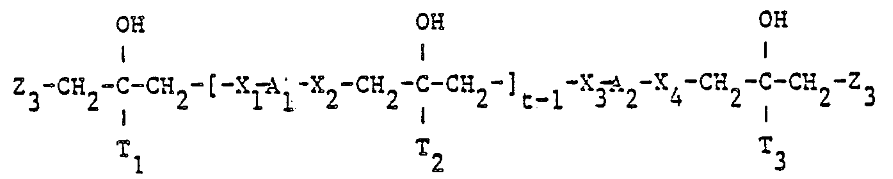

überführt und anschliessend mit einem ditertiären Amin der Formel

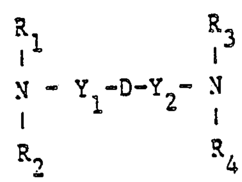

umsetzt, und die erhaltenen Umsetzungsprodukte oder Umsetzungsproduktgemische, die Einheiten der Formel

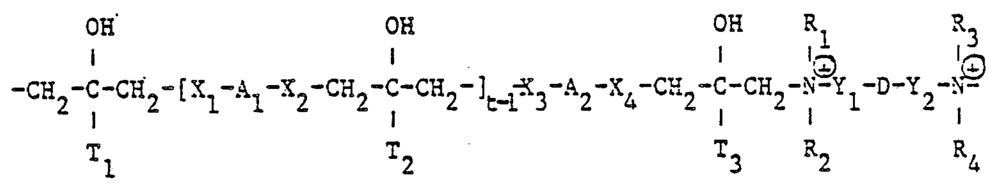

aufweisen, mit einem Säureanhydrid oder Säureanhydridgemisch der Formeln

$M_6$—OC—O—CO—$M_6$,
$M_7$—OC—O—CO—$M_7$ und/oder
$M_8$—OC—O—CO—$M_8$

zu den entsprechenden Estern, die Einheiten der Formel

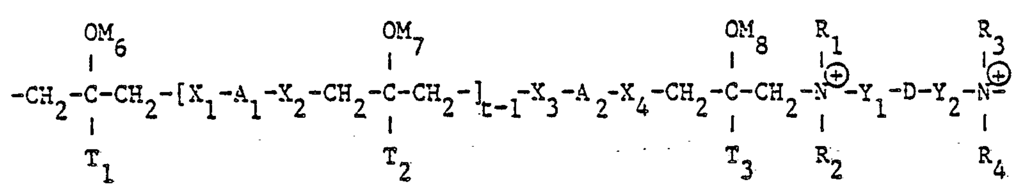

aufweisen, gegebenenfalls weiter umsetzt, wobei in den angegebenen Formeln $Z_3$ Halogen und $M_6$, $M_7$ und $M_8$ je Acyl mit 2 bis 5 Kohlenstoffatomen bedeuten und $A_1$, $A_2$, D, $R_1$, $R_2$, $R_3$, $R_4$, $T_1$, $T_2$, $T_3$, $X_1$, $X_2$, $X_3$, $X_4$, $Y_1$, $Y_2$ und t die in Anspruch 1 angegebenen Bedeutungen haben.

9. Verwendung der Ammoniumsalze gemäss einem der Ansprüche 1 bis 6 als kosmetisches Mittel, als Auswaschmittel für kationisch gefärbte oder bedruckte Textilmaterialien, als Papierleimungsmittel, als Fixierungsmittel für anionisch gefärbte, cellulosehaltige Materialien und als algizide, fungizide und bakterizide Mittel.

10. Verfahren zur Behandlung von Haaren, dadurch gekennzeichnet, dass man mindestens ein Ammoniumsalz gemäss einem der Ansprüche 1 bis 6 auf das Haar aufbringt.

11. Verfahren zum Auswaschen von Textilmaterial in wässrigem Medium, das mit mindestens einem kationischen Farbstoff bedruckt oder gefärbt ist, wobei bedrucktes oder gefärbtes Material oder Materialien, die jeweils mit verschiedenartigen kationischen Farbstoffen bedruckt oder gefärbt sind, oder ein einzelnes, stellenweise bedrucktes Material ausgewaschen werden, dadurch gekennzeichnet, dass das Waschwasser mindestens ein Ammoniumsalz gemäss einem der Ansprüche 1 bis 6 enthält.

12. Verfahren zum Leimen von Papier, in welchem die Leimflotte in die Faserstoffsuspension eingearbeitet wird, oder das Papier mit der Leimflotte imprägniert und getrocknet wird, dadurch gekennzeichnet, dass die Leimflotte mindestens ein Ammoniumsalz gemäss einem der Ansprüche 1 bis 6 enthält.

13. Verfahren zum Nachbehandeln von cellulosehaltigen Materialien, die mit anionischen Farbstoffen gefärbt sind, dadurch gekennzeichnet, dass man mindestens ein Ammoniumsalz gemäss einem der Ansprüche 1 bis 6 als wässrige Lösung einsetzt.

14. Verfahren zur Behandlung von Substraten, dadurch gekennzeichnet, dass man mindestens ein Ammoniumsalz gemäss einem der Ansprüche 1 bis 6 als algizides, bakterizides und/oder fungizides Mittel einsetzt.

**Claims**

1. A quaternary ammonium salt which contains units which contain diepoxide and diamine radicals and have the formula

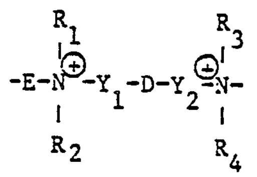

in which E is a divalent bridge member which has one of the formulae

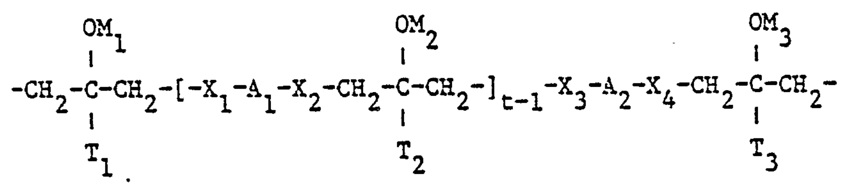

,

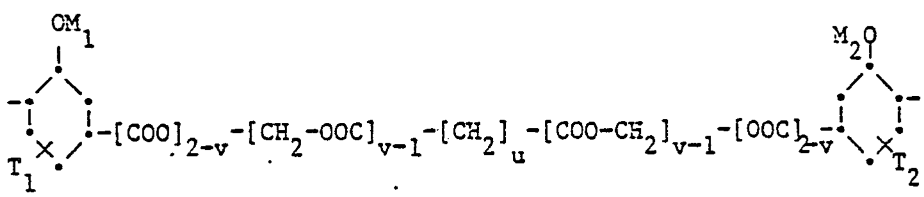

,

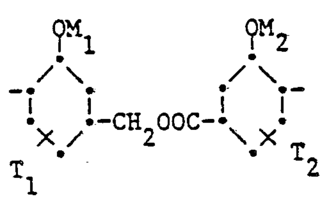

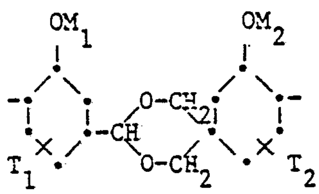

or

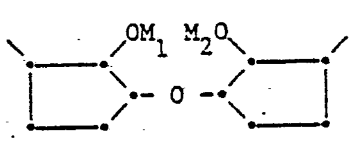

in which t is a number from 1 to 4, u is an integer from 1 to 8 and v is 1 or 2 and $R_1$, $R_2$, $R_3$ and $R_4$ are identical or different and are alkyl, cycloalkyl or alkenyl having not more than 8 carbon atoms, which radicals are unsubstituted or substituted by hydroxyl, alkoxy, alkylthio or cyano, or are aryl or aralkyl which are unsubstituted or substituted by hydroxyl, hydroxyalkyl, alkoxy, alkoxyalkyl, alkyl, alkylthio, cyano or halogen, or ($R_1$ and $R_2$) and/or ($R_3$ and $R_4$) together with the nitrogen atom to which they are attached form a heterocyclic ring having 5 or 6 ring members, $M_1$, $M_2$ and $M_3$ are each acyl having 2 to 5 carbon atoms or hydrogen, $Y_1$ and $Y_2$ are identical or different from each other and have the formula

$$-C_mH_{2m}-$$

in which m is an integer from 1 to 12, the sum of the m's in $Y_1$ and $Y_2$ is not less than 3 and, when m is 1, the bond to the bridge member D is not made via a nitrogen atom or oxygen atom, or $Y_1$ and $Y_2$ are phenylene which is unsubstituted or substituted by halogen, hydroxyl, alkyl, haloalkyl, hydroxyalkyl or alkoxy, D is a divalent bridge member of one of the formulae

$-NHCONH-$, $-NHCOD_1CONH-$, $-CONH-$,

$-OCONH-$, $-COO-$, $-COD_2CO-$,

$-O\overset{\overset{O}{\|}}{C}-D_3-\overset{\overset{O}{\|}}{C}O-$ or $-O\overset{\overset{O}{\|}}{C}-NH-D_4-HN-\overset{\overset{O}{\|}}{C}O-$

in which $D_1$ is a direct bond, alkylene, alkenylene, arylene, heteroarylene, diaminoalkylene, diaminoarylene or unsubstituted or halogen-substituted dioxyalkylene, polyoxyalkyleneoxy or dioxyarylene, $D_2$ is diaminoalkylene or unsubstituted or halogen-substituted dioxyalkylene, polyoxyalkyleneoxy or dithioalkylene, $D_3$ is arylene and $D_4$ is alkylene or arylene, $T_1$, $T_2$ and $T_3$ are each hydrogen or methyl, $X_1$, $X_2$, $X_3$ and $X_4$ are each —COO—, —OOC—, —O— or a direct bond and $A_1$ and $A_2$ are each a heterocyclic ring which has 5 or 6 ring members and 2 nitrogen atoms and can be substituted by alkyl, or alkylene which can be interrupted by hetero-atoms, or cycloalkylene, cycloalkenylene, arylene or aralkylene which have 1 or 2 rings and can be substituted by alkyl or halogen, the bridge members in ring form being bonded via a direct bond, via a hetero-atom or via an alkylene bridge which can be interrupted by hetero-atoms.

2. An ammonium salt according to claim 1, which contains units of the formula

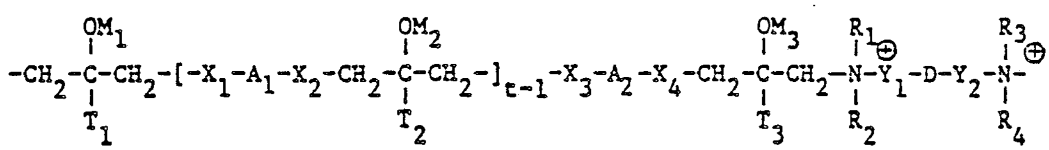

wherein $A_1$ and $A_2$, D, $M_1$, $M_2$, $M_3$, $R_1$, $R_2$, $R_3$, $R_4$, $T_1$, $T_2$, $T_3$, $X_1$, $X_2$, $X_3$, $X_4$, $Y_1$, $Y_2$ and t are as defined in claim 1.

3. An ammonium salt according to claim 1, which contains units of one of the formulae

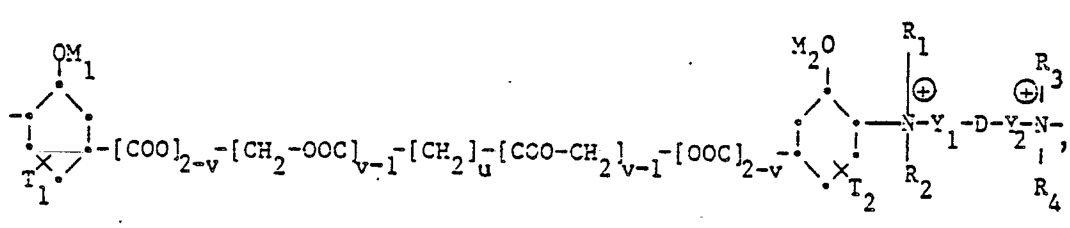

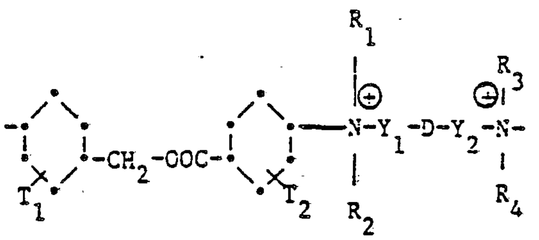

,

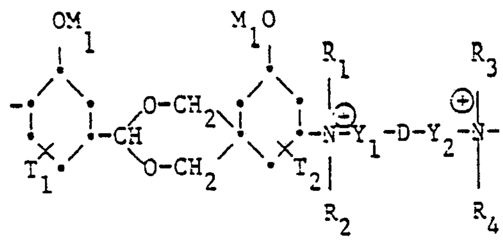

or

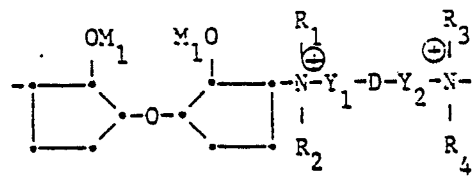

in which D, $M_1$, $M_2$, $R_1$, $R_2$, $R_3$, $R_4$, $T_1$, $T_2$, $Y_1$, $Y_2$, u and v are as defined in claim 1.

4. An ammonium salt according to any one of claims 1 to 3, which contains units of one of the formulae

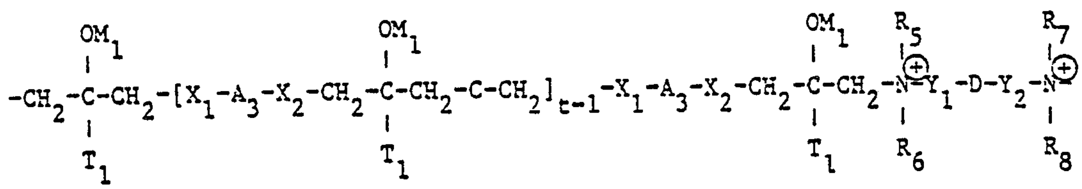

,

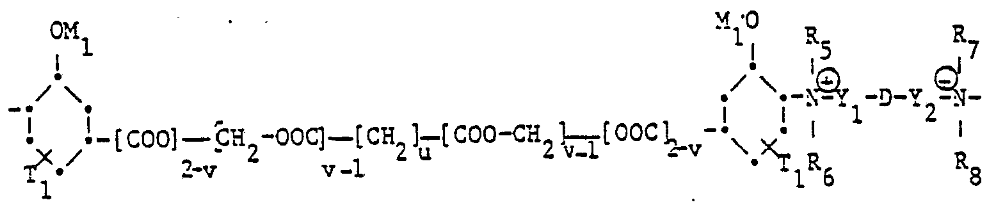

,

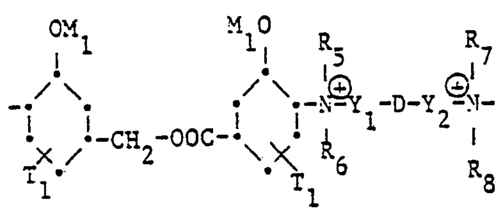

,

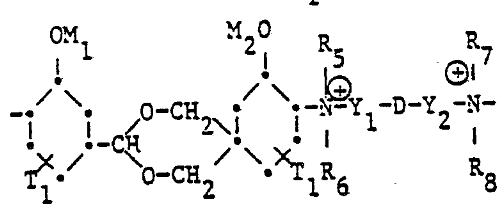

or

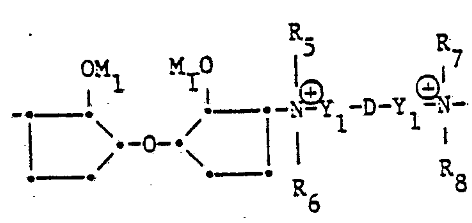

in which $R_5$, $R_6$, $R_7$ and $R_8$ are identical to or different from one another and are alkyl, hydroxyalkyl, alkoxyalkyl, alkylthioalkyl or cyanoalkyl having a total of 1 to 8 carbon atoms, cyclopentyl, cyclohexyl, $C_2$-$C_4$-alkenyl, unsubstituted phenyl or benzyl, or phenyl or benzyl substituted by hydroxyl, cyano, chlorine, bromine or alkyl, hydroxyalkyl, alkoxy, alkylthio or alkoxyalkyl, each having 1 or 2 carbon atoms in the alkyl moiety and alkoxy moiety ; or ($R_5$ and $R_6$) and ($R_7$ and $R_8$), together with the nitrogen atom to which they are attached, form a heterocyclic ring of one of the formulae

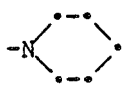 , 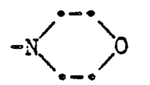 , 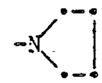 or 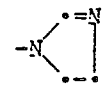

$A_3$ is a heterocyclic ring having 5 or 6 ring members and 2 nitrogen atoms and is unsubstituted or substituted by methyl, ethyl or isopropyl, or is $C_2$-$C_{34}$ alkylene which can be interrupted by oxygen atoms, $C_6$-$C_{10}$ cycloalkenylene which can be substituted by methyl or ethyl, or $C_{16}$-$C_{18}$ cycloalkylene which has one or two rings or $C_6$-$C_{18}$ arylene which has one or two rings or $C_8$-$C_{26}$ aralkylene which has one or two rings, the two latter radicals being unsubstituted or substituted by bromine or chlorine, and D, $M_1$, $T_1$, $X_1$, $X_2$, $Y_1$, $Y_2$, t, u and v are as defined in claim 1.

5. An ammonium salt according to claim 4, which contains units of one of the formulae

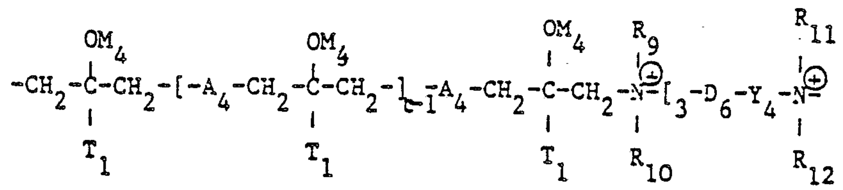 ,

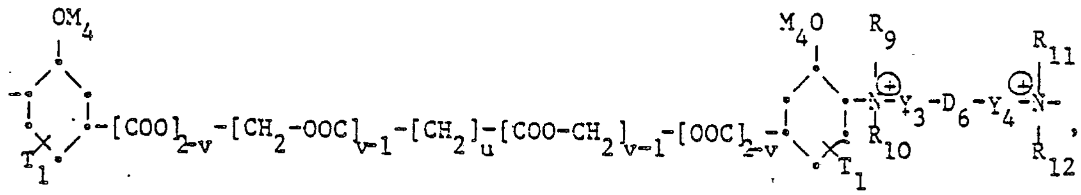 ,

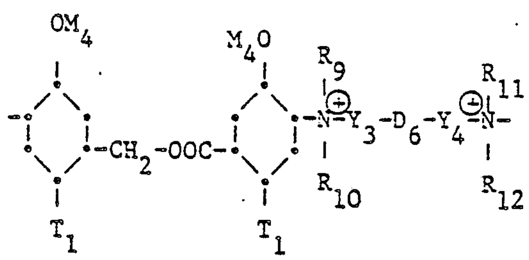 ,

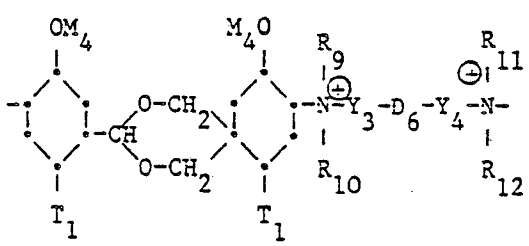

or

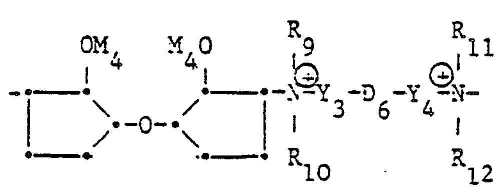

in which $D_6$ has one of the formulae

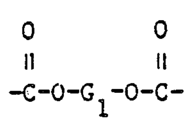 , 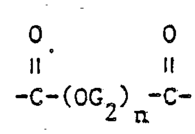 , 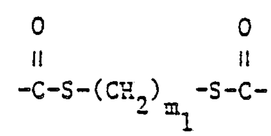 ,

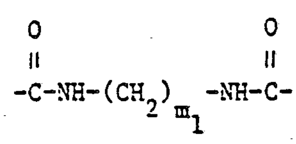 , 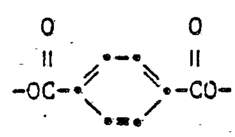 , 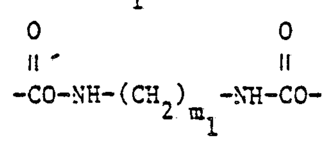 ,

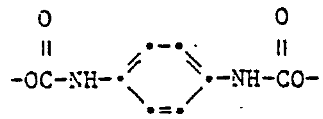 , $-HN-OC-CO-NH-$, 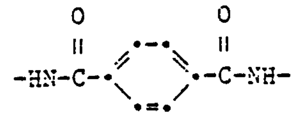 ,

$-HN-CO-(CH_2)_{m_1}-CO-NH-$ , $-NH-CO-NH-$ , $-CONH-$ ,

$-OCONH$ or $-COO-$

in which $G_1$ is $C_2$-$C_{12}$ alkylene, n is an integer from 2 to 15 and $m_1$ is an integer from 2 to 12, $R_9$, $R_{10}$, $R_{11}$ and $R_{12}$ are identical to or different from one another and are phenyl, benzyl or $C_1$-$C_4$ alkyl, or ($R_9$ and $R_{10}$) and ($R_{11}$ and $R_{12}$), together with the nitrogen atom to which they are attached, form a piperidine ring, $M_4$ is propionyl, acetyl or hydrogen, $A_4$ is a divalent radical of one of the formulae

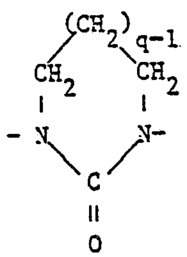 , 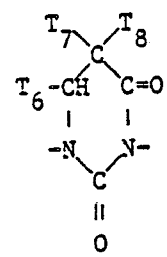 ,

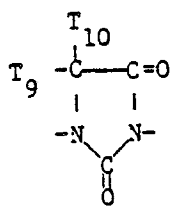 , $-O-(OC)_{q-1}-Q-(CO)_{q-1}-O-$

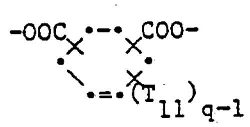 , 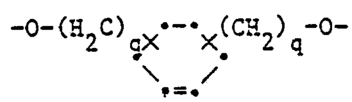 ,

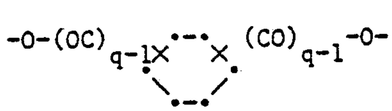 , 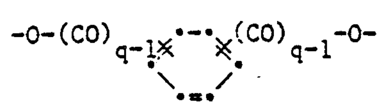

or

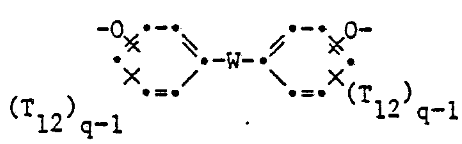

in which Q is $C_2$-$C_8$ alkylene which can be interrupted by 1 or 2 oxygen atoms, W is a direct bond, $—SO_2—$ or $C_1$-$C_4$ alkylene, $T_6$, $T_7$, $T_8$ and $T_9$ are each hydrogen or methyl, $T_{10}$ is hydrogen, methyl, ethyl or isopropyl, $T_{11}$ is methyl or ethyl, $T_{12}$ is chlorine or bromine and q is 1 or 2, and $Y_1$, $Y_2$, $T_1$, t, u and v are as defined in claim 1.

6. An ammonium salt according to claim 5, which contains units of one of the formulae

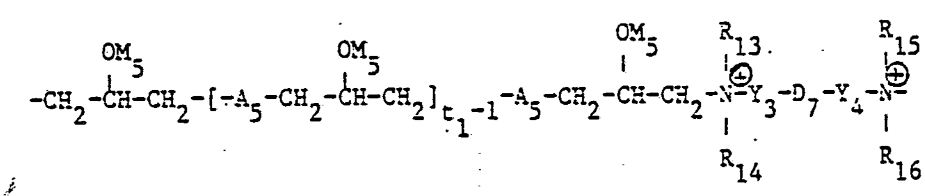

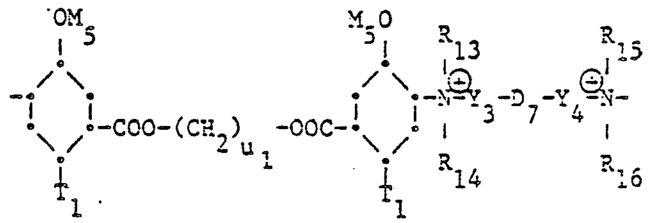

,

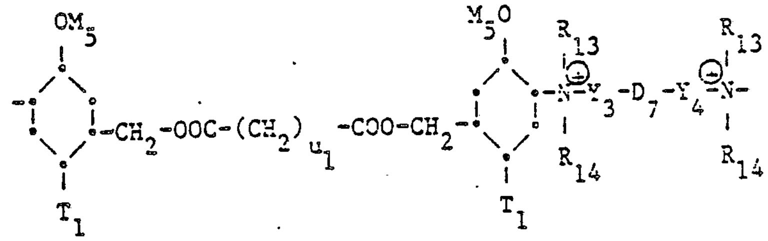

,

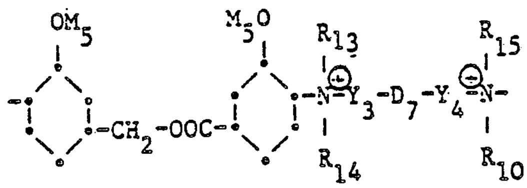

,

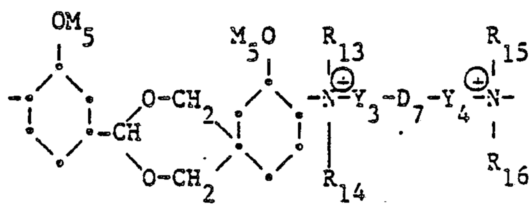

or

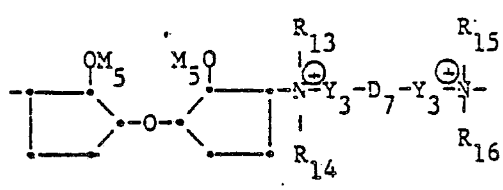

in which $R_{13}$, $R_{14}$, $R_{15}$ and $R_{16}$ are each benzyl, methyl or ethyl, or ($R_{13}$ and $R_{14}$) and ($R_{15}$ and $R_{16}$), together with the nitrogen atom to which they are attached, form a piperidine ring, $M_5$ is acetyl or hydrogen, $T_1$ is methyl or hydrogen, $Y_3$ and $Y_4$ are each $C_1$-$C_6$ alkylene or phenylene, $t_1$ is any desired number from 1 to 3 und u is an integer from 1 to 4, $A_5$ has one of the formulae

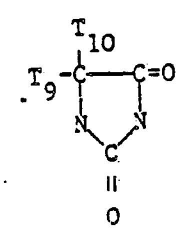

,

$-O-(OC)_{q-1}-Q-(CO)_{q-1}-O-$ ,

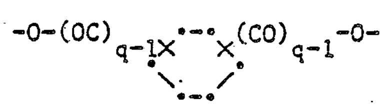

or

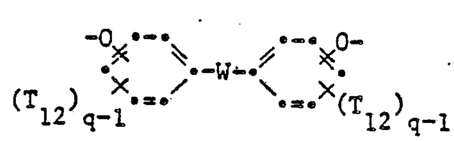

in which Q is $C_2$-$C_8$ alkylene, which can be interrupted by 1 or 2 oxygen atoms, W is a direct bond, —$SO_2$— or $C_1$-$C_4$ alkylene, $T_9$ is hydrogen or methyl, $T_{10}$ is hydrogen, methyl, ethyl or isopropyl, $T_{12}$ is chlorine or bromine and q is 1 or 2, and $D_7$ has one of the formulae

$-NH-CO-NH-$, $-CO-NH-$, 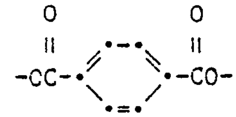

$-HN-OC-CO-NH-$, 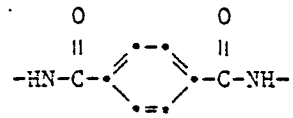 or

$-HN-CO-(CH_2)_{m_1}-CO-NH-$

in which $m_1$ is an integer from 2 to 12.

7. A process for the preparation of an ammonium salt according to any one of claims 1 to 6, which comprises reacting a diepoxide or a diepoxide mixture of one of the formulae

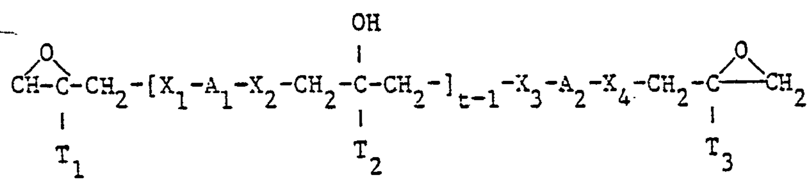

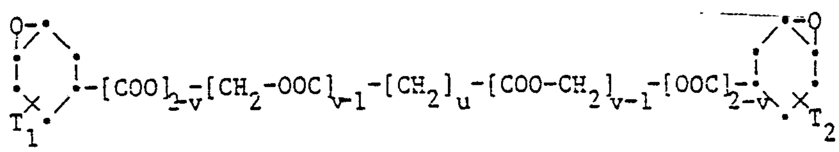

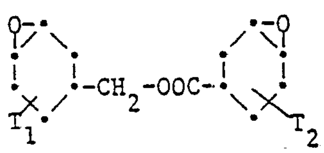 , 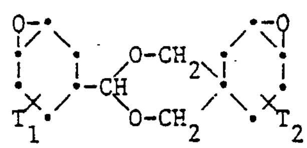 or

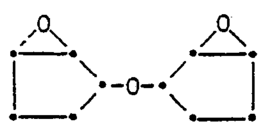

with a di-tertiary amine salt of the formula

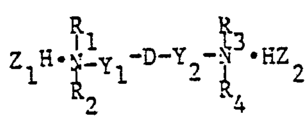

or converting a diepoxide or diepoxide mixture of one of the formulae indicated above to a dihalohydrin or dihalohydrin mixture of one of the formulae

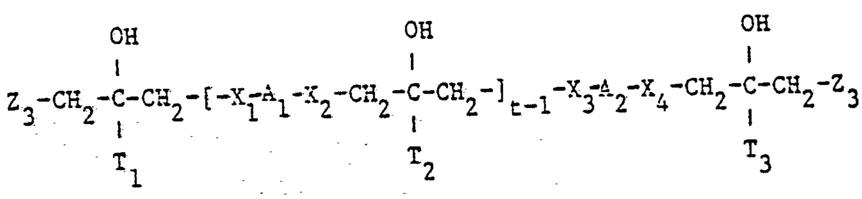

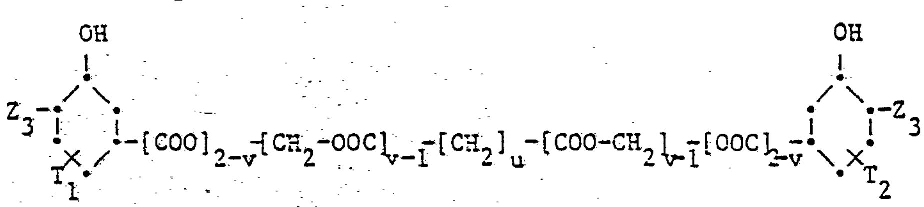 ,

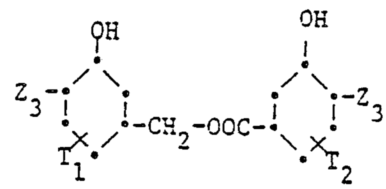

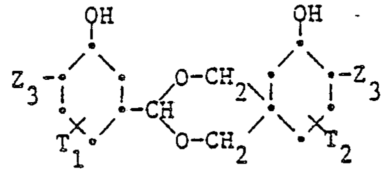

or

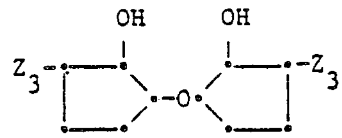

with a hydrogen halide of the formula

$Z_3H$

and then reacting the said dihalohydrin or dihalohydrin mixture mixture with a di-tertiary amine of the formula

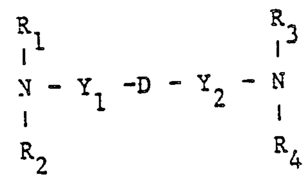

and, if desired, further reacting the resulting reaction products or mixtures of reaction products, which contain units of one of the formulae

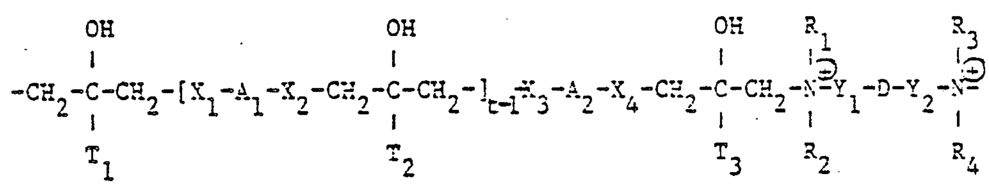

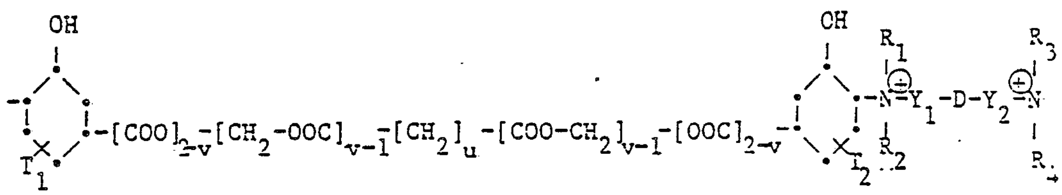

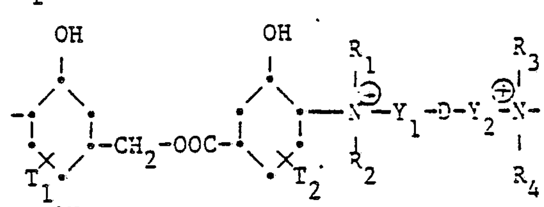

,

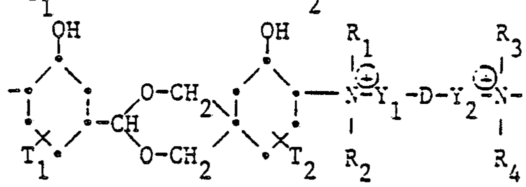

or

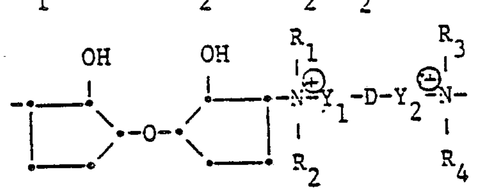

with an acid anhydride or mixture of acid anhydrides of the formulae

$M_6$—OC—O—CO—$M_6$,
$M_7$—OC—O—CO—$M_7$ and/or
$M_8$—OC—O—CO—$M_8$

to give the corresponding esters which contain units of one of the formulae

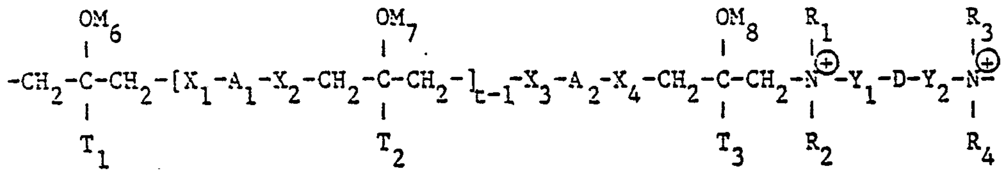
,

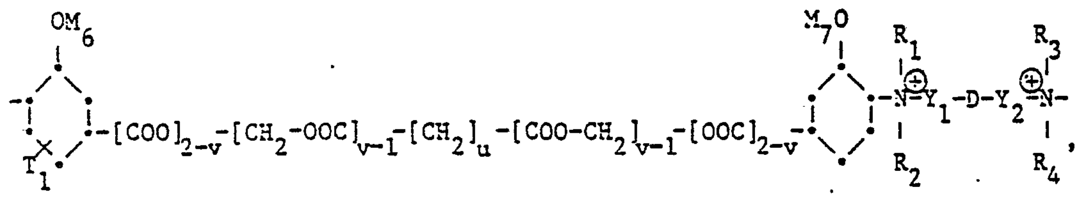
,

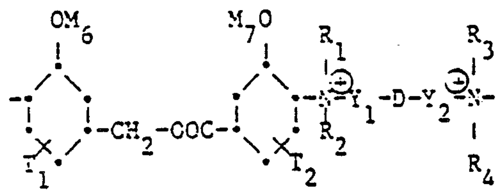
,

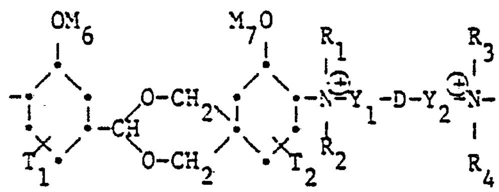
or

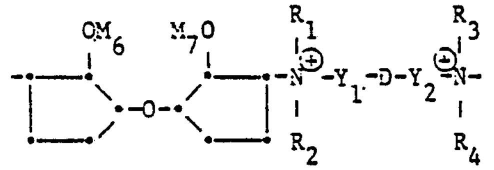

in which formulae above $Z_1$ and $Z_2$ are a surfactant anion, an anion of a fatty acid, an anion of organic acids having 1 to 4 carbon atoms, an anion of aromatic sulfonic acids having 6 to 9 carbon atoms or an anion of mineral acids or of alkyl esters thereof having 1 to 4 carbon atoms in the alkyl radical, $Z_3$ is halogen and $M_6$, $M_7$ and $M_8$ are each acyl having 2 to 5 carbon atoms, and $A_1$, $A_2$, D, $R_1$, $R_2$, $R_3$, $R_4$, $T_1$, $T_2$, $T_3$, $X_1$, $X_2$, $X_3$, $X_4$, $Y_1$, $Y_2$, t, u and v are as defined in claim 1.

8. A process according to claim 7, which comprises reacting a diepoxide or a mixture of diepoxides of the formula

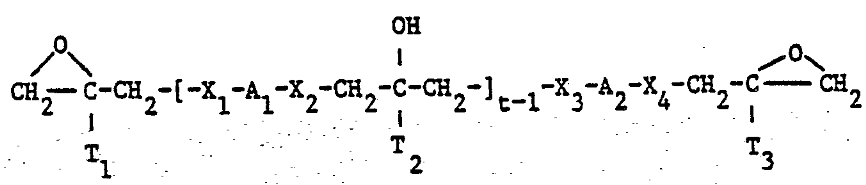
,

with a di-tertiary amine salt of the formula

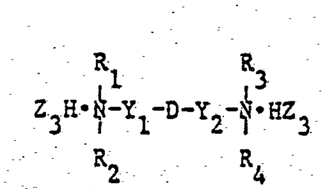

or converting a diepoxide or mixture of diepoxides of the formula indicated above to a dihalogenohydrin or mixture of dihalogenohydrins of the formula

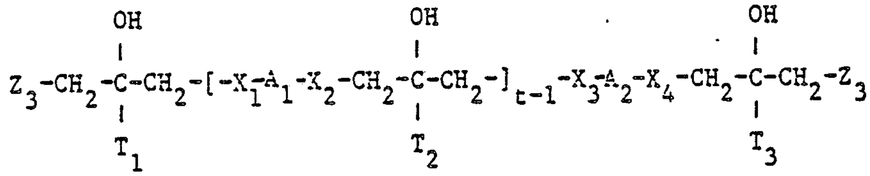

with a hydrogen halide of the formula

$Z_3H$

and then reacting the said dihalohydrin or dihalo hydrin mixture with a di-tertiary amine of the formula

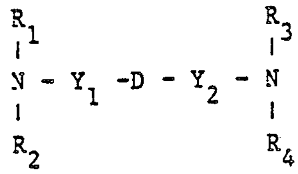

and, if desired, further reacting the resulting reaction products or mixtures of reaction products which contain units of the formula

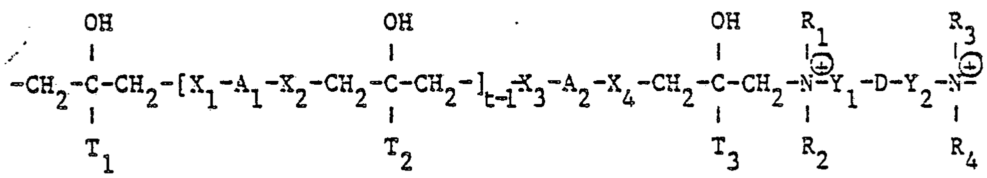 ,

with an acid anhydride or mixture of acid anhydrides of the formulae

$M_6$—OC—O—CO—$M_6$,
$M_7$—OC—O—CO—$M_7$ and/or
$M_8$—OC—O—CO—$M_8$

to give the corresponding esters which contain units of the formula

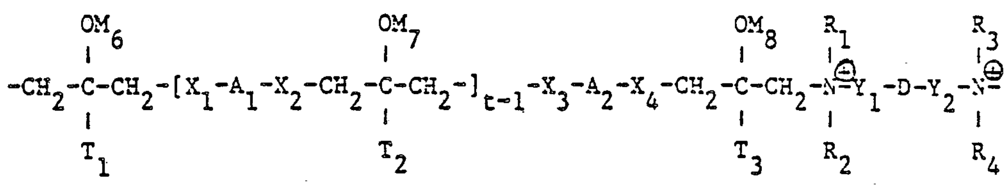

in which formulae above $Z_3$ is halogen and $M_6$, $M_7$ and $M_8$ are each acyl having 2 to 5 carbon atoms and $A_1$, $A_2$, D, $R_1$, $R_2$, $R_3$, $R_4$, $T_1$, $T_2$, $T_3$, $X_1$, $X_2$, $X_3$, $X_4$, $Y_1$, $Y_2$ and t are as defined in claim 1.

9. The use of ammonium salt according to any one of claims 1 to 6 as a cosmetic, as an agent for washing-off textile materials dyed or printed with cationic dyes, as a paper size, as a fixing agent for cellulosic materials dyed with anionic dyes or as an algicide, fungicide or bactericide.

10. A process for the treatment of hair, which comprises applying at least one ammonium salt according to any one of claims 1 to 6 to the hair.

11. A process for washing-off textile material in an aqueous medium, which material has been printed or dyed with at least one cationic dye, the material washed-off being printed or dyed material or materials which have each been printed or dyed with cationic dyes of different types or a single material printed in parts, wherein the wash water contains at least one ammonium salt according to any one of claims 1 to 6.

12. A process for sizing paper, in which the size liquor is incorporated in the fibre suspension, or the paper is impregnated with the size liquor and dried, wherein the size liquor contains at least one ammonium salt according to any one of claims 1 to 6.

13. A process for the after-treatment of cellulosic materials which have been dyed with anionic dyes, wherein at least one ammonium salt according to any one of claims 1 to 6 is employed in the form of an aqueous solution.

14. A process for the treatment of substrates, wherein at least one ammonium salt according to any one of claims 1 to 6 is employed as an algicide, bactericide and/or fungicide.

## Revendications

1. Sels d'ammonium quaternaires, caractérisés par le fait qu'ils présentent des unités contenant des restes diépoxy et diamine et ayant la formule

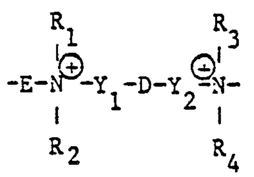

dans laquelle E est un chaînon de pontage divalent, qui répond à l'une des formules

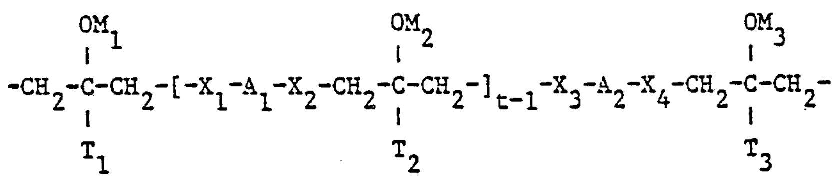

,

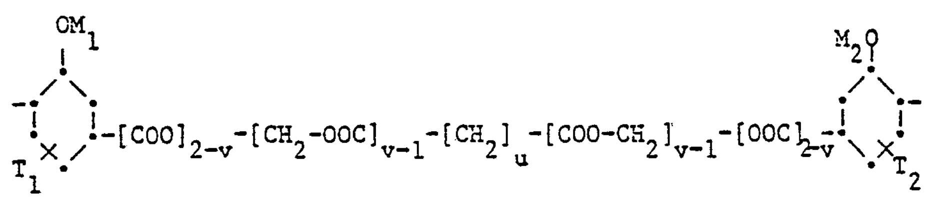

,

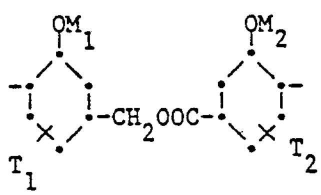

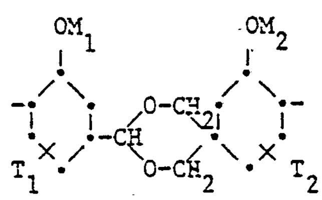

ou

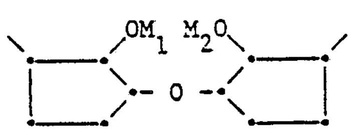

dans lesquelles t est un nombre de 1 à 4 ; u un nombre entier de 1 à 8, et v est 1 ou 2, $R_1$, $R_2$, $R_3$ et $R_4$ sont identiques ou différents les uns des autres et désignent chacun un alkyle, cycloalkyle ou alcényle à huit atomes de carbone au maximum éventuellement substitués par un hydroxyle, alcoxy, alkylthio ou cyano ou un aryle ou arylalkyle éventuellement substitué par un hydroxyle, hydroxyalkyle, alcoxy, alcoxyalkyle, alkyle, alkylthio, cyano ou halogène, ou ($R_1$ et $R_2$) et/ou ($R_3$ et $R_4$) forment conjointement avec l'atome d'azote, avec lequel ils sont liés, un noyau hétérocyclique à 5 ou 6 chaînons ; $M_1$, $M_2$ et $M_3$ désignant chacun un acyle à 2 à 5 atomes de carbone ou l'hydrogène ; $Y_1$ et $Y_2$ sont identiques ou différents l'un de l'autre et correspondent à la formule

$$—C_mH_{2m}—$$

dans laquelle m est un nombre entier de 1 à 12, la somme de m dans $Y_1$ et $Y_2$ s'élevant au minimum à 3 : et

pour m étant égal à 1 la liaison au chaînon de pontage D n'ayant pas lieu par l'intermédiaire d'un atome d'azote ou d'oxygène, ou $Y_1$ et $Y_2$ désignent des groupes phénylène éventuellement substitués par un halogène, hydroxyle, alkyle, halogénoalkyle, hydroxyalkyle ou alcoxy ; D correspond à un chaînon de pontage divalent de l'une des formules

$-NHCONH-$, $-NHCOD_1CONH-$, $-CONH-$,

$-OCONH-$, $-COO-$, $-COD_2CO-$,

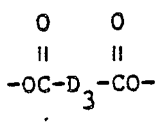

ou

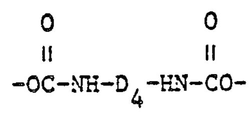

dans lesquelles $D_1$ est la liaison directe, un alkylène, alcénylène, arylène, hétéroarylène, diaminoalkylène, diaminoarylène, un dioxyalkylène, polyoxyalkylèneoxy ou dioxyarylène, éventuellement halogénés ; $D_2$ est un diaminoalkylène, un dioxyalkylène, polyoxyalkylèneoxy ou dithioalkylène, éventuellement halogénés ; $D_3$ est un arylène, et $D_4$ est un alkylène ou arylène ; $T_1$, $T_2$ et $T_3$ sont chacun un hydrogène ou méthyle ; $X_1$, $X_2$, $X_3$ et $X_4$ sont chacun —COO—, —OOC—, —O— ou la liaison directe, et $A_1$ et $A_2$ désignent chacun un noyau hétérocyclique à 5 ou 6 chaînons et deux atomes d'azote éventuellement substitué par un alkyle, un alkylène éventuellement interrompu par un hétéroatome, un cycloalkylène, cycloalcénylène, arylène ou arylalkylène présentant un ou deux noyaux et éventuellement substitué par un alkyle ou un halogène, les chaînons de pontage cycliques étant mutuellement reliés par une liaison directe, par un hétéroatome ou par un pont alkylène éventuellement interrompu par un hétéroatome.

2. Sels d'ammonium selon la revendication 1, caractérisés par le fait qu'ils présentent des unités de formule

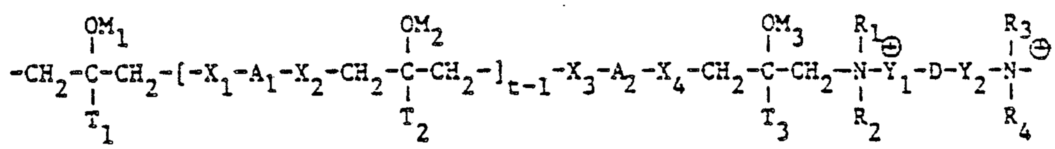

dans laquelle $A_1$ et $A_2$, D, $M_1$, $M_2$, $M_3$, $R_1$, $R_2$, $R_3$, $R_4$, $T_1$, $T_2$, $T_3$, $X_1$, $X_2$, $X_3$, $X_4$, $Y_1$, $Y_2$ et t sont définis comme spécifié dans la revendication 1.

3. Sels d'ammonium selon la revendication 1, caractérisés par le fait qu'ils présentent des unités de l'une des formules

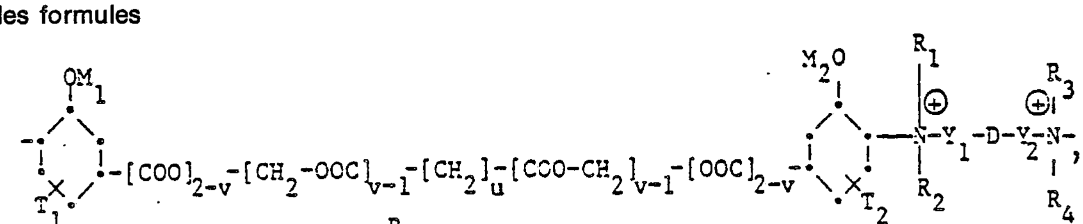

,

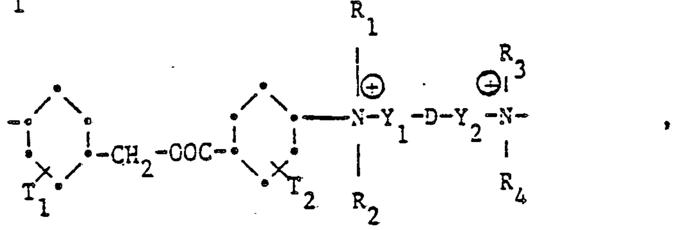

,

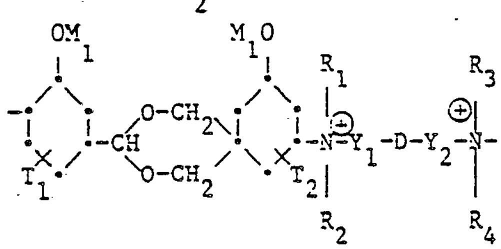

ou

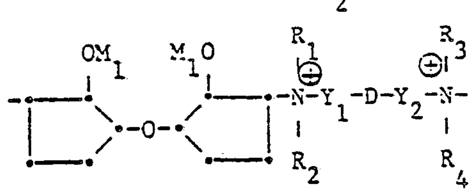

dans lesquelles D, $M_1$, $M_2$, $R_1$, $R_2$, $R_3$, $R_4$, $T_1$, $T_2$, $Y_1$, $Y_2$, u et v sont définis comme spécifié dans la revendication 1.

4. Sels d'ammonium selon l'une quelconque des revendications 1 à 3, caractérisés par le fait qu'ils présentent des unités de l'une des formules

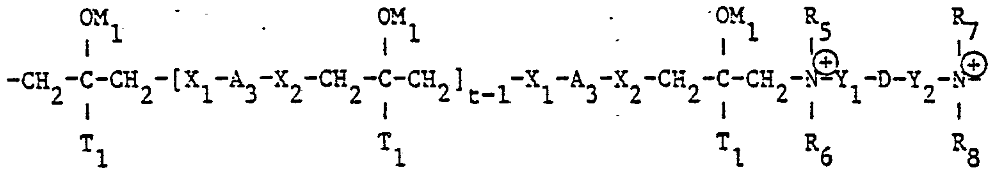

,

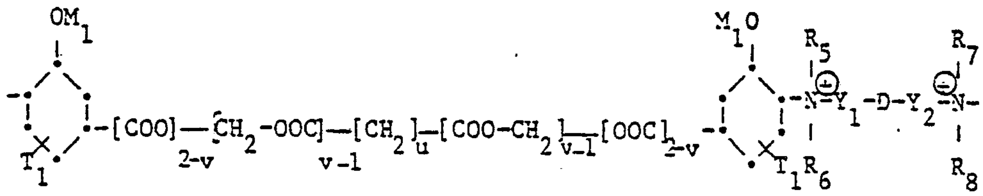

,

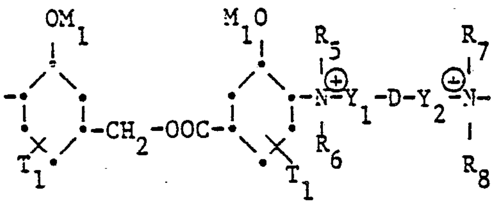

,

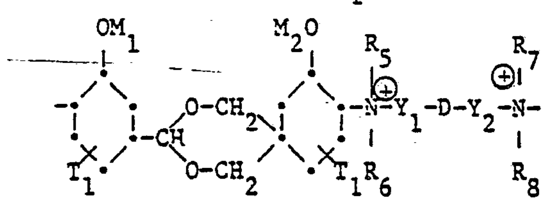

ou

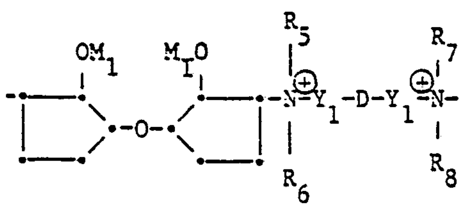

dans lesquelles $R_5$, $R_6$, $R_7$ et $R_8$ sont identiques ou différents les uns des autres et désignent chacun un alkyle hydroxyalkyle, alcoxyalkyle, alkylthioalkyle ou cyanoalkyle ayant au total 1 à 8 atomes de carbone, un cyclopentyle, cyclohexyle, alcényle ayant 2 à 4 atomes de carbone, un phényle ou benzyle non substitué, ou un phényle ou benzyle substitué par un hydroxyle, cyano, chlore, brome, alkyle, hydroxyalkyle, alcoxy, alkylthio, alcoxyalkyle ayant chacun 1 ou 2 atomes de carbone dans les parties alkyle et alcoxy ; ou ($R_5$ et $R_6$) et ($R_7$ et $R_8$) forment conjointement avec l'atome d'azote auquel ils sont liés, un noyau hétérocyclique de l'une des formules

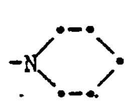 , 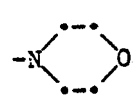 , 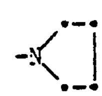 ou 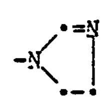

$A_3$ désigne un noyau hétérocyclique à 5 ou 6 chaînons et deux atomes d'azote éventuellement substitué par un méthyle, éthyle ou isopropyle, un alkylène à 2 à 34 atomes de carbone éventuellement interrompu par des atomes d'oxygène, un cycloalcénylène à 6 à 10 atomes de carbone éventuellement substitué par un méthyle ou éthyle ou un cycloalkylène à 6 à 18 atomes de carbone présentant un ou deux noyaux, un arylène à 6 à 18 atomes de carbone ou un arylalkylène à 8 à 26 atomes de carbone présentant chacun un ou deux noyaux et éventuellement substitués chacun par un atome de brome ou de chlore, et D, $M_1$, $T_1$, $X_1$, $X_2$, $Y_1$, $Y_2$, t, u et v sont définis comme spécifié dans la revendication 1.

5. Sels d'ammonium selon la revendication 4, caractérisés par le fait qu'ils présentent des unités de l'une des formules

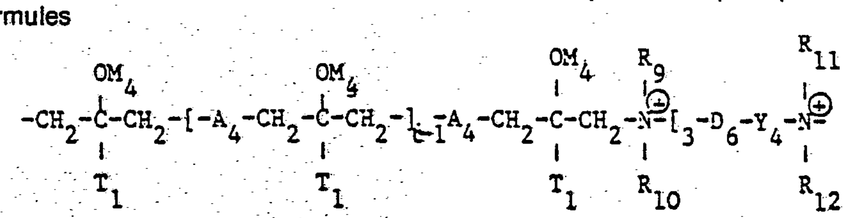

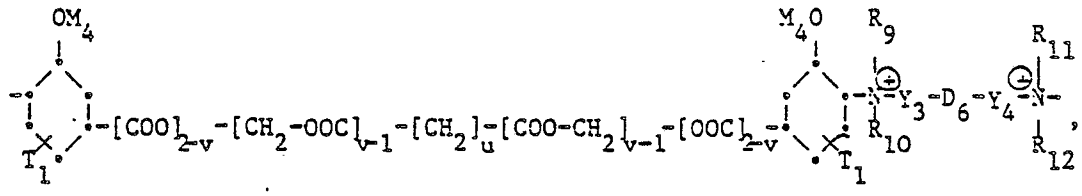

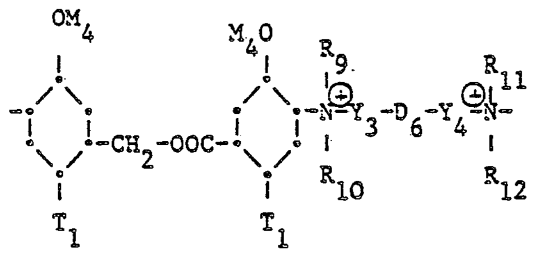

,

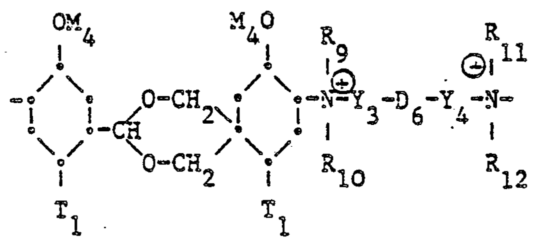

ou

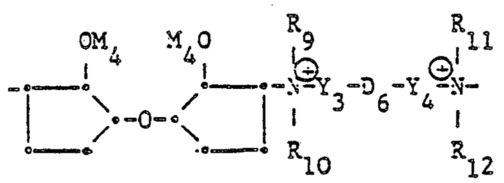

.

dans lesquelles $D_6$ correspond à l'une des formules

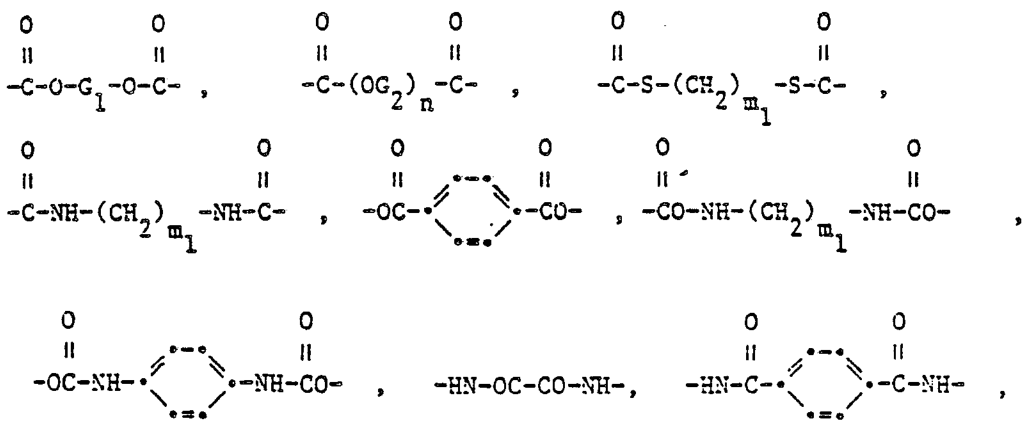

$-HN-CO-(CH_2)_{m_1}-CO-NH-$ , $-NH-CO-NH-$ , $-CONH-$ ,

$-OCONH$ ou $-COO-$

dans lesquelles $G_1$ est un alkylène à 2 à 12 atomes de carbone ; n est un nombre entier de 2 à 15, et $m_1$ est un nombre entier de 2 à 12 ; $R_9$, $R_{10}$, $R_{11}$ et $R_{12}$ étant identiques ou différents les uns des autres et désignant chacun un phényle, benzyle ou alkyle à 1 à 4 atomes de carbone, ou ($R_9$ et $R_{10}$) et ($R_{11}$ et $R_{12}$) forment conjointement avec l'atome d'azote auquel ils sont liés, un noyau pipéridine ; $M_4$ désigne un propionyle, acétyle ou l'hydrogène ; $A_4$ correspond à un reste divalent de l'une des formules

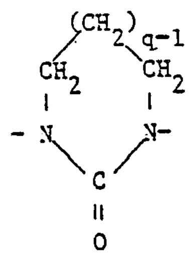 , 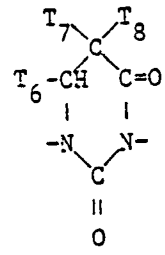 ,

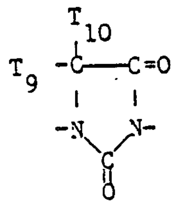 , $-O-(OC)_{q-1}-Q-(CO)_{q-1}-O-$

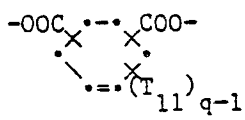 , 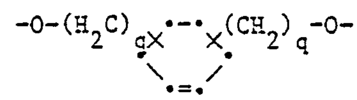 ,

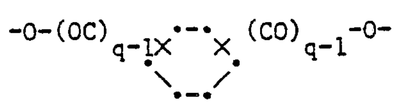 , 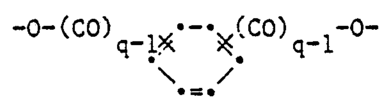

ou

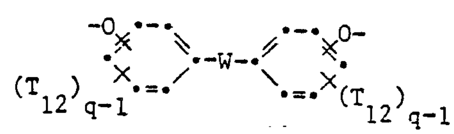

dans lesquelles Q désigne un alkylène à 2 à 8 atomes de carbone éventuellement interrompu par un ou deux atomes d'oxygène ; W désigne la liaison directe, $-SO_2-$ ou alkylène à 1 à 4 atomes de carbone ; $T_6$, $T_7$, $T_8$ et $T_9$ désignent chacun un hydrogène ou méthyle ; $T_{10}$ un hydrogène, méthyle, éthyle ou isopropyle ; $T_{11}$ un méthyle ou éthyle ; $T_{12}$ un chlore ou brome, et q est 1 ou 2, et $Y_1$, $Y_2$, $T_1$, t, u et v sont définis comme spécifié dans la revendication 1.

6. Sels d'ammonium selon la revendication 5, caractérisés par le fait qu'ils présentent des unités de l'une des formules

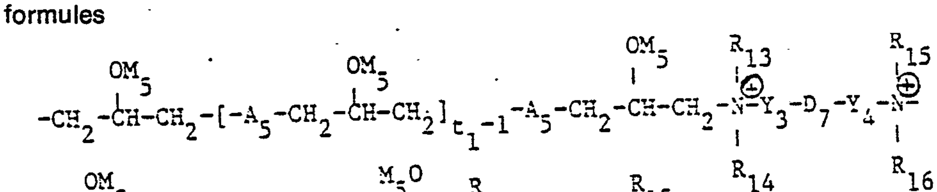

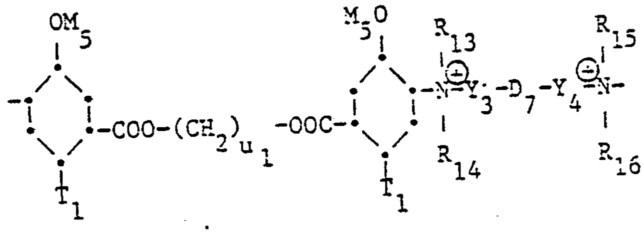 ,

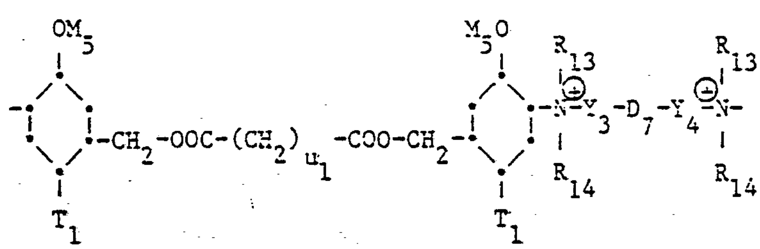 ,

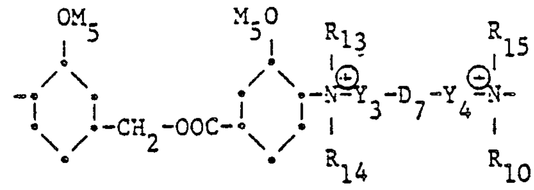

,

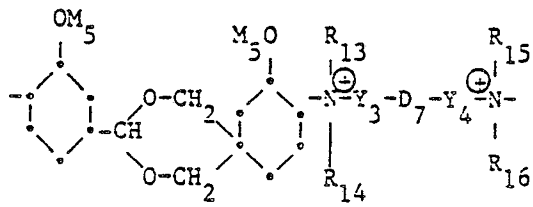

ou

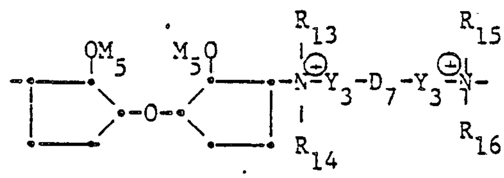

dans lesquelles $R_{13}$, $R_{14}$, $R_{15}$ et $R_{16}$ sont chacun un benzyle, méthyle ou éthyle, ou ($R_{13}$ et $R_{14}$) et ($R_{15}$ et $R_{16}$) forment conjointement avec l'atome d'azote auquel ils sont liés, un noyau pipéridine ; $M_5$ désigne un acétyle ou l'hydrogène ; $T_1$ un méthyle ou l'hydrogène ; $Y_3$ et $Y_4$ chacun un alkylène à 1 à 6 atomes de carbone ou un phénylène ; $t_1$ désigne un nombre quelconque de 1 à 3, et u désigne un nombre entier de 1 à 4 ; $A_5$ correspond à l'une des formules

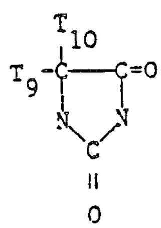

,

$-O-(OC)_{q-1}-Q-(CO)_{q-1}-O-$ ,

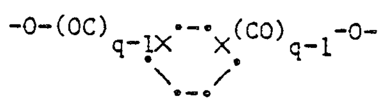

ou

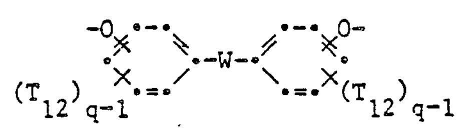

dans lesquelles Q désigne un alkylène à 2 à 8 atomes de carbone éventuellement interrompu par 1 ou 2 atomes d'oxygène ; W désigne la liaison directe, $-SO_2-$ ou un alkylène à 1 à 4 atomes de carbone ; $T_9$ désigne un hydrogène ou méthyle ; $T_{10}$ un hydrogène, méthyle, éthyle ou isopropyle ; $T_{12}$ un chlore ou un brome, et q est 1 ou 2, et $D_7$ correspond à l'une des formules

$-NH-CO-NH-$ , $-CO-NH-$ ,

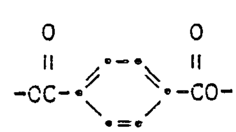

$-HN-OC-CO-NH-$ ,

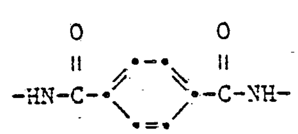

ou

$$-HN-CO-(CH_2)_{m_1}-CO-NH-$$

dans lesquelles $m_1$ est un nombre entier de 2 à 12.

7. Procédé de préparation des sels d'ammonium selon l'une quelconque des revendications 1 à 6, caractérisé par le fait que l'on fait réagir un diépoxyde ou un mélange de diépoxydes de l'une des formules

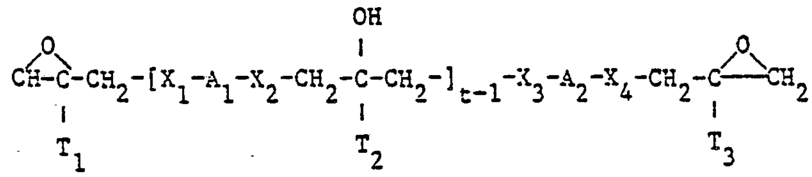

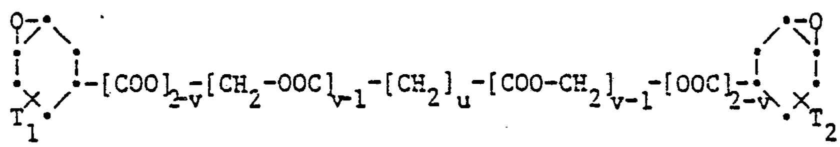

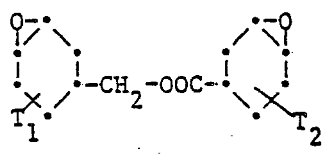 , 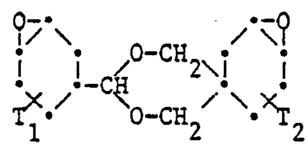 ou

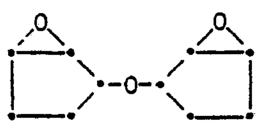

avec un sel d'amine ditertiaire de formule

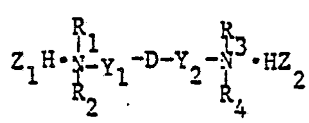

ou on convertit un diépoxyde ou un mélange de diépoxydes de l'une des formules indiquées ci-dessus, avec un hydracide halogéné de formule

$$Z_3H$$

en une dihalogénohydrine ou en un mélange de dihalogénohydrines de l'une des formules

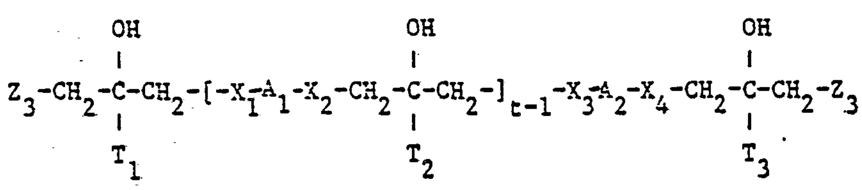

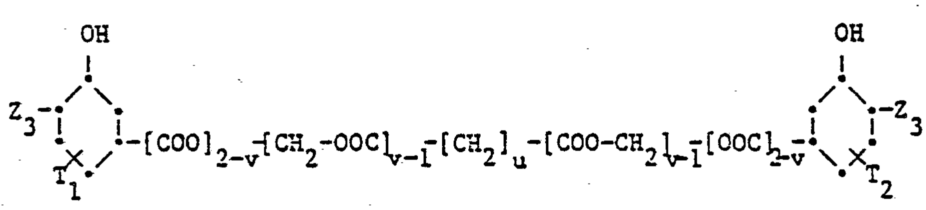 ,

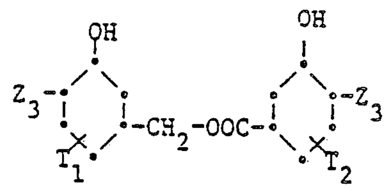

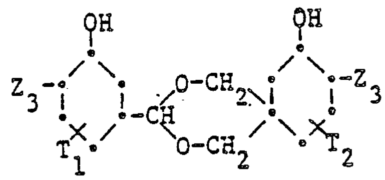

ou

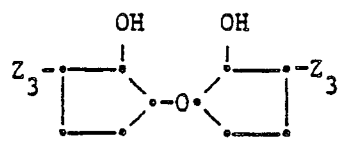

et consécutivement on la fait réagir avec une amine ditertiaire de formule

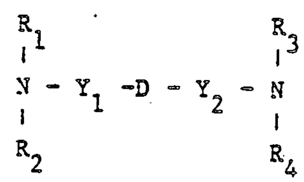

et éventuellement on fait réagir les produits de réaction ou les mélanges de produits de réaction obtenus, qui présentent les unités de l'une des formules

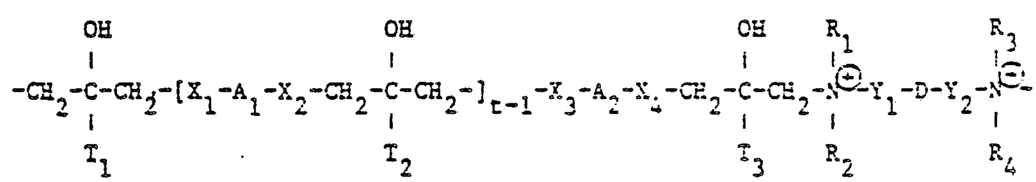

,

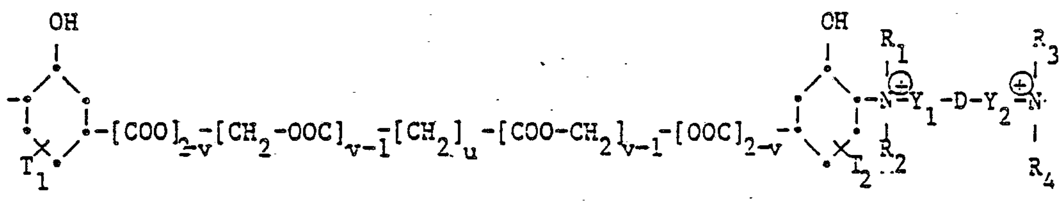

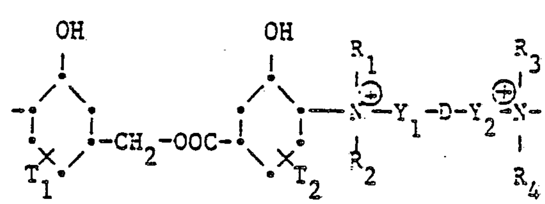

,

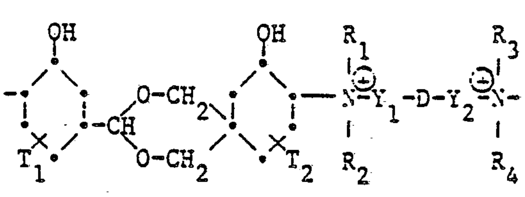

ou

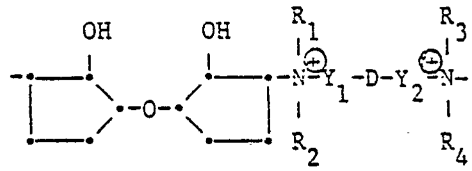

avec un anhydride d'acide ou un mélange d'anhydrides d'acides de formules

$M_6$—OC—O—CO—$M_6$
$M_7$—OC—O—CO—$M_7$ et/ou
$M_8$—OC—O—CO—$M_8$

pour obtenir les esters correspondants, qui présentent les unités de l'une des formules

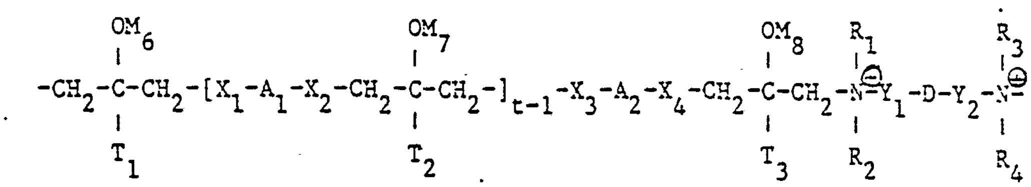

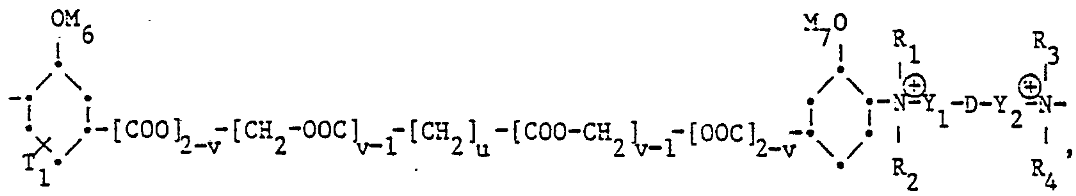

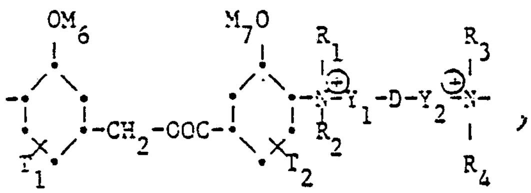

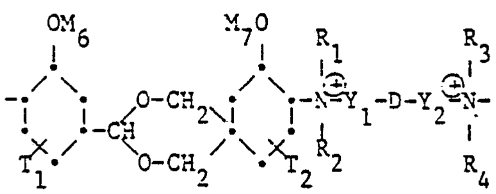

ou

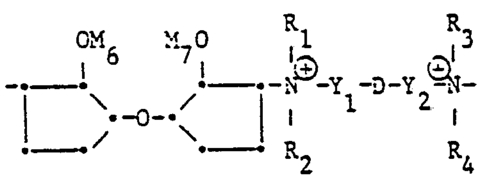

dans les formules précitées $Z_1$ et $Z_2$ désignant un anion tensio-actif, un anion d'un acide gras, un anion d'acides organiques à 1 à 4 atomes de carbone, un anion d'acides sulfoniques aromatiques à 6 à 9 atomes de carbone ou un anion d'acides minéraux ou de leurs esters alkyliques à 1 à 4 atomes de carbone dans le reste alkyle ; $Z_3$ désignant un halogène, et $M_6$, $M_7$ et $M_8$ désignant chacun un acyle à deux à cinq atomes de carbone, et $A_1$, $A_2$, D, $R_1$, $R_2$, $R_3$, $R_4$, $T_1$, $T_2$, $T_3$, $X_1$, $X_2$, $X_3$, $X_4$, $Y_1$, $Y_2$, t, u et v étant définis comme spécifié dans la revendication 1.

8. Procédé selon la revendication 7, caractérisé par le fait que l'on fait réagir un diépoxyde ou un mélange de diépoxydes de formule

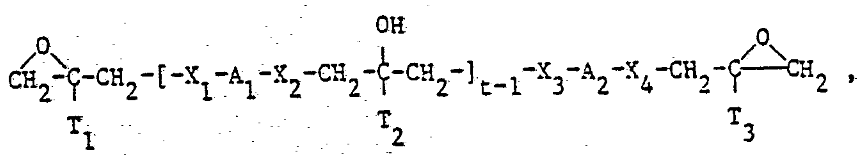

avec une amine ditertiaire de formule

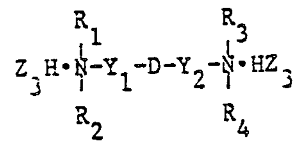

ou on convertit un diépoxyde ou un mélange de diépoxydes de la formule indiquée ci-dessus avec un hydracide halogéné de formule

$Z_3H$

en une dihalogénohydrine ou en un mélange de dihalogénohydrines de formule

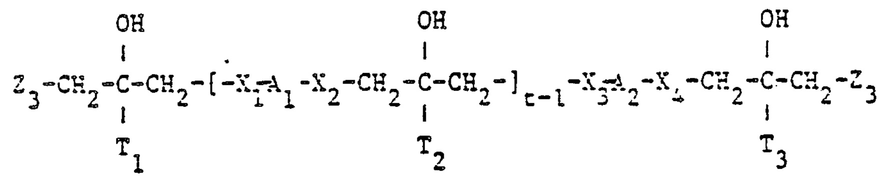

,

que l'on fait réagir consécutivement avec une amine ditertiaire de formule

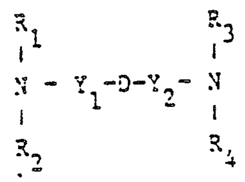

et éventuellement on fait ultérieurement réagir les produits de réaction ou les mélanges de produits de réaction obtenus, qui présentent les unités de formule

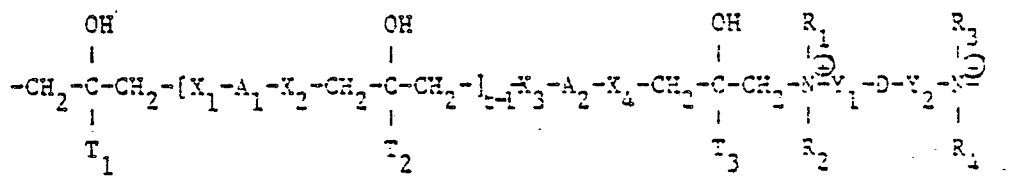

,

avec un anhydride d'acide ou un mélange d'anhydrides d'acides de formules

$M_6$—OC—O—CO—$M_6$,
$M_7$—OC—O—CO—$M_7$ et/ou
$M_8$—OC—O—CO—$M_8$

pour obtenir les esters correspondants, qui présentent des unités de formule

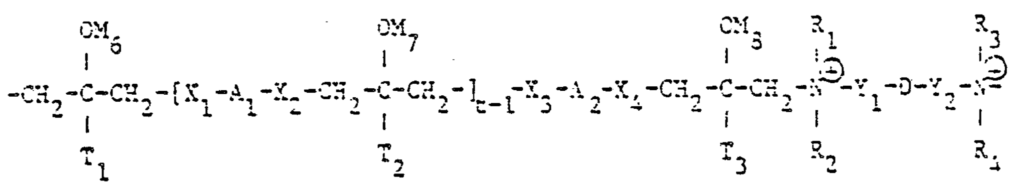

,

dans les formules précitées $Z_3$ désignant un halogène ; et $M_6$, $M_7$ et $M_8$ désignant chacun un acyle à deux à cinq atomes de carbone, et $A_1$, $A_2$, D, $R_1$, $R_2$, $R_3$, $R_4$, $T_1$, $T_2$, $T_3$, $X_1$, $X_2$, $X_3$, $X_4$, $Y_1$, $Y_2$ et t étant définis comme spécifié ci-dessus.

9. Application des sels d'ammonium selon l'une quelconque des revendications 1 à 6 en tant qu'agents cosmétiques, qu'agents de lavage pour des matières textiles teintes ou imprimées par voie cationique, qu'agents de collage du papier, qu'agents de fixation pour des matières à teneur cellulosique, teintes par voie anionique et qu'agents algicides, fongicides et bactéricides.

10. Procédé de traitement des cheveux, caractérisé par le fait que l'on applique sur le cheveu au moins un sel d'ammonium selon l'une quelconque des revendications 1 à 6.

11. Procédé pour le lavage d'une matière textile dans un milieu aqueux, ladite matière étant imprimée ou teinte par au moins un colorant cationique, dans lequel la matière ou les matières imprimée(s) ou teintes(s), qui sont à chaque fois imprimées ou teintes avec des colorants cationiques de types différents, ou une matière individuelle imprimée par zones, sont exposées à un lavage, caractérisé par le fait que l'eau de lavage contient au moins un sel d'ammonium selon l'une quelconque des revendications 1 à 6.

12. Procédé pour le collage du papier, dans lequel on incorpore le bain de collage à la suspension de matière fibreuse ou on imprègne le papier avec le bain de collage et on le sèche, caractérisé par le fait que le bain de collage contient au moins un sel d'ammonium selon l'une quelconque des revendications 1 à 6.

13. Procédé pour le traitement ultérieur de matières à teneur cellulosique, qui sont teintes avec des colorants anioniques, caractérisé par le fait que l'on utilise à cet effet au moins un sel d'ammonium selon l'une quelconque des revendications 1 à 6 sous forme d'une solution aqueuse.

14. Procédé pour traiter des substrats, caractérisé par le fait que l'on utilise au moins un sel d'ammonium selon l'une quelconque des revendications 1 à 6 comme agents algicides, bactéricides et/ou fongicides.